# EUROPEAN PATENT SPECIFICATION

(11) **EP 3 573 623 B1**
(45) Date of publication and mention of the grant of the patent: **04.03.2026**
(21) Application number: 18744616.6
(22) Date of filing: 30.01.2018
(51) Int. Cl.: A61K 31/7088, A61K 31/7115, A61K 31/7125, A61K 31/713, A61K 48/00, C12N 15/11, A61P 7/02

(54) **COMPOSITIONS AND METHODS FOR INHIBITION OF FACTOR XII GENE EXPRESSION**
ZUSAMMENSETZUNGEN UND VERFAHREN ZUR HEMMUNG DER FAKTOR XII-GENEXPRESSION
COMPOSITIONS ET MÉTHODES D'INHIBITION DE L'EXPRESSION GÉNIQUE DU FACTEUR XII

(30) Priority: 30.01.2017 US 201762451868 P
(43) Date of publication of application: 04.12.2019
(73) Proprietor: Arrowhead Pharmaceuticals, Inc., Pasadena, CA 91105 (US)
(72) Inventor: LI, Zhen, Madison WI 53719 (US); PEI, Tao, Madison WI 53719 (US); KANNER, Steven, B., Berkeley CA 94710 (US); ZHU, Rui, Madison WI 53719 (US); MELQUIST, Stacey, Madison WI 53719 (US); ALMEIDA, Lauren, J., Madison WI 53711 (US)
(74) Representative: Arnold & Siedsma
(86) International application number: PCT/US2018/015866
(87) International publication number: WO 2018/140920

(56) References cited:
- WO-A1-2011/121123
- WO-A2-2016/179342
- US-A1- 2016 272 978
- US-A1- 2016 272 978
- AKINC ET AL.: "An Investigational RNAi Therapeutic Targeting Factor XII (ALN-F12) for the Treatment of Hereditary Angioedema", JOURNAL OF ALLERGY AND CLINICAL IMMUNOLOGY, vol. 137, no. 2, 10 February 2016 (2016-02-10), pages AB254, XP029414882
- HEESTERMANS ET AL.: "Role of platelets, neutrophils, and factor XII in spontaneous venous thrombosis in mice", BLOOD, vol. 127, no. 21, 26 May 2016 (2016-05-26), pages 2630 - 2637, XP055522971
- STAVROU ET AL.: "Factor XII and uPAR upregulate neutrophil functions to influence wound healing", THE JOURNAL OF CLINICAL INVESTIGATION, vol. 128, no. 3, 29 January 2018 (2018-01-29), pages 944 - 959, XP055509140

## Description

### FIELD OF THE INVENTION

Disclosed herein are RNA interference (RNAi) agents for inhibition of Factor XII gene expression, compositions that include FXII RNAi agents.

### BACKGROUND

Factor XII (also referred to as FXII, F12, or Hageman factor), a serine protease expressed predominantly in the liver and found in blood, has dual functions in both the intrinsic coagulation pathway and the kinin-kallikrein system. The kinin-kallikrein system plays a role in inflammation, blood pressure control, coagulation and pain. The active form of Factor XII binds and cleaves both Factor XI in the coagulation cascade and prekallikrein in the kinin-kininogen system, yielding the active forms FXI and kallikrein, respectively.

Patients with complete loss of FXII do not present with a bleeding disorder. Further, it has been shown that mice lacking FXII by gene knockout are protected from thrombosis (Renne et al JEM 2005, 202:271-281). The thrombo-protective effect of FXII depletion was also observed in FXII-inhibitory antibody treated mice, rabbits and primates (Larsson et al. Science Trans Med, 2014 6:22ra17). Current treatments for thromboembolic events target enzymes downstream in the coagulation pathway that are critical for controlling injury-related blood loss through fibrin formation, and therefore, treatment with these agents have the downside of potential life-threatening hemorrhage. Particularly where Factor Xa inhibitors are contraindicated, the administration of current anticoagulant treatments elevate the risk of major bleeding events.

Hereditary angioedema (HAE) is a rare disease characterized by recurrent episodes of severe swelling. The most common areas of the body to develop swelling are the limbs, face, intestinal tract, and airway. Episodes may be spontaneous or be induced by physical trauma or stress. Laryngeal (airway) edema can be life-threatening, as it can lead to death by asphyxiation. The majority of HAE treatment options are for administration at the time of attack, focusing on either C1INH replacement, inhibiting kallikrein, or signaling through the bradykinin 2 receptor. Currently, the only long-term prophylactic treatment is C1INH replacement therapy.

Previously discovered RNAi agents targeting FXII are described in, among others, International Patent Application Publication No. WO 2016/149331 A2. Additionally, FXII iRNA compositions are disclosed in International Patent Application Publication Nos. WO 2016/179342, US 2016/272978 and WO 2017/120397. However, the sequences and modifications of the FXII RNAi agents disclosed herein differ from those previously disclosed or known in the art. The FXII RNAi agents disclosed herein provide for potent and efficient inhibition of the expression of an FXII gene.

### SUMMARY

There exists a need for novel FXII RNA interference (RNAi) agents (also herein termed RNAi agent, RNAi trigger, or trigger) that are able to selectively and efficiently inhibit the expression of an FXII. Further, there exists a need for compositions of novel FXII-specific RNAi agents. Because both thrombosis (including venous thromboembolism, VTE) and angioedema are thought to occur through overactive signaling of their respective pathways, inhibition of FXII gene expression would be useful for, among other things, being used as a prophylactic treatment to prevent thrombosis and/or angioedema.

In general, the disclosure herein features FXII RNA interference (RNAi) agents, compositions containing FXII RNAi agents, and methods for inhibiting expression of an FXII gene *in vitro* and/or *in vivo* using the FXII RNAi agents and compositions that include FXII RNAi agents. The FXII RNAi agents described herein can be used for treating conditions or diseases caused by over-activation of the kinin-kallikrein and intrinsic coagulation pathways, such as thrombosis and/or HAE.

The FXII gene-specific RNAi agents described herein are able to selectively and efficiently decrease expression of FXII. The described FXII RNAi agents can be used in methods for therapeutic treatment (including prophylactic treatment) of diseases and conditions associated with angioedema, including but not limited to: hereditary angioedema (HAE), acquired angioedema (AAE), ACE inhibitor associated angioedema, allergic angioedema, nonhistaminergic angioedema (INAE), idiopathic angioedema, thrombosis, venous thromboembolism (VTE), thrombotic occlusive disease, and peri-operative venous occlusive disease prophylaxis. Use of the described FXII RNAi agents further provides treatment and prevention of venous occlusive disease such as deep venous thrombosis or pulmonary embolism, and treatment or prevention of arterial thromboembolic disease.

In one aspect, the disclosure features RNAi agents as described in the appended claims for inhibiting expression of the human FXII gene, wherein the RNAi agent includes a sense strand and an antisense strand. Also described herein are compositions comprising an RNAi agent capable of inhibiting the expression of an FXII gene, wherein the RNAi agent comprises a sense strand and an antisense strand, and the composition further comprises at least one pharmaceutically acceptable excipient.

Each FXII RNAi agent described herein includes a sense strand and an antisense strand. The RNAi agents described herein, upon delivery to a cell expressing FXII, inhibit the expression of one or more FXII genes *in vivo* or *in vitro.*

An FXII RNAi agent includes a sense strand (also referred to as a passenger strand), and an antisense strand (also referred to as a guide strand).

In some embodiments, the FXII RNAi agents disclosed herein target a portion of an FXII gene.

Examples of FXII RNAi agent sense strands and antisense strands that can be used in FXII RNAi agents are provided in Tables 2, 3, 4, and 6. Examples of duplexes that include FXII RNAi agent are provided in Table 5 and Table 6. Examples of 19-nucleotide core stretch sequences that may consist of or may be included in the sense strands and antisense strands of certain FXII RNAi agents disclosed herein, are provided in Table 2.

FXII RNAi agents can be delivered to target cells or tissues using any oligonucleotide delivery technology known in the art. Nucleic acid delivery methods include, but are not limited to, encapsulation in liposomes, iontophoresis, or incorporation into other vehicles, such as hydrogels, cyclodextrins, biodegradable nanocapsules, and bioadhesive microspheres, proteinaceous vectors, or Dynamic Polyconjugates^{™} (DPCs) (see, for example WO 2000/053722, WO 2008/0022309, WO 2011/104169, and WO 2012/083185). A delivery vehicle, such as a polymer, an amphipathic polymer, a membrane active polymer, a peptide, such as a melittin or melittin-like peptide, a reversibly modified polymer or peptide, or a lipid, can be used with the FXII RNAi agents disclosed herein.

FXII RNAi agent can be delivered to target cells or tissues by covalently linking or conjugating the RNAi agent to a targeting group such as an asialoglycoprotein receptor ligand. An asialoglycoprotein receptor ligand includes, consists of, or consists essentially of, a galactose or galactose derivative cluster. An FXII RNAi agent can be linked to a targeting ligand comprising the galactose derivative N-acetyl-galactosamine. A galactose derivative cluster includes an N-acetyl-galactosamine trimer or an N-acetyl-galactosamine tetramer. A galactose derivative cluster can be an N-acetyl-galactosamine trimer or an N-acetyl-galactosamine tetramer. Example targeting groups useful for delivering RNAi agents are disclosed, for example, in U.S. Patent Application No. 15/452,324 and International Patent Application Publication No. WO 2017/156012.

A targeting group can be linked to the 3' or 5' end of a sense strand or an antisense strand of an FXII RNAi agent. A targeting group can be linked to the 3' or 5' end of the sense strand. A targeting group can be linked to the 5' end of the sense strand. A targeting group can be linked internally to a nucleotide on the sense strand and/or the antisense strand of the RNAi agent. A targeting group can be linked to the RNAi agent via a linker.

A targeting group, with or without a linker, can be linked to the 5' or 3' end of any of the sense and/or antisense strands disclosed in Tables 2, 3, 4, and 6. A linker, with or without a targeting group, can be attached to the 5' or 3' end of any of the sense and/or antisense strands disclosed in Tables 2, 3, 4, and 6.

In another aspect, the disclosure features compositions that include one or more FXII RNAi agents as defined in the appended claims.

Described herein are compositions that include a combination or cocktail of at least two FXII RNAi agents having different nucleotide sequences. The two or more different FXII RNAi agents are each separately and independently linked to targeting groups. The two or more different FXII RNAi agents are each linked to targeting groups that include or consist of targeting ligands that include one or more moieties that target an asialoglycoprotein receptor. The two or more different FXII RNAi agents can each be linked to targeting groups that include or consist of targeting ligands that include one or more galactose derivatives. The two or more different FXII RNAi agents can each be linked to targeting groups that include or consist of targeting ligands that include one or more N-acetyl-galactosamines. When the two or more RNAi agents are included in a composition, each RNAi agent can independently be linked to the same targeting group. When two or more RNAi agents are included in a composition, each RNAi agent can independently be linked to a different targeting group, such as targeting groups having different chemical structures.

In some embodiments are described pharmaceutical compositions comprising one or more FXII RNAi agents as described in the appended claims. In some embodiments, an FXII RNAi agent is optionally combined with one or more additional (i.e., second, third, etc.) therapeutics. An additional therapeutic can be another FXII RNAi agent (e.g., an FXII RNAi agent which targets a different sequence within the FXII target). An additional therapeutic can also be a small molecule drug, an antibody, an antibody fragment, an aptamer, and/or a vaccine. The FXII RNAi agents, with or without the one or more additional therapeutics, can be combined with one or more excipients to form pharmaceutical compositions.

The FXII RNAi agents disclosed herein are designed to target specific positions on an FXII gene (SEQ ID NO: 1). As defined herein, an antisense strand sequence is designed to target an FXII gene at a given position on the gene when the 5' terminal nucleobase of the antisense strand would be aligned with the position that is 19 nucleotides downstream (towards the 3' end) from the position on the gene when base pairing to the gene. For example, as illustrated in Tables 1 and 2 herein, an antisense strand sequence designed to target an FXII gene at position 127 requires that when base pairing to the gene, the 5' terminal nucleobase of the antisense strand is aligned with position 145 of the FXII gene. As provided herein, an FXII RNAi agent does not require that the nucleobase at position 1 (5' → 3') of the antisense strand be complementary to the gene, provided that there is at least 85% complementarity (e.g., at least 90, 91, 92, 93, 94, 95, 96, 97, 98, 99, or 100% complementarity) of the antisense strand and the gene across a core stretch sequence of at least 16 consecutive nucleotides. For example, for an FXII RNAi agent disclosed herein that is designed to target position 127 of an FXII gene, the 5' terminal nucleobase of the antisense strand of the of the FXII RNAi agent must be aligned with position 145 of the gene; however, the 5' terminal nucleobase of the antisense strand may be, but is not required to be, complementary to position 145 of an FXII gene, provided that there is at least 85% complementarity (e.g., at least 90, 91, 92, 93, 94, 95, 96, 97, 98, 99, or 100% complementarity) of the antisense strand and the gene across a core stretch sequence of at least 16 consecutive nucleotides. As shown by, among other things, the various examples disclosed herein, the specific site of binding of the gene by the antisense strand of the FXII RNAi agent (e.g., whether the FXII RNAi agent is designed to target an FXII gene at position 127, at position 91, at position 321, or at some other position) is highly important to the level of inhibition achieved by the FXII RNAi agent.

The present FXII RNAi agent comprises, consists of, or consists essentially of the duplex structure of AD05333 or the present FXII RNAi agent comprises, consists of, or consists essentially of the duplex structure of AD04625.

The present FXII RNAi agents described herein include modified nucleotides. The FXII RNAi agents described herein can also include one or more phosphorothioate internucleoside linkages.

The antisense strand of an FXII RNAi agent comprises or consists of the sequence (5' → 3') usUfsusGfuAfcUfuAfuGfcUfcCfuUfgGfsc (SEQ ID NO: 404), wherein a, c, g, and u are 2'-O-methyl adenosine, cytidine, guanosine, or uridine, respectively; Af, Cf, Gf, and Uf are 2'-fluoro adenosine, cytidine, guanosine, or uridine, respectively; and s is a phosphorothioate linkage, and the sense strand is at least substantially complementary to the antisense strand.

In some embodiments, the antisense strand of an FXII RNAi agent comprises or consists of the sequence (5' → 3') usUfsusGfuAfcUfuAfuGfcUfcCfuUfgGfsc (SEQ ID NO: 404) and the sense strand of an FXII RNAi agent comprises or consists of the sequence (5' → 3') (NAG37)gsccaaggaGfCfAfuaaguacaaas(invAb) (SEQ ID NO: 643), wherein a, c, g, and u are 2'-O-methyl adenosine, cytidine, guanosine, or uridine, respectively; Af, Cf, Gf, and Uf are 2'-fluoro adenosine, cytidine, guanosine, or uridine, respectively; s is a phosphorothioate linkage; (invAb) is inverted abasic deoxyribose (invAb); and (NAG37) is the targeting ligand that includes N-acetyl-galactosamine having the structure shown in Table 7 herein.

In some embodiments, the antisense strand of an FXII RNAi agent comprises or consists of the sequence (5' → 3') usUfsusGfuAfcUfuAfuGfcUfcCfuUfgGfsg (SEQ ID NO: 375) and the sense strand of an FXII RNAi agent comprises or consists of the sequence (5' → 3') (NAG37)scsccaaggaGfCfAfuaaguacaaas(invAb) (SEQ ID NO: 611), wherein a, c, g, and u are 2'-O-methyl adenosine, cytidine, guanosine, or uridine, respectively; Af, Cf, Gf, and Uf are 2'-fluoro adenosine, cytidine, guanosine, or uridine, respectively; s is a phosphorothioate linkage; (invAb) is inverted abasic deoxyribose (invAb); and (NAG37) is the targeting ligand that includes N-acetyl-galactosamine having the structure shown in Table 7 herein.

Disclosed herein are targeting groups having the structure of (PAZ), (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s, (NAG38), (NAG38)s, (NAG39), (NAG39)s, as defined herein in Table 7.

Disclosed herein are targeting group at the 5' end of the sense strand having the structure of (PAZ), (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s, (NAG38), (NAG38)s, (NAG39), (NAG39)s, as defined herein in Table 7.

The FXII RNAi agents disclosed herein can be incorporated into a composition comprising one or more disclosed FXII RNAi agent and at least one pharmaceutically acceptable excipient. In some embodiments, the compositions disclosed herein comprising one or more of the disclosed FXII RNAi agents and at least one pharmaceutically acceptable excipient is a pharmaceutical composition.

The pharmaceutical compositions comprising one or more FXII RNAi agents can be administered in a number of ways depending upon whether local or systemic treatment is desired. Administration can be made by any way commonly known in the art, such as, but not limited to, intravenous, intraarterial, subcutaneous, intraperitoneal, subdermal (e.g., via an implanted device), and intraparenchymal administration. In some embodiments, the pharmaceutical compositions described herein are administered by subcutaneous injection.

In some embodiments, the compositions comprising one or more disclosed FXII RNAi agents and at least one pharmaceutically acceptable excipient can further comprise one or more additional therapeutics or treatments.

In some embodiments, the compositions described herein comprising one or more FXII RNAi agents are packaged in a kit, container, pack, dispenser, pre-filled syringes, or vials. In some embodiments, the compositions described herein are administered parenterally.

The FXII RNAi agents and compositions comprising same disclosed herein are for use in the treatment (including prophylactic treatment) of a pathological state (such as a condition or disease) mediated at least in part by FXII expression. The condition or disease that may be treated, prevented, and/or managed by administration of the FXII RNAi agents and compositions comprising same disclosed herein include HAE, AAE, ACE inhibitor associated angioedema, allergic angioedema, INAE, idiopathic angioedema, thrombosis, VTE, thrombotic occlusive disease, and peri-operative venous occlusive disease prophylaxis.

As used herein, the terms "oligonucleotide" and "polynucleotide" mean a polymer of linked nucleosides each of which can be independently modified or unmodified.

As used herein, an "RNAi agent" (also referred to as an "RNAi trigger") means a composition that contains an RNA or RNA-like (e.g., chemically modified RNA) oligonucleotide molecule that is capable of degrading or inhibiting translation of messenger RNA (mRNA) transcripts of a target mRNA in a sequence specific manner. As used herein, RNAi agents may operate through the RNA interference mechanism (i.e., inducing RNA interference through interaction with the RNA interference pathway machinery (RNA-induced silencing complex or RISC) of mammalian cells), or by any alternative mechanism(s) or pathway(s). While it is believed that RNAi agents, as that term is used herein, operate primarily through the RNA interference mechanism, the disclosed RNAi agents are not bound by or limited to any particular pathway or mechanism of action. RNAi agents disclosed herein are comprised of a sense strand and an antisense strand, and include, but are not limited to: short interfering RNAs (siRNAs), double-strand RNAs (dsRNA), micro RNAs (miRNAs), short hairpin RNAs (shRNA), and dicer substrates. The antisense strand of the RNAi agents described herein is at least partially complementary to the mRNA being targeted (e.g. FXII mRNA). RNAi agents can include one or more modified nucleotides and/or one or more non-phosphodiester linkages.

As used herein, the terms "silence," "reduce," "inhibit," "down-regulate," or "knockdown" when referring to expression of a given gene, mean that the expression of the gene, as measured by the level of RNA transcribed from the gene or the level of polypeptide, protein or protein subunit translated from the mRNA in a cell, group of cells, tissue, organ, or subject in which the gene is transcribed, is reduced when the cell, group of cells, tissue, organ, or subject is treated with the RNAi agents described herein as compared to a second cell, group of cells, tissue, organ, or subject that has not or have not been so treated.

As used herein, the terms "sequence" and "nucleotide sequence" mean a succession or order of nucleobases or nucleotides, described with a succession of letters using standard nomenclature.

As used herein, a "base", "nucleotide base," or "nucleobase," is a heterocyclic pyrimidine or purine compound, which is a standard constituent of all nucleic acids, and includes the bases that form the nucleotides adenine (A), guanine (G), cytosine (C), thymine (T), and uracil (U). A nucleobase may further be modified to include, without limitation, universal bases, hydrophobic bases, promiscuous bases, size-expanded bases, and fluorinated bases. As used herein, the term "nucleotide" can include a modified nucleotide (such as, for example, a nucleotide mimic, abasic residue (Ab), or a surrogate replacement moiety).

As used herein, and unless otherwise indicated, the term "complementary," when used to describe a first nucleobase or nucleotide sequence (e.g., RNAi agent sense strand or targeted mRNA) in relation to a second nucleobase or nucleotide sequence (e.g., RNAi agent antisense strand or a single-stranded antisense oligonucleotide), means the ability of an oligonucleotide or polynucleotide including the first nucleotide sequence to hybridize (form base pair hydrogen bonds under mammalian physiological conditions (or similar conditions in vitro)) and form a duplex or double helical structure under certain standard conditions with an oligonucleotide or polynucleotide including the second nucleotide sequence. Complementary sequences include Watson-Crick base pairs or non-Watson-Crick base pairs and include natural or modified nucleotides or nucleotide mimics, at least to the extent that the above hybridization requirements are fulfilled. Sequence identity or complementarity is independent of modification. For example, a and Af, as defined herein, are complementary to U (or T) and identical to A for the purposes of determining identity or complementarity.

As used herein, "perfectly complementary" or "fully complementary" means that all (100%) of the nucleobases or nucleotides in a contiguous sequence of a first polynucleotide will hybridize with the same number of nucleobases or nucleotides in a contiguous sequence of a second polynucleotide. The contiguous sequence may comprise all or a part of a first or second nucleotide sequence.

As used herein, "partially complementary" means that in a hybridized pair of nucleobase sequences, at least 70%, but not all, of the bases in a contiguous sequence of a first polynucleotide will hybridize with the same number of bases in a contiguous sequence of a second polynucleotide.

As used herein, "substantially complementary" means that in a hybridized pair of nucleobase sequences, at least 85%, but not all, of the bases in a contiguous sequence of a first polynucleotide will hybridize with the same number of bases in a contiguous sequence of a second polynucleotide. The terms "complementary," "fully complementary," "partially complementary," and "substantially complementary" herein are used with respect to the nucleobase or nucleotide matching between the sense strand and the antisense strand of an RNAi agent, or between the antisense strand of an RNAi agent and a sequence of an FXII mRNA.

As used herein, the term "substantially identical" or "substantially identity" as applied to nucleic acid sequence means that a nucleic acid sequence comprises a sequence that has at least about 85% sequence identity or more, e.g., at least 90%, at least 95%, or at least 99% identity, compared to a reference sequence. Percentage of sequence identity is determined by comparing two optimally aligned sequences over a comparison window. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity. The inventions disclosed herein encompass nucleotide sequences substantially identical to those disclosed herein.

As used herein, the terms "treat," "treatment," and the like, mean the methods or steps taken to provide relief from or alleviation of the number, severity, and/or frequency of one or more symptoms of a disease in a subject. As used herein, "treat" and treatment" may include the prevention, management, prophylactic treatment, and/or inhibition of the number, severity, and/or frequency of one or more symptoms of a disease in a subject.

As used herein, the phrase "introducing into a cell," when referring to an RNAi agent, means functionally delivering the RNAi agent into a cell. The phrase "functional delivery," means that delivering the RNAi agent to the cell in a manner that enables the RNAi agent to have the expected biological activity, e.g., sequence-specific inhibition of gene expression.

Unless stated otherwise, use of the symbol 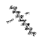 as used herein means that any group or groups may be linked thereto that is in accordance with the scope of the inventions described herein.

As used herein, the term "isomers" refers to compounds that have identical molecular formulae, but that differ in the nature or the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images are termed "enantiomers," or sometimes optical isomers. A carbon atom bonded to four non-identical substituents is termed a "chiral center."

As used herein, unless specifically identified in a structure as having a particular conformation, for each structure in which asymmetric centers are present and thus give rise to enantiomers, diastereomers, or other stereoisomeric configurations, each structure disclosed herein is intended to represent all such possible isomers, including their optically pure and racemic forms. For example, the structures disclosed herein are intended to cover mixtures of diastereomers as well as single stereoisomers.

As used in a claim herein, the phrase "consisting of" excludes any element, step, or ingredient not specified in the claim. When used in a claim herein, the phrase "consisting essentially of" limits the scope of a claim to the specified materials or steps and those that do not materially affect the basic and novel characteristic(s) of the claimed invention.

The person of ordinary skill in the art would readily understand and appreciate that the compounds and compositions disclosed herein may have certain atoms (e.g., N, O, or S atoms) in a protonated or deprotonated state, depending upon the environment in which the compound or composition is placed. Accordingly, as used herein, the structures disclosed herein envisage that certain functional groups, such as, for example, OH, SH, or NH, may be protonated or deprotonated. The disclosure herein is intended to cover the disclosed compounds and compositions regardless of their state of protonation based on the environment (such as pH), as would be readily understood by the person of ordinary skill in the art.

Unless otherwise defined, all technical and scientific terms used herein have the same meaning as commonly understood by one of ordinary skill in the art. Although methods and materials similar or equivalent to those described herein can be used in the practice or testing of the present invention, suitable methods and materials are described below..

Other objects, features, aspects, and advantages of the invention will be apparent from the following detailed description, accompanying figures, and from the claims.

### BRIEF DESCRIPTION OF THE DRAWINGS

FIG. 1. Graph showing knockdown in mice treated with a single subcutaneous dose of 3 mg/kg FXII RNAi agent AD03632 on day 1, compared to mice treated with saline. Standard error of the mean is displayed as error bars graphed above the mean.
FIG. 2. Graph showing knockdown in mice treated with (i) a single subcutaneous dose of 0.6 mg/kg FXII RNAi agent AD03632 (dashed line with triangles); (ii) a single subcutaneous dose of 2 mg/kg FXII RNAi agent AD03632 (solid line with triangles); and (iii) saline (solid line with circles). Standard deviation is displayed as error bars graphed above the mean.
FIG. 3. Graph showing knockdown in mice treated with (i) 0.6 mg/kg FXII RNAi agent AD03632 (dashed line with squares) administered subcutaneously once weekly for six consecutive weeks starting at day 1; (ii) 2 mg/kg FXII RNAi agent AD03632 (solid line with squares) administered subcutaneously once weekly for six consecutive weeks starting at day 1; and (iii) saline (solid line with circles). Standard error of the mean is displayed as error bars graphed above the mean.
FIG. 4. Plot showing FXII serum levels in rats treated with a single subcutaneous dose on day 1 of either (i) saline; (ii) heparin (1000 U/kg, dosed at cannulation); (iii) 1 mg/kg FXII RNAi agent AD03224; or (iv) 3 mg/kg FXII RNAi agent AD03224.
FIG. 5. Plot showing clot weight (in mg) from arterio-venous shunt model in rats taken on day 14, after a single subcutaneous dose on day 1 of either (i) saline; (ii) heparin; (iii) 1 mg/kg FXII RNAi agent AD03224; or (iv) 3 mg/kg FXII RNAi agent AD03224.

### DETAILED DESCRIPTION

### RNAi Agents

Described herein are RNAi agents for inhibiting expression of the Factor XII gene (referred to herein as FXII RNAi agents or FXII RNAi triggers) as disclosed in the appended claims.

An antisense strand core stretch sequence is 100% (perfectly) complementary or at least about 85% (substantially) complementary to a nucleotide sequence (sometimes referred to, e.g., as a target sequence) present in the FXII mRNA target. A sense strand core stretch sequence is 100% (perfectly) complementary or at least about 85% (substantially) complementary to a core stretch sequence in the antisense strand, and thus the sense strand core stretch sequence is perfectly identical or at least about 85% identical to a nucleotide sequence (target sequence) present in the FXII mRNA target. A sense strand core stretch sequence can be the same length as a corresponding antisense core sequence or it can be a different length.

Examples of nucleotide sequences used in forming FXII RNAi agents are provided in Tables 2, 3, 4, and 6. Examples of FXII RNAi agent duplexes, that include the sense strand and antisense strand sequences in Tables 2, 3, and 4, are shown in Table 5.

The FXII RNAi agent sense and antisense strands anneal to form a duplex.

The 3' end of the antisense strand can include additional abasic residues (Ab). An "abasic residue" or "abasic site" is a nucleotide or nucleoside that lacks a nucleobase at the 1' position of the sugar. Ab or AbAb can be added to the 3' end of the antisense strand. The abasic residue(s) can be added as inverted abasic residues (invAb) (see Table 7). (*See, e.g.,* F. Czauderna, Nucleic Acids Res., 2003, 31(11), 2705-16).

Examples of sequences which can be used in forming FXII RNAi agents are provided in Tables 2, 3, 4, and 6.

Modified nucleotides, when used in various polynucleotide or oligonucleotide constructs, can preserve activity of the compound in cells while at the same time increasing the serum stability of these compounds, and can also minimize the possibility of activating interferon activity in humans upon administering of the polynucleotide or oligonucleotide construct.

In some embodiments, an FXII RNAi agent is prepared or provided as a salt, mixed salt, or a free-acid. In some embodiments, an FXII RNAi agent is prepared as a sodium salt. Such forms are within the scope of the inventions disclosed herein.

### Modified Nucleotides

The present FXII RNAi agent contains modified nucleotides. As used herein, a "modified nucleotide" is a nucleotide other than a ribonucleotide (2'-hydroxyl nucleotide). In some embodiments, at least 50% (e.g., at least 60%, at least 70%, at least 80%, at least 90%, at least 95%, at least 97%, at least 98%, at least 99%, or 100%) of the nucleotides are modified nucleotides. As used herein, modified nucleotides include, but are not limited to, deoxyribonucleotides, nucleotide mimics, abasic nucleotides (represented herein as Ab), 2'-modified nucleotides, 3' to 3' linkages (inverted) nucleotides (represented herein as invdN, invN, invn), modified nucleobase-comprising nucleotides, bridged nucleotides, peptide nucleic acids (PNAs), 2',3'-seco nucleotide mimics (unlocked nucleobase analogues, represented herein as N_{UNA or} NUNA), locked nucleotides (represented herein as N_{LNA} or NLNA), 3'-O-methoxy (2' internucleoside linked) nucleotides (represented herein as 3'-OMen), 2'-F-Arabino nucleotides (represented herein as NfANA or Nf_{ANA}), 5'-Me, 2'-fluoro nucleotide (represented herein as 5Me-Nf), morpholino nucleotides, vinyl phosphonate deoxyribonucleotides (represented herein as vpdN), vinyl phosphonate containing nucleotides, and cyclopropyl phosphonate containing nucleotides (cPrpN). 2'-modified nucleotides (i.e. a nucleotide with a group other than a hydroxyl group at the 2' position of the five-membered sugar ring) include, but are not limited to, 2'-O-methyl nucleotides (represented herein as a lower case letter 'n' in a nucleotide sequence), 2'-deoxy-2'-fluoro nucleotides (represented herein as Nf, also represented herein as 2'-fluoro nucleotide), 2'-deoxy nucleotides (represented herein as dN), 2'-methoxyethyl (2'-O-2-methoxylethyl) nucleotides (represented herein as NM or 2'-MOE), 2'-amino nucleotides, and 2'-alkyl nucleotides. It is not necessary for all positions in a given compound to be uniformly modified. Conversely, more than one modification can be incorporated in a single FXII RNAi agent or even in a single nucleotide thereof. The FXII RNAi agent sense strands and antisense strands can be synthesized and/or modified by methods known in the art. Modification at one nucleotide is independent of modification at another nucleotide.

Modified nucleobases include synthetic and natural nucleobases, such as 5-substituted pyrimidines, 6-azapyrimidines and N-2, N-6 and O-6 substituted purines, (e.g., 2-aminopropyladenine, 5-propynyluracil, or 5-propynylcytosine), 5-methylcytosine (5-me-C), 5-hydroxymethyl cytosine, inosine, xanthine, hypoxanthine, 2-aminoadenine, 6-alkyl (e.g., 6-methyl, 6-ethyl, 6-isopropyl, or 6-n-butyl) derivatives of adenine and guanine, 2-alkyl (e.g., 2-methyl, 2-ethyl, 2-isopropyl, or 2-n-butyl) and other alkyl derivatives of adenine and guanine, 2-thiouracil, 2-thiothymine, 2-thiocytosine, 5-halouracil, cytosine, 5-propynyl uracil, 5-propynyl cytosine, 6-azo uracil, 6-azo cytosine, 6-azo thymine, 5-uracil (pseudouracil), 4-thiouracil, 8-halo, 8-amino, 8-sulfhydryl, 8-thioalkyl, 8-hydroxyl and other 8-substituted adenines and guanines, 5-halo (e.g., 5-bromo), 5-trifluoromethyl, and other 5-substituted uracils and cytosines, 7-methylguanine and 7-methyladenine, 8-azaguanine and 8-azaadenine, 7-deazaguanine, 7-deazaadenine, 3-deazaguanine, and 3-deazaadenine.

### Modified Internucleoside Linkages

FXII RNAi agent can be linked by non-standard linkages or backbones (i.e., modified internucleoside linkages or modified backbones). Modified internucleoside linkages or backbones include, but are not limited to, 5'-phosphorothioate groups (represented herein as a lower case "s"), chiral phosphorothioates, thiophosphates, phosphorodithioates, phosphotriesters, aminoalkyl-phosphotriesters, alkyl phosphonates (e.g., methyl phosphonates or 3'-alkylene phosphonates), chiral phosphonates, phosphinates, phosphoramidates (e.g., 3'-amino phosphoramidate, aminoalkylphosphoramidates, or thionophosphoramidates), thionoalkyl-phosphonates, thionoalkylphosphotriesters, morpholino linkages, boranophosphates having normal 3'-5' linkages, 2'-5' linked analogs of boranophosphates, or boranophosphates having inverted polarity wherein the adjacent pairs of nucleoside units are linked 3'-5' to 5'-3' or 2'-5' to 5'-2'. In some embodiments, a modified internucleoside linkage or backbone lacks a phosphorus atom. Modified internucleoside linkages lacking a phosphorus atom include, but are not limited to, short chain alkyl or cycloalkyl inter-sugar linkages, mixed heteroatom and alkyl or cycloalkyl inter-sugar linkages, or one or more short chain heteroatomic or heterocyclic inter-sugar linkages. In some embodiments, modified internucleoside backbones include, but are not limited to, siloxane backbones, sulfide backbones, sulfoxide backbones, sulfone backbones, formacetyl and thioformacetyl backbones, methylene formacetyl and thioformacetyl backbones, alkene-containing backbones, sulfamate backbones, methyleneimino and methylenehydrazino backbones, sulfonate and sulfonamide backbones, amide backbones, and other backbones having mixed N, O, S, and CH₂ components.

### FXII RNAi Agents

FXII RNAi agents can target an FXII gene at or near the positions of the FXII sequence shown in Table 1.

**Table 1. FXII 19-mer mRNA Target Sequences (taken from homo sapiens coagulation factor XII (F12) cDNA, GenBank NM_000505.3 (SEQ ID NO:1)).**

| **SEQ ID No.** | **FXII 19-mer Target Sequences (5' → 3')** | **Positions on SEQ ID NO: 1** |
|---|---|---|
| 2 | CAAGGAGCAUAAGUACAAA | 127-145 |
| 3 | UGGAGUCAACACUUUCGAU | 90-108 |
| 4 | GGAGUCAACACUUUCGAUU | 91-109 |
| 5 | CCCAAGGAGCAUAAGUACA | 125-143 |
| 6 | CCAAGGAGCAUAAGUACAA | 126-144 |
| 7 | AAGGAGCAUAAGUACAAAG | 128-146 |
| 8 | AGCAUAAGUACAAAGCUGA | 132-150 |
| 9 | CCACAAAUGUACCCACAAG | 223-241 |
| 10 | CCAAGAAAGUGAAAGACCA | 321-339 |
| 11 | GGGGUCGCUGCCUAGAGGU | 666-684 |
| 12 | GCCGGAACCCGGACAACGA | 846-864 |
| 13 | CGGAACCCGGACAACGACA | 848-867 |
| 14 | GGAACCCGGACAACGACAU | 849-867 |
| 15 | GCUCCGCAAGAGUCUGUCU | 1135-1153 |
| 16 | CGCAAGAGUCUGUCUUCGA | 1139-1157 |
| 17 | CGCUCCUGUCGCCUUACGU | 1476-1494 |
| 18 | GCUCCUGUCGCCUUACGUU | 1477-1495 |
| 19 | GCAACAAGCCAGGCGUCUA | 1827-1845 |
| 20 | GAACUCAAUAAAGUGCUUU | 2019-2037 |
| 21 | CUCAAUAAAGUGCUUUGAA | 2022-2040 |
| 22 | UCAAUAAAGUGCUUUGAAA | 2023-2041 |

FXII RNAi agents can include core 19-mer nucleotide sequences shown in the following Table 2.

**Table 2. Example F12 RNAi Agent Antisense Strand and Sense Strand Core Stretch Base Sequences (N=any nucleobase).**

| **SEQ ID NO:** | **Antisense Base Sequence (5' → 3')** | **SEQ ID NO:** | **Sense Base Sequence (5' → 3')** | **Corresponding Positions on SEQ ID NO: 1** |
|---|---|---|---|---|
| 23 | UUUGUACUUAUGCUCCUUG | 96 | CAAGGAGCAUAAGUACAAA | 127-145 |
| 24 | NUUGUACUUAUGCUCCUUG | 97 | CAAGGAGCAUAAGUACAAN | 127-145 |
| 25 | NUUGUACUUAUGCUCCUUN | 98 | NAAGGAGCAUAAGUACAAN | 127-145 |
| 26 | AUCGAAAGUGUUGACUCCA | 99 | UGGAGUCAACACUUUCGAU | 90-108 |
| 27 | UUCGAAAGUGUUGACUCCA | 100 | UGGAGUCAACACUUUCGAA | 90-108 |
| 28 | NUCGAAAGUGUUGACUCCA | 101 | UGGAGUCAACACUUUCGAN | 90-108 |
| 29 | NUCGAAAGUGUUGACUCCN | 102 | NGGAGUCAACACUUUCGAN | 90-108 |
| 30 | AAUCGAAAGUGUUGACUCC | 103 | GGAGUCAACACUUUCGAUU | 91-109 |
| 31 | UAUCGAAAGUGUUGACUCC | 104 | GGAGUCAACACUUUCGAUA | 91-109 |
| 32 | NAUCGAAAGUGUUGACUCC | 105 | GGAGUCAACACUUUCGAUN | 91-109 |
| 33 | NAUCGAAAGUGUUGACUCN | 106 | NGAGUCAACACUUUCGAUN | 91-109 |
| 34 | UGUACUUAUGCUCCUUGGG | 107 | CCCAAGGAGCAUAAGUACA | 125-143 |
| 35 | NGUACUUAUGCUCCUUGGG | 108 | CCCAAGGAGCAUAAGUACN | 125-143 |
| 36 | NGUACUUAUGCUCCUUGGN | 109 | NCCAAGGAGCAUAAGUACN | 125-143 |
| 37 | UUGUACUUAUGCUCCUUGG | 110 | CCAAGGAGCAUAAGUACAA | 126-144 |
| 38 | NUGUACUUAUGCUCCUUGG | 111 | CCAAGGAGCAUAAGUACAN | 126-144 |
| 39 | NUGUACUUAUGCUCCUUGN | 112 | NCAAGGAGCAUAAGUACAN | 126-144 |
| 40 | CUUUGUACUUAUGCUCCUU | 113 | AAGGAGCAUAAGUACAAAG | 128-146 |
| 41 | UUUUGUACUUAUGCUCCUU | 114 | AAGGAGCAUAAGUACAAAA | 128-146 |
| 42 | NUUUGUACUUAUGCUCCUU | 115 | AAGGAGCAUAAGUACAAAN | 128-146 |
| 43 | NUUUGUACUUAUGCUCCUN | 116 | NAGGAGCAUAAGUACAAAN | 128-146 |
| 44 | UCAGCUUUGUACUUAUGCU | 117 | AGCAUAAGUACAAAGCUGA | 132-150 |
| 45 | NCAGCUUUGUACUUAUGCU | 118 | AGCAUAAGUACAAAGCUGN | 132-150 |
| 46 | NCAGCUUUGUACUUAUGCN | 119 | NGCAUAAGUACAAAGCUGN | 132-150 |
| 47 | CUUGUGGGUACAUUUGUGG | 120 | CCACAAAUGUACCCACAAG | 223-241 |
| 48 | UUUGUGGGUACAUUUGUGG | 121 | CCACAAAUGUACCCACAAA | 223-241 |
| 49 | NUUGUGGGUACAUUUGUGG | 122 | CCACAAAUGUACCCACAAN | 223-241 |
| 50 | NUUGUGGGUACAUUUGUGN | 123 | NCACAAAUGUACCCACAAN | 223-241 |
| 51 | UGGUCUUUCACUUUCUUGG | 124 | CCAAGAAAGUGAAAGACCA | 321-339 |
| 52 | NGGUCUUUCACUUUCUUGG | 125 | CCAAGAAAGUGAAAGACCN | 321-339 |
| 53 | NGGUCUUUCACUUUCUUGN | 126 | NCAAGAAAGUGAAAGACCN | 321-339 |
| 54 | ACCUCUAGGCAGCGACCCC | 127 | GGGGUCGCUGCCUAGAGGU | 666-684 |
| 55 | UCCUCUAGGCAGCGACCCC | 128 | GGGGUCGCUGCCUAGAGGA | 666-684 |
| 56 | NCCUCUAGGCAGCGACCCC | 129 | GGGGUCGCUGCCUAGAGGN | 666-684 |
| 57 | NCCUCUAGGCAGCGACCCN | 130 | NGGGUCGCUGCCUAGAGGN | 666-684 |
| 58 | UCGUUGUCCGGGUUCCGGC | 131 | GCCGGAACCCGGACAACGA | 846-864 |
| 59 | NCGUUGUCCGGGUUCCGGC | 132 | GCCGGAACCCGGACAACGN | 846-864 |
| 60 | NCGUUGUCCGGGUUCCGGN | 133 | NCCGGAACCCGGACAACGN | 846-864 |
| 61 | UGUCGUUGUCCGGGUUCCG | 134 | CGGAACCCGGACAACGACA | 848-866 |
| 62 | NGUCGUUGUCCGGGUUCCG | 135 | CGGAACCCGGACAACGACN | 848-866 |
| 63 | NGUCGUUGUCCGGGUUCCN | 136 | NGGAACCCGGACAACGACN | 848-866 |
| 64 | AUGUCGUUGUCCGGGUUCC | 137 | GGAACCCGGACAACGACAU | 849-867 |
| 65 | UUGUCGUUGUCCGGGUUCC | 138 | GGAACCCGGACAACGACAA | 849-867 |
| 66 | NUGUCGUUGUCCGGGUUCC | 139 | GGAACCCGGACAACGACAN | 849-867 |
| 67 | NUGUCGUUGUCCGGGUUCN | 140 | NGAACCCGGACAACGACAN | 849-867 |
| 68 | AGACAGACUCUUGCGGAGC | 141 | GCUCCGCAAGAGUCUGUCU | 1135-1153 |
| 69 | UGACAGACUCUUGCGGAGC | 142 | GCUCCGCAAGAGUCUGUCA | 1135-1153 |
| 70 | NGACAGACUCUUGCGGAGC | 143 | GCUCCGCAAGAGUCUGUCN | 1135-1153 |
| 71 | NGACAGACUCUUGCGGAGN | 144 | NCUCCGCAAGAGUCUGUCN | 1135-1153 |
| 72 | UCGAAGACAGACUCUUGCG | 145 | CGCAAGAGUCUGUCUUCGA | 1139-1157 |
| 73 | NCGAAGACAGACUCUUGCG | 146 | CGCAAGAGUCUGUCUUCGN | 1139-1157 |
| 74 | NCGAAGACAGACUCUUGCN | 147 | NGCAAGAGUCUGUCUUCGN | 1139-1157 |
| 75 | ACGUAAGGCGACAGGAGCG | 148 | CGCUCCUGUCGCCUUACGU | 1476-1494 |
| 76 | UCGUAAGGCGACAGGAGCG | 149 | CGCUCCUGUCGCCUUACGA | 1476-1494 |
| 77 | NCGUAAGGCGACAGGAGCG | 150 | CGCUCCUGUCGCCUUACGN | 1476-1494 |
| 78 | NCGUAAGGCGACAGGAGCN | 151 | NGCUCCUGUCGCCUUACGN | 1476-1494 |
| 79 | AACGUAAGGCGACAGGAGC | 152 | GCUCCUGUCGCCUUACGUU | 1477-1495 |
| 80 | UACGUAAGGCGACAGGAGC | 153 | GCUCCUGUCGCCUUACGUA | 1477-1495 |
| 81 | NACGUAAGGCGACAGGAGC | 154 | GCUCCUGUCGCCUUACGUN | 1477-1495 |
| 82 | NACGUAAGGCGACAGGAGN | 155 | NCUCCUGUCGCCUUACGUN | 1477-1495 |
| 83 | UAGACGCCUGGCUUGUUGC | 156 | GCAACAAGCCAGGCGUCUA | 1827-1845 |
| 84 | NAGACGCCUGGCUUGUUGC | 157 | GCAACAAGCCAGGCGUCUN | 1827-1845 |
| 85 | NAGACGCCUGGCUUGUUGN | 158 | NCAACAAGCCAGGCGUCUN | 1827-1845 |
| 86 | AAAGCACUUUAUUGAGUUC | 159 | GAACUCAAUAAAGUGCUUU | 2019-2037 |
| 87 | UAAGCACUUUAUUGAGUUC | 160 | GAACUCAAUAAAGUGCUUA | 2019-2037 |
| 88 | NAAGCACUUUAUUGAGUUC | 161 | GAACUCAAUAAAGUGCUUN | 2019-2037 |
| 89 | NAAGCACUUUAUUGAGUUN | 162 | NAACUCAAUAAAGUGCUUN | 2019-2037 |
| 90 | UUCAAAGCACUUUAUUGAG | 163 | CUCAAUAAAGUGCUUUGAA | 2022-2040 |
| 91 | NUCAAAGCACUUUAUUGAG | 164 | CUCAAUAAAGUGCUUUGAN | 2022-2040 |
| 92 | NUCAAAGCACUUUAUUGAN | 165 | NUCAAUAAAGUGCUUUGAN | 2022-2040 |
| 93 | UUUCAAAGCACUUUAUUGA | 166 | UCAAUAAAGUGCUUUGAAA | 2023-2041 |
| 94 | NUUCAAAGCACUUUAUUGA | 167 | UCAAUAAAGUGCUUUGAAN | 2023-2041 |
| 95 | NUUCAAAGCACUUUAUUGN | 168 | NCAAUAAAGUGCUUUGAAN | 2023-2041 |

The FXII RNAi agent sense strands and antisense strands that comprise or consist of the nucleotide sequences in Table 2 can be modified nucleotides or unmodified nucleotides. In some embodiments, the FXII RNAi agents having the sense and antisense strand sequences that comprise or consist of any of the nucleotide sequences in Table 2 are all or substantially all modified nucleotides.

As used herein, each N listed in a sequence disclosed in Table 2 may be independently selected. In some embodiments, an N nucleotide listed in a sequence disclosed in Table 2 has a nucleobase that is complementary to the N nucleotide at the corresponding position on the other strand. In some embodiments, an N nucleotide listed in a sequence disclosed in Table 2 has a nucleobase that is not complementary to the N nucleotide at the corresponding position on the other strand. In some embodiments, an N nucleotide listed in a sequence disclosed in Table 2 has a nucleobase that is the same as the N nucleotide at the corresponding position on the other strand. In some embodiments, an N nucleotide listed in a sequence disclosed in Table 2 has a nucleobase that is different from the N nucleotide at the corresponding position on the other strand.

Certain modified FXII RNAi agent sense and antisense strands are provided in Table 3 and Table 4. Modified FXII RNAi agent antisense strands, as well as their underlying unmodified nucleobase sequences, are provided in Table 3. Modified FXII RNAi agent sense strands, as well as their underlying unmodified sequences, are provided in Table 4. In forming FXII RNAi agents, each of the nucleotides in each of the underlying base sequences listed in Tables 3 and 4, as well as in Table 2, above, can be a modified nucleotide.

The FXII RNAi agents can be formed by annealing an antisense strand with a sense strand. A sense strand containing a sequence listed in Table 2, Table 3, or Table 6, can be hybridized to any antisense strand containing a sequence listed in Table 2, Table 4, or Table 6, provided the two sequences have a region of at least 85% complementarity over a contiguous 16, 17, 18, 19, 20, or 21 nucleotide sequence.

An FXII RNAi agent antisense strand can comprise a nucleotide sequence of any of the sequences in Table 2, Table 3, or Table 6.

An FXII RNAi agent can comprise or consists of a duplex having the nucleobase sequences of the sense strand and the antisense strand of any of the sequences in Table 2, Table 3, Table 4, or Table 6.

Examples of antisense strands containing modified nucleotides are provided in Table 3. Examples of sense strands containing modified nucleotides are provided in Table 4.

As used in Tables 3 and 4, the following notations are used to indicate modified nucleotides, targeting groups, and linking groups. As the person of ordinary skill in the art would readily understand, unless otherwise indicated by the sequence, that when present in an oligonucleotide, the monomers are mutually linked by 5'-3'-phosphodiester bonds:

| | |
|---|---|
| A | = adenosine-3'-phosphate; |
| C | = cytidine-3'-phosphate; |
| G | = guanosine-3'-phosphate; |
| U | = uridine-3'-phosphate |
| n | = any 2'-OMe modified nucleotide |
| a | = 2'-O-methyladenosine-3'-phosphate |
| as | = 2'-O-methyladenosine-3'-phosphorothioate |
| c | = 2'-O-methylcytidine-3'-phosphate |
| cs | = 2'-O-methylcytidine-3'-phosphorothioate |
| g | = 2'-O-methylguanosine-3'-phosphate |
| gs | = 2'-O-methylguanosine-3'-phosphorothioate |
| t | = 2'-O-methyl-5-methyluridine-3'-phosphate |
| ts | = 2'-O-methyl-5-methyluridine-3'-phosphorothioate |
| u | = 2'-O-methyluridine-3'-phosphate |
| us | = 2'-O-methyluridine-3'-phosphorothioate |
| Nf | = any 2'-fluoro modified nucleotide |
| Af | = 2'-fluoroadenosine-3'-phosphate |
| Afs | = 2'-fluoroadenosine-3'-phosporothioate |
| Cf | = 2'-fluorocytidine-3'-phosphate |
| Cfs | = 2'-fluorocytidine-3'-phosphorothioate |
| Gf | = 2'-fluoroguanosine-3''-phosphate |
| Gfs | = 2'-fluoroguanosine-3'-phosphorothioate |
| Tf | = 2'-fluoro-5'-methyluridine-3'-phosphate |
| Tfs | = 2'-fluoro-5'-methyluridine-3'-phosphorothioate |
| Uf | = 2'-fluorouridine-3'-phosphate |
| Ufs | = 2'-fluorouridine-3'-phosphorothioate |
| dN | = any 2'-deoxyribonucleotide |
| dT | = 2'-deoxythymidine-3'-phosphate |
| NUNA | = 2',3'-seco nucleotide mimics (unlocked nucleobase analogs)-3'-Phosphate |
| N_{UNA}s | = 2',3'-seco nucleotide mimics (unlocked nucleobase analogs)-3'-phosphorothioate |
| UUNA | = 2',3'-seco-uridine-3'-phosphate |
| U_{UNA}s | = 2',3'-seco-uridine-3'-phosphorothioate |
| a_2N | = see Table 7 |
| a_2Ns | = see Table 7 |
| pu_2N | = see Table 7 |
| pu_2Ns | = see Table 7 |
| Npu | = see Table 7 |
| Nus | = see Table 7 |
| NLNA | = locked nucleotide |
| NF_{ANA} | = 2'-F-Arabino nucleotide |
| NM | = 2'-methoxyethyl nucleotide |
| AM | = 2'-methoxyethyladenosine-3'-phosphate |
| AMs | = 2'-methoxyethyladenosine-3'-phosphorothioate |
| TM | = 2'-methoxyethylthymidine-3'-phosphate |
| TMs | = 2'-methoxyethylthymidine-3'-phosphorothioate |
| R | = ribitol |
| (invdN) | = any inverted deoxyribonucleotide (3'-3' linked nucleotide) |
| (invAb) | = inverted (3'-3' linked) abasic deoxyribonucleotide, see Table 7 |
| (invAb)s | = inverted (3'-3' linked) abasic deoxyribonucleotide-5'-phosphorothioate, see Table 7 |
| (invn) | = any inverted 2'-OMe nucleotide (3'-3' linked nucleotide) |
| s | = phosphorothioate linkage |
| vpdN | = vinyl phosphonate deoxyribonucleotide |
| (5Me-Nf) | = 5'-Me, 2'-fluoro nucleotide |
| cPrp | = cyclopropyl phosphonate, see Table 7 |
| epTcPr | = see Table 7 |
| epTM | = see Table 7 |
| (Chol-TEG) | = see Table 7 |
| (TEG-Biotin) | = see Table 7 |
| (PEG-C3-SS) | = see Table 7 |
| (Alk-SS-C6) | = see Table 7 |
| (C6-SS-Alk) | = see Table 7 |
| (C6-SS-Alk-Me) | = see Table 7 |

The person of ordinary skill in the art would readily understand that the terminal nucleotide at the 3' end of a given oligonucleotide sequence would typically have a hydroxyl (-OH) group at the respective 3' position of the given monomer instead of a phosphate moiety *ex vivo.* Unless expressly indicated otherwise herein, such understandings of the person of ordinary skill in the art are used when describing the FXII RNAi agents and compositions of FXII RNAi agents.

Targeting groups and linking groups include the following, for which their chemical structures are provided below in Table 7: (PAZ), (NAG13), (NAG13)s, (NAG18), (NAG18)s, (NAG24), (NAG24)s, (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s, (NAG38), (NAG38)s, (NAG39), (NAG39)s. Each sense strand and/or antisense strand can have any targeting groups or linking groups listed above, as well as other targeting or linking groups, conjugated to the 5' and/or 3' end of the sequence.

**Table 3. FXII RNAi Agent Antisense Strand Sequences.**

| **Antisense Strand ID:** | **SEQ ID NO.** | **Antisense Sequence (Modified) (5' → 3')** | **SEQ ID NO.** | **Underlying Antisense Base Sequence (5' → 3')** |
|---|---|---|---|---|
| AM03157-AS | 169 | usGfsgucuuUfcAfcuuUfcuugggcsuscuAu | 744 | UGGUCUUUCACUUUCUUGGGCUCUAU |
| AM03844-SS | 170 | (NAG118)gcgaugscsccaagaAfaGfugaaagacc(invdA) | 671 | GCGAUGCCCAAGAAAGUGAAAGACCA |
| AM03965-AS | 171 | usGfsgucuuUfcAfcuuUfcuugggcsusc | 739 | UGGUCUUUCACUUUCUUGGGCUC |
| AM03977-AS | 172 | usGfsgsucuuUfcAfcuuUfcuuggusu | 751 | UGGUCUUUCACUUUCUUGGUU |
| AM04047-AS | 173 | usGfsgucuuUfcAfcuuUfcuugggesasuuca | 736 | UGGUCUUUCACUUUCUUGGGCAUUCA |
| AM04048-AS | 174 | usGfsgucuuUfcAfcuuUfcuugggcsasucgc | 735 | UGGUCUUUCACUUUCUUGGGCAUCGC |
| AM04516-AS | 175 | usGfsgucuuUfcAfcuuucuugggcsuscuau | 744 | UGGUCUUUCACUUUCUUGGGCUCUAU |
| AM04568-AS | 176 | usGfsgucuuUfcAfcuuUfcuugggcsuscgc | 743 | UGGUCUUUCACUUUCUUGGGCUCGC |
| AM04569-AS | 177 | usGfsgucuuUfcAfcuuUfcuuggsgsc | 724 | UGGUCUUUCACUUUCUUGGGC |
| AM04570-AS | 178 | usGfsgucuuUfcAfcuuUfcuugggcsuscGc | 743 | UGGUCUUUCACUUUCUUGGGCUCGC |
| AM04571-AS | 179 | usGfsgucuuUfcAfcuuUfcuugggcsusuAu | 747 | UGGUCUUUCACUUUCUUGGGCUUAU |
| AM04575-AS | 180 | usCfsaCfcUfuCfuUfgGfgCfuCfcAfaAfcsascgc | 684 | UCACCUUCUUGGGCUCCAAACACGC |
| AM04576-AS | 181 | usCfsaCfcUfuCfuUfgGfgCfuCfcAfaAfcsasc | 683 | UCACCUUCUUGGGCUCCAAACAC |
| AM04577-AS | 182 | usCfsaCfcUfuCfuUfgGfgCfuCfcAfasasc | 682 | UCACCUUCUUGGGCUCCAAAC |
| AM04593-AS | 183 | usGfsgucuuUfcAfcuuUfcuugggcsusu | 746 | UGGUCUUUCACUUUCUUGGGCUU |
| AM04595-AS | 184 | usGfsgsUfcUfuUfcAfcUfuUfCfuuGfgGfcgusu | 737 | UGGUCUUUCACUUUCUUGGGCGUU |
| AM04601-AS | 185 | usGfsgsUfcUfuUfcAfcUfuUfCfuuGfgusu | 751 | UGGUCUUUCACUUUCUUGGUU |
| AM04603-AS | 186 | usGfsgsUfcUfuUfcAfcUfuUfCfuuGfgGfsu | 748 | UGGUCUUUCACUUUCUUGGGU |
| AM04606-AS | 187 | usGfsgsUfcUfuUfcAfcUfUfuCfuuGfgusu | 751 | UGGUCUUUCACUUUCUUGGUU |
| AM04607-AS | 188 | usGfsgsUfcUfuUfcAfcUfUfucuuGfgusu | 751 | UGGUCUUUCACUUUCUUGGUU |
| AM04619-AS | 189 | uusGfsgucuuUfcAfcuuUfcuugggcsuscgc | 779 | UUGGUCUUUCACUUUCUUGGGCUCGC |
| AM04620-AS | 190 | ususGfsgucuuUfcAfcuuUfcuugggcsuscgc | 779 | UUGGUCUUUCACUUUCUUGGGCUCGC |
| AM04841-AS | 191 | usGfsgucuuUfcAfcUfuUfcuugggcsasucgc | 735 | UGGUCUUUCACUUUCUUGGGCAUCGC |
| AM04842-AS | 192 | usGfsgucuuUfCfacuuUfcuugggcsasucgc | 735 | UGGUCUUUCACUUUCUUGGGCAUCGC |
| AM04843-AS | 193 | usGfsgucuuUfCfacUfuUfcuugggcsasucgc | 735 | UGGUCUUUCACUUUCUUGGGCAUCGC |
| AM04844-AS | 194 | usGfsgucuuUfcAfcuuUfcuugggcsasu | 733 | UGGUCUUUCACUUUCUUGGGCAU |
| AM04903-AS | 195 | usCfsusUfuCfaCfuUfuCfuUfgGfgCfuCfcasauca | 695 | UCUUUCACUUUCUUGGGCUCCAAUCA |
| AM04905-AS | 196 | usCfsusUfuCfaCfuUfuCfuUfgGfgCfuusu | 697 | UCUUUCACUUUCUUGGGCUUU |
| AM04906-AS | 197 | usCfsusUfuCfaCfuUfuCfuUfgGfgCfuCfcusu | 696 | UCUUUCACUUUCUUGGGCUCCUU |
| AM04907-AS | 198 | usGfsusCfuUfuCfaCfuUfuCfuUfgGfgCfucscuca | 760 | UGUCUUUCACUUUCUUGGGCUCCUCA |
| AM04909-AS | 199 | usGfsusCfuUfuCfaCfuUfuCfuUfgGfgusu | 762 | UGUCUUUCACUUUCUUGGGUU |
| AM04910-AS | 200 | usGfsusCfuUfuCfaCfuUfuCfuUfgGfgCfuusu | 761 | UGUCUUUCACUUUCUUGGGCUUU |
| AM04911-AS | 201 | usUfsgsGfuCfuUfuCfaCfuUfuCfuUfgGfgcsuuca | 780 | UUGGUCUUUCACUUUCUUGGGCUUCA |
| AM04913-AS | 202 | usUfsgsGfuCfuUfuCfaCfuUfuCfuUfgusu | 782 | UUGGUCUUUCACUUUCUUGUU |
| AM04914-AS | 203 | usUfsgsGfuCfuUfuCfaCfuUfuCfuUfgGfgusu | 781 | UUGGUCUUUCACUUUCUUGGGUU |
| AM04915-AS | 204 | usGfsasAfgCfuGfaGfgCfuCfaAfaGfcAfcusuuca | 706 | UGAAGCUGAGGCUCAAAGCACUUUCA |
| AM04917-AS | 205 | usGfsasAfgCfuGfaGfgCfuCfaAfaGfcusu | 707 | UGAAGCUGAGGCUCAAAGCUU |
| AM04918-AS | 206 | usGfsasAfgCfuGfaGfgCfuCfaAfaGfcAfcusu | 704 | UGAAGCUGAGGCUCAAAGCACUU |
| AM04919-AS | 207 | usGfsasGfaAfgCfuGfaGfgCfuCfaAfaGfcascuca | 714 | UGAGAAGCUGAGGCUCAAAGCACUCA |
| AM04921-AS | 208 | usGfsasGfaAfgCfuGfaGfgCfuCfaAfausu | 716 | UGAGAAGCUGAGGCUCAAAUU |
| AM04922-AS | 209 | usGfsasGfaAfgCfuGfaGfgCfuCfaAfaGfcusu | 715 | UGAGAAGCUGAGGCUCAAAGCUU |
| AM04923-AS | 210 | usUfsusUfuCfaAfaGfcAfcUfuUfaUfuGfagsuuca | 821 | UUUUUCAAAGCACUUUAUUGAGUUCA |
| AM04925-AS | 211 | usUfsusUfuCfaAfaGfcAfcUfuUfaUfuusu | 825 | UUUUUCAAAGCACUUUAUUUU |
| AM04926-AS | 212 | usUfsusUfuCfaAfaGfcAfcUfuUfaUfuGfausu | 822 | UUUUUCAAAGCACUUUAUUGAUU |
| AM04927-AS | 213 | usGfsgsUfcUfuUfcAfcUfuUfcUfuGfgGfcuscuca | 745 | UGGUCUUUCACUUUCUUGGGCUCUCA |
| AM04929-AS | 214 | usGfsgsUfcUfuUfcAfcUfuUfcUfuGfgusu | 751 | UGGUCUUUCACUUUCUUGGUU |
| AM04930-AS | 215 | usGfsgsUfcUfuUfcAfcUfuUfcUfuGfgGfcusu | 746 | UGGUCUUUCACUUUCUUGGGCUU |
| AM04932-AS | 216 | usGfsgsucuuUfcAfcuuUfcuugggcuscuca | 745 | UGGUCUUUCACUUUCUUGGGCUCUCA |
| AM04936-AS | 217 | usGfsgucuuUfcAfcuuUfcuugggcuscsuca | 745 | UGGUCUUUCACUUUCUUGGGCUCUCA |
| AM04938-AS | 218 | usGfsgucuuUfcAfcuuUfcuugggcsascuau | 731 | UGGUCUUUCACUUUCUUGGGCACUAU |
| AM04941-AS | 219 | usGfsgucuuUfcAfcuuUfcuugggcsascuca | 732 | UGGUCUUUCACUUUCUUGGGCACUCA |
| AM04943-AS | 220 | usGfsgucuuUfcAfcuuUfcuugggcsasccgc | 728 | UGGUCUUUCACUUUCUUGGGCACCGC |
| AM04945-AS | 221 | usGfsgUfcUfuUfcAfcUfuUfcUfuGfgGfcsascuca | 732 | UGGUCUUUCACUUUCUUGGGCACUCA |
| AM04947-AS | 222 | usGfsgUfcUfuUfcAfcUfuUfcUfuGfgGfcsasccgc | 728 | UGGUCUUUCACUUUCUUGGGCACCGC |
| AM04949-AS | 223 | usGfsgUfcUfuUfcAfcUfuUfcUfuGfgGfcsascuau | 731 | UGGUCUUUCACUUUCUUGGGCACUAU |
| AM04951-AS | 224 | usGfsgucuuUfcAfcuuUfcuugggcsasccaa | 726 | UGGUCUUUCACUUUCUUGGGCACCAA |
| AM04953-AS | 225 | usGfsgucuuUfcAfcuuUfcuugggcsasccuu | 730 | UGGUCUUUCACUUUCUUGGGCACCUU |
| AM04955-AS | 226 | usGfsgucuuUfcAfcuuUfcuugggcsasccac | 727 | UGGUCUUUCACUUUCUUGGGCACCAC |
| AM04957-AS | 227 | usGfsgucuuUfcAfcuuUfcuugggcsasccuc | 729 | UGGUCUUUCACUUUCUUGGGCACCUC |
| AM04959-AS | 228 | usGfsgsucuuUfcAfcuuUfcuugggcusu | 746 | UGGUCUUUCACUUUCUUGGGCUU |
| AM04996-AS | 229 | usGfsgsUfcUfuUfcAfcUfuUfcUfuGfgGfsc | 724 | UGGUCUUUCACUUUCUUGGGC |
| AM05002-AS | 230 | usGfsgsUfcUfuUfcAfcUfuUfcUfuGfgGfcasuc | 734 | UGGUCUUUCACUUUCUUGGGCAUC |
| AM05003-AS | 231 | usGfsgsUfcUfuUfcAfcUfuUfcUfuGfgGfscauc | 734 | UGGUCUUUCACUUUCUUGGGCAUC |
| AM05005-AS | 232 | usGfsgsUfcUfuUfcAfcUfuUfCfuuGfgGfsuuu | 749 | UGGUCUUUCACUUUCUUGGGUUU |
| AM05006-AS | 233 | usGfsgsUfcUfuUfcAfcUfuUfCfuuGfgGfsa | 723 | UGGUCUUUCACUUUCUUGGGA |
| AM05038-AS | 234 | usGfsgsUfcUfuUfcAfcUfuUfcAfuGfgGfsu | 719 | UGGUCUUUCACUUUCAUGGGU |
| AM05040-AS | 235 | usGfsgsUfcUfuUfcAfcUfuUfcAfuGfggsu | 719 | UGGUCUUUCACUUUCAUGGGU |
| AM05042-AS | 236 | asGfsgsUfcUfuUfcAfcUfuUfcAfuGfggsu | 664 | AGGUCUUUCACUUUCAUGGGU |
| AM05043-AS | 237 | usGfsgsUfcUfuUfcAfcUfuUfcAfuGfgGfsuuu | 720 | UGGUCUUUCACUUUCAUGGGUUU |
| AM05066-AS | 238 | usGfsgsUfcUfuUfcAfcUfuUfcUfuGfgGfsu | 748 | UGGUCUUUCACUUUCUUGGGU |
| AM05093-AS | 239 | usCfuUfuCfaCfuUfuCfuUfgGfgCfuCfcAfasa | 694 | UCUUUCACUUUCUUGGGCUCCAAA |
| AM05095-AS | 240 | usCfuUfuCfaCfuUfuCfuUfgGfgCfuCfcasa | 693 | UCUUUCACUUUCUUGGGCUCCAA |
| AM05097-AS | 241 | usCfuUfuCfaCfuUfuCfuUfgGfgCfuCfcsa | 692 | UCUUUCACUUUCUUGGGCUCCA |
| AM05099-AS | 242 | usCfuUfuCfaCfuUfuCfuUfgGfgCfucsc | 691 | UCUUUCACUUUCUUGGGCUCC |
| AM05101-AS | 243 | usCfuUfuCfaCfuUfuCfuUfgGfgCfusc | 690 | UCUUUCACUUUCUUGGGCUC |
| AM05103-AS | 244 | usCfuUfuCfaCfuUfuCfuUfgGfgcsu | 689 | UCUUUCACUUUCUUGGGCU |
| AM05105-AS | 245 | usGfuCfuUfuCfaCfuUfuCfuUfgGfgCfuCfcsa | 759 | UGUCUUUCACUUUCUUGGGCUCCA |
| AM05107-AS | 246 | usGfuCfuUfuCfaCfuUfuCfuUfgGfgCfucsc | 758 | UGUCUUUCACUUUCUUGGGCUCC |
| AM05109-AS | 247 | usGfuCfuUfuCfaCfuUfuCfuUfgGfgCfusc | 757 | UGUCUUUCACUUUCUUGGGCUC |
| AM05111-AS | 248 | usGfuCfuUfuCfaCfuUfuCfuUfgGfgcsu | 756 | UGUCUUUCACUUUCUUGGGCU |
| AM05113-AS | 249 | usGfuCfuUfuCfaCfuUfuCfuUfgGfgsc | 755 | UGUCUUUCACUUUCUUGGGC |
| AM05115-AS | 250 | usGfuCfuUfuCfaCfuUfuCfuUfggsg | 754 | UGUCUUUCACUUUCUUGGG |
| AM05117-AS | 251 | usGfgUfcUfuUfcAfcUfuUfcUfuGfgGfcUfcCfasa | 741 | UGGUCUUUCACUUUCUUGGGCUCCAA |
| AM05120-AS | 252 | usGfgUfcUfuUfcAfcUfuUfcUfuGfgGfcUfcsc | 740 | UGGUCUUUCACUUUCUUGGGCUCC |
| AM05122-AS | 253 | usGfgUfcUfuUfcAfcUfuUfcUfuGfgGfcUfsc | 739 | UGGUCUUUCACUUUCUUGGGCUC |
| AM05124-AS | 254 | usGfgUfcUfuUfcAfcUfuUfcUfuGfgGfcsu | 738 | UGGUCUUUCACUUUCUUGGGCU |
| AM05126-AS | 255 | usGfgUfcUfuUfcAfcUfuUfcUfuGfggsc | 724 | UGGUCUUUCACUUUCUUGGGC |
| AM05128-AS | 256 | usGfgUfcUfuUfcAfcUfuUfcUfuGfgsg | 722 | UGGUCUUUCACUUUCUUGGG |
| AM05130-AS | 257 | usGfgUfcUfuUfcAfcUfuUfcUfugsg | 721 | UGGUCUUUCACUUUCUUGG |
| AM05132-AS | 258 | usUfgGfuCfuUfuCfaCfuUfuCfuUfgGfgCfusc | 778 | UUGGUCUUUCACUUUCUUGGGCUC |
| AM05134-AS | 259 | usUfgGfuCfuUfuCfaCfuUfuCfuUfgGfgcsu | 777 | UUGGUCUUUCACUUUCUUGGGCU |
| AM05136-AS | 260 | usUfgGfuCfuUfuCfaCfuUfuCfuUfgGfgsc | 776 | UUGGUCUUUCACUUUCUUGGGC |
| AM05138-AS | 261 | usUfgGfuCfuUfuCfaCfuUfuCfuUfggsg | 775 | UUGGUCUUUCACUUUCUUGGG |
| AM05140-AS | 262 | usUfgGfuCfuUfuCfaCfuUfuCfuUfgsg | 774 | UUGGUCUUUCACUUUCUUGG |
| AM05142-AS | 263 | usUfgGfuCfuUfuCfaCfuUfuCfuusg | 773 | UUGGUCUUUCACUUUCUUG |
| AM05144-AS | 264 | usGfaAfgCfuGfaGfgCfuCfaAfaGfcAfcUfusc | 705 | UGAAGCUGAGGCUCAAAGCACUUC |
| AM05146-AS | 265 | usGfaAfgCfuGfaGfgCfuCfaAfaGfcAfcusu | 704 | UGAAGCUGAGGCUCAAAGCACUU |
| AM05148-AS | 266 | usGfaAfgCfuGfaGfgCfuCfaAfaGfcAfcsu | 703 | UGAAGCUGAGGCUCAAAGCACU |
| AM05150-AS | 267 | usGfaAfgCfuGfaGfgCfuCfaAfaGfcasc | 702 | UGAAGCUGAGGCUCAAAGCAC |
| AM05152-AS | 268 | usGfaAfgCfuGfaGfgCfuCfaAfaGfcsa | 701 | UGAAGCUGAGGCUCAAAGCA |
| AM05154-AS | 269 | usGfaAfgCfuGfaGfgCfuCfaAfagsc | 700 | UGAAGCUGAGGCUCAAAGC |
| AM05156-AS | 270 | usGfaGfaAfgCfuGfaGfgCfuCfaAfaGfcAfcsu | 713 | UGAGAAGCUGAGGCUCAAAGCACU |
| AM05158-AS | 271 | usGfaGfaAfgCfuGfaGfgCfuCfaAfaGfcasc | 712 | UGAGAAGCUGAGGCUCAAAGCAC |
| AM05160-AS | 272 | usGfaGfaAfgCfuGfaGfgCfuCfaAfaGfcsa | 711 | UGAGAAGCUGAGGCUCAAAGCA |
| AM05162-AS | 273 | usGfaGfaAfgCfuGfaGfgCfuCfaAfagsc | 710 | UGAGAAGCUGAGGCUCAAAGC |
| AM05164-AS | 274 | usGfaGfaAfgCfuGfaGfgCfuCfaAfasg | 709 | UGAGAAGCUGAGGCUCAAAG |
| AM05166-AS | 275 | usGfaGfaAfgCfuGfaGfgCfuCfaasa | 708 | UGAGAAGCUGAGGCUCAAA |
| AM05168-AS | 276 | usUfuUfuCfaAfaGfcAfcUfuUfaUfuGfaGfusu | 819 | UUUUUCAAAGCACUUUAUUGAGUU |
| AM05170-AS | 277 | usUfuUfuCfaAfaGfcAfcUfuUfaUfuGfagsu | 818 | UUUUUCAAAGCACUUUAUUGAGU |
| AM05172-AS | 278 | usUfuUfuCfaAfaGfcAfcUfuUfaUfuGfasg | 817 | UUUUUCAAAGCACUUUAUUGAG |
| AM05174-AS | 279 | usUfuUfuCfaAfaGfcAfcUfuUfaUfugsa | 816 | UUUUUCAAAGCACUUUAUUGA |
| AM05176-AS | 280 | usUfuUfuCfaAfaGfcAfcUfuUfaUfusg | 815 | UUUUUCAAAGCACUUUAUUG |
| AM05178-AS | 281 | usUfuUfuCfaAfaGfcAfcUfuUfausu | 814 | UUUUUCAAAGCACUUUAUU |
| AM05231-AS | 282 | cPrspusGfsgsUfcUfuUfcAfcUfuUfcUfuGfgGfcasuc | 734 | UGGUCUUUCACUUUCUUGGGCAUC |
| AM05232-AS | 283 | cPrspusGfsgsUfcUfuUfcAfcUfuUfcAfuGfgGfsu | 719 | UGGUCUUUCACUUUCAUGGGU |
| AM05246-AS | 284 | usGfsgucuuUfcAfcUfuUfcuugggcsusc | 739 | UGGUCUUUCACUUUCUUGGGCUC |
| AM05247-AS | 285 | usGfsgUfcUfuUfcAfcUfuUfcUfuGfgGfcsusc | 739 | UGGUCUUUCACUUUCUUGGGCUC |
| AM05248-AS | 286 | usGfsgUfcUfuUfcAfcUfuUfcUfuGfgsGfsc | 724 | UGGUCUUUCACUUUCUUGGGC |
| AM05256-AS | 287 | usGfsgucuuUfcAfcUfuUfcuugggcsusccgc | 742 | UGGUCUUUCACUUUCUUGGGCUCCGC |
| AM05257-AS | 288 | usGfsgucuuUfcAfcUfuUfcuugggcsusu | 746 | UGGUCUUUCACUUUCUUGGGCUU |
| AM05261-AS | 289 | usGfsgucuuUfcAfcUfuUfcuuggsusu | 751 | UGGUCUUUCACUUUCUUGGUU |
| AM05262-AS | 290 | usGfsgucuuUfcAfcUfuUfcuuggsgsc | 724 | UGGUCUUUCACUUUCUUGGGC |
| AM05265-AS | 291 | usUfsusuuCfaaaGfcAfcUfuUfauugagsuuca | 821 | UUUUUCAAAGCACUUUAUUGAGUUCA |
| AM05266-AS | 292 | ususUfsUfuCfaAfaGfcAfcUfuUfaUfuGfagsuuca | 821 | UUUUUCAAAGCACUUUAUUGAGUUCA |
| AM05268-AS | 293 | asUfsusUfuCfaAfaGfcAfcUfuUfaUfuGfagsuuca | 670 | AUUUUCAAAGCACUUUAUUGAGUUCA |
| AM05270-AS | 294 | usUfsusUfuCfaAfaGfcAfcUfuUfaUfuGfagsu | 818 | UUUUUCAAAGCACUUUAUUGAGU |
| AM05274-AS | 295 | usUfsusUfuCfaAfaGfcAfcUfuUfaUfugsa | 816 | UUUUUCAAAGCACUUUAUUGA |
| AM05276-AS | 296 | usUfsusUfuCfaAfaGfcAfcUfuUfaUfugsc | 823 | UUUUUCAAAGCACUUUAUUGC |
| AM05278-AS | 297 | usUfsusUfuCfaAfaGfcAfcUfuUfaUfcgsa | 813 | UUUUUCAAAGCACUUUAUCGA |
| AM05279-AS | 298 | usGfsasagCfuGfaGfgCfuCfaaagcusu | 707 | UGAAGCUGAGGCUCAAAGCUU |
| AM05289-AS | 299 | usGfsasAfgCfuGfaGfgCfuCfaAfaGfcasc | 702 | UGAAGCUGAGGCUCAAAGCAC |
| AM05327-AS | 300 | usAfsasGfcAfcUfuUfaUfuGfaGfuUfcCfsu | 673 | UAAGCACUUUAUUGAGUUCCU |
| AM05328-AS | 301 | usAfsasGfcAfcUfuUfaUfuGfaGfuUfcCfusu | 675 | UAAGCACUUUAUUGAGUUCCUU |
| AM05329-AS | 302 | usCfsasAfaGfcAfcUfuUfaUfuGfaGfuUfsc | 680 | UCAAAGCACUUUAUUGAGUUC |
| AM05330-AS | 303 | usCfsasAfaGfcAfcUfuUfaUfuGfaGfuUfcsu | 681 | UCAAAGCACUUUAUUGAGUUCU |
| AM05331-AS | 304 | usUfscsAfaAfgCfaCfuUfuAfuUfgAfgUfsu | 767 | UUCAAAGCACUUUAUUGAGUU |
| AM05332-AS | 305 | usUfscsAfaAfgCfaCfuUfuAfuUfgAfgUfusc | 768 | UUCAAAGCACUUUAUUGAGUUC |
| AM05333-AS | 306 | usUfscsAfaAfgCfaCfuUfuAfuUfgAfgUfuCfsu | 771 | UUCAAAGCACUUUAUUGAGUUCU |
| AM05334-AS | 307 | usUfsusCfaAfaGfcAfcUfaUfaUfuGfaGfsu | 788 | UUUCAAAGCACUAUAUUGAGU |
| AM05335-AS | 308 | asUfsusCfaAfaGfcAfcUfaUfaUfuGfaGfsu | 667 | AUUCAAAGCACUAUAUUGAGU |
| AM05336-AS | 309 | usUfsusCfaAfaGfcAfcUfaUfaUfuGfaGfuUfsc | 789 | UUUCAAAGCACUAUAUUGAGUUC |
| AM05355-AS | 310 | usUfsuUfuCfaAfaGfcAfcUfuUfaUfuGfasgsuuAu | 820 | UUUUUCAAAGCACUUUAUUGAGUUAU |
| AM05358-AS | 311 | usGfsgsUfcUfuUfcAfcUfuUfCfuuGfgGfsc | 724 | UGGUCUUUCACUUUCUUGGGC |
| AM05360-AS | 312 | usGfsgsUfcUfuUfcAfcUfuUfCfuuGfgGfcsa | 725 | UGGUCUUUCACUUUCUUGGGCA |
| AM05367-AS | 313 | usAfsasGfcAfcUfuUfaUfuGfAfguUfcCfsu | 673 | UAAGCACUUUAUUGAGUUCCU |
| AM05370-AS | 314 | usCfsasAfaGfcAfcUfuUfaUfUfgaGfuUfsc | 680 | UCAAAGCACUUUAUUGAGUUC |
| AM05373-AS | 315 | usUfscsAfaAfgCfaCfuUfuAfUfugAfgUfsu | 767 | UUCAAAGCACUUUAUUGAGUU |
| AM05376-AS | 316 | usUfsusCfaAfaGfcAfcUfaUfAfuuGfaGfsu | 788 | UUUCAAAGCACUAUAUUGAGU |
| AM05379-AS | 317 | usUfsusUfuCfaAfaGfcAfcUfuUfaUfuGfsu | 824 | UUUUUCAAAGCACUUUAUUGU |
| AM05382-AS | 318 | usUfsusUfuCfaAfaGfcAfcUfUfuaUfuGfsu | 824 | UUUUUCAAAGCACUUUAUUGU |
| AM05478-AS | 319 | usUfsusCfaAfaGfcAfcUfuUfaUfuGfaGfsu | 793 | UUUCAAAGCACUUUAUUGAGU |
| AM05479-AS | 320 | asUfsusCfaAfaGfcAfcUfuUfaUfuGfaGfsu | 668 | AUUCAAAGCACUUUAUUGAGU |
| AM05480-AS | 321 | usUfsusCfaAfaGfcAfcUfuUfaUfuGfaGfuUfsc | 797 | UUUCAAAGCACUUUAUUGAGUUC |
| AM05482-AS | 322 | usUfsusCfaAfaGfcAfcUfuUfAfuuGfaGfsu | 793 | UUUCAAAGCACUUUAUUGAGU |
| AM05590-AS | 323 | usGfsgucuuUfcAfcUfuUfcuugggscsu | 738 | UGGUCUUUCACUUUCUUGGGCU |
| AM05591-AS | 324 | usGfsgsucuuUfcAfcUfuUfcuugggcusu | 746 | UGGUCUUUCACUUUCUUGGGCUU |
| AM05596-AS | 325 | usGfsgucuuUfcAfcUfuUfcuuggsusc | 750 | UGGUCUUUCACUUUCUUGGUC |
| AM05597-AS | 326 | usGfsgsucuuUfcAfcUfuUfcuuggusu | 751 | UGGUCUUUCACUUUCUUGGUU |
| AM05641-AS | 327 | cPrpusUfsusCfaAfaGfcAfcUfuUfaUfuGfaGfsu | 793 | UUUCAAAGCACUUUAUUGAGU |
| AM05679-AS | 328 | usUfsusCfaAfaGfcAfcUfuUfaUfuGfaGfsc | 790 | UUUCAAAGCACUUUAUUGAGC |
| AM05681-AS | 329 | usUfscsAfaAfgCfaCfuUfuAfuUfgAfgUfsc | 765 | UUCAAAGCACUUUAUUGAGUC |
| AM05683-AS | 330 | usUfscsAfaAfgCfaCfuUfuAfuUfgAfgCfsu | 764 | UUCAAAGCACUUUAUUGAGCU |
| AM05685-AS | 331 | usUfscsAfaAfgCfaCfuUfuAfuUfgAfgCfsc | 763 | UUCAAAGCACUUUAUUGAGCC |
| AM05686-AS | 332 | usUfscsAfaAfgcacuUfuAfuUfgAfgUfsu | 767 | UUCAAAGCACUUUAUUGAGUU |
| AM05687-AS | 333 | usUfscsAfaAfgcAfcuUfuAfuUfgAfgUfsu | 767 | UUCAAAGCACUUUAUUGAGUU |
| AM05689-AS | 334 | usUfscsAfaAfgCfaCfuUfuAfuUfgAfgUfcsc | 766 | UUCAAAGCACUUUAUUGAGUCC |
| AM05691-AS | 335 | usUfscsAfaAfgCfaCfuUfuAfuUfgAfgusu | 767 | UUCAAAGCACUUUAUUGAGUU |
| AM05692-AS | 336 | usUfscsAfaAfgCfaCfuUfuAfuUfgAfgcsc | 763 | UUCAAAGCACUUUAUUGAGCC |
| AM05695-AS | 337 | usUfscsAfaAfgCfAfCfuUfuAfuUfgAfgusu | 767 | UUCAAAGCACUUUAUUGAGUU |
| AM05696-AS | 338 | usUfsuscaAfagcAfcUfuUfaUfuGfaGfsu | 793 | UUUCAAAGCACUUUAUUGAGU |
| AM05698-AS | 339 | usUfsusCfaAfaGfcAfcUfuUfaUfuGfaGfusu | 796 | UUUCAAAGCACUUUAUUGAGUU |
| AM05700-AS | 340 | usUfsusCfaAfaGfcAfcUfuUfaUfuGfaGfcsu | 792 | UUUCAAAGCACUUUAUUGAGCU |
| AM05702-AS | 341 | usUfsusCfaAfaGfcAfcUfuUfaUfuGfaGfusc | 794 | UUUCAAAGCACUUUAUUGAGUC |
| AM05704-AS | 342 | usUfsusCfaAfaGfcAfcUfuUfaUfuGfaGfuCfsc | 795 | UUUCAAAGCACUUUAUUGAGUCC |
| AM05705-AS | 343 | usUfsusCfaAfaGfcAfcUfuUfaUfuGfaGfuusc | 797 | UUUCAAAGCACUUUAUUGAGUUC |
| AM05707-AS | 344 | usUfsusCfaAfaGfcAfcUfuUfaUfuGfagsu | 793 | UUUCAAAGCACUUUAUUGAGU |
| AM05708-AS | 345 | usUfsusCfaAfaGfcAfcUfuUfaUfuGfausu | 798 | UUUCAAAGCACUUUAUUGAUU |
| AM05711-AS | 346 | usUfsusCfaAfaGfcAfcUfuUfaUfuGfaGfcsc | 791 | UUUCAAAGCACUUUAUUGAGCC |
| AM05713-AS | 347 | usUfsusCfaAfaGfCfAfcUfuUfaUfuGfagsu | 793 | UUUCAAAGCACUUUAUUGAGU |
| AM05718-AS | 348 | usUfsusCfaAfaGfCfAfcUfuUfaUfuGfausu | 798 | UUUCAAAGCACUUUAUUGAUU |
| AM05720-AS | 349 | usUfsusCfaAfaGfcAfcUfuUfaUfUfgaGfsu | 793 | UUUCAAAGCACUUUAUUGAGU |
| AM05722-AS | 350 | usUfsusCfaAfaGfcAfcUfuUfaUfUfgaGfuUfsc | 797 | UUUCAAAGCACUUUAUUGAGUUC |
| AM05726-AS | 351 | usAfsasGfcAfcUfuUfaUfuGfaGfuUfcCfsc | 672 | UAAGCACUUUAUUGAGUUCCC |
| AM05728-AS | 352 | asAfsasGfcAfcUfuUfaUfuGfaGfuUfcCfsu | 662 | AAAGCACUUUAUUGAGUUCCU |
| AM05729-AS | 353 | usAfsasgcAfcUfuUfaUfuGfaGfuUfcCfsu | 673 | UAAGCACUUUAUUGAGUUCCU |
| AM05731-AS | 354 | usAfsasGfcAfcUfuUfaUfuGfaGfuUfcCfusg | 674 | UAAGCACUUUAUUGAGUUCCUG |
| AM05733-AS | 355 | usAfsasGfcAfcUfuUfaUfuGfaGfuUfccsu | 673 | UAAGCACUUUAUUGAGUUCCU |
| AM05736-AS | 356 | usAfsasgcAfcUfuUfaUfuGfaGfuUfccsu | 673 | UAAGCACUUUAUUGAGUUCCU |
| AM05742-AS | 357 | usAfsasGfCfaCfUfuUfaUfuGfaGfUfucCfsu | 673 | UAAGCACUUUAUUGAGUUCCU |
| AM05750-AS | 358 | usUfscsAfAfaGfCfaCfuUfuAfuUfGfagUfsu | 767 | UUCAAAGCACUUUAUUGAGUU |
| AM05753-AS | 359 | usUfscsAfAfaGfCfaCfuUfuAfuUfGfagUfusc | 768 | UUCAAAGCACUUUAUUGAGUUC |
| AM05818-AS | 360 | cPrpusGfsgucuuUfcAfcUfuUfcuugggcsusc | 739 | UGGUCUUUCACUUUCUUGGGCUC |
| AM05819-AS | 361 | usGfsgucUfuUfCfacuuUfcUfugggcsusc | 739 | UGGUCUUUCACUUUCUUGGGCUC |
| AM05820-AS | 362 | usGfsgUfcUfuucacUfuUfcUfuGfgGfcsusc | 739 | UGGUCUUUCACUUUCUUGGGCUC |
| AM05823-AS | 363 | usUfscsAfaAfgCfaCfuUfuAfuUfgAfgUfucsc | 769 | UUCAAAGCACUUUAUUGAGUUCC |
| AM05824-AS | 364 | cPrpusUfscsAfaAfgCfaCfuUfuAfuUfgAfgUfucsc | 770 | UUCAAAGCACUUUAUUGAGUUCC |
| AM05825-AS | 365 | usUfscsAfaAfgcacuUfuAfuUfgAfgUfucsc | 770 | UUCAAAGCACUUUAUUGAGUUCC |
| AM05826-AS | 366 | usUfscsaaAfgCfAfcuuuAfuUfgaguucsc | 770 | UUCAAAGCACUUUAUUGAGUUCC |
| AM05836-AS | 367 | usUfsusCfaCfuUfuCfuUfgGfgCfuCfcAfaasc | 800 | UUUCACUUUCUUGGGCUCCAAAC |
| AM05837-AS | 368 | usUfsusCfaCfuuucuUfgGfgCfuCfcAfaasc | 800 | UUUCACUUUCUUGGGCUCCAAAC |
| AM05838-AS | 369 | usUfsuscaCfuUfUfcuUfgGfgCfuccaaasc | 800 | UUUCACUUUCUUGGGCUCCAAAC |
| AM05839-AS | 370 | usUfsuscaCfuuucuUfgGfgCfuCfcAfaasc | 800 | UUUCACUUUCUUGGGCUCCAAAC |
| AM05840-AS | 371 | usUfsusCfaCfuuucuUfgGfgCfuCfcAfsa | 799 | UUUCACUUUCUUGGGCUCCAA |
| AM05841-AS | 372 | usUfsusCfaCfuuucuUfgGfgCfuCfcasa | 799 | UUUCACUUUCUUGGGCUCCAA |
| AM05842-AS | 373 | usUfsusCfaCfuuucuUfgGfgCfuCfcusu | 801 | UUUCACUUUCUUGGGCUCCUU |
| AM05902-AS | 374 | cPrpusUfscsAfaAfgCfaCfuUfuAfuUfgAfgUfsu | 767 | UUCAAAGCACUUUAUUGAGUU |
| AM05903-AS | 375 | usUfsusGfuAfcUfuAfuGfcUfcCfuUfgGfsg | 805 | UUUGUACUUAUGCUCCUUGGG |
| AM05904-AS | 376 | usAfsusCfgAfaAfgUfgUfuGfaCfuCfcAfsa | 676 | UAUCGAAAGUGUUGACUCCAA |
| AM05905-AS | 377 | usUfsgsUfaCfuUfaUfgCfuCfcUfuGfgusu | 787 | UUGUACUUAUGCUCCUUGGUU |
| AM05906-AS | 378 | usCfsgsAfaGfaCfaGfaCfuCfuUfgCfgusu | 688 | UCGAAGACAGACUCUUGCGUU |
| AM05950-AS | 379 | cPrpdUsUfscsAfaAfgCfaCfuUfuAfuUfgAfgUfsu | 767 | UUCAAAGCACUUUAUUGAGUU |
| AM06060-AS | 380 | usUfscsAfaAfgcacuUfuAfuUfgAfgUfucsu | 771 | UUCAAAGCACUUUAUUGAGUUCU |
| AM06061-AS | 381 | usUfscsAfaAfgcacuUfuAfuUfgAfgUfucusu | 772 | UUCAAAGCACUUUAUUGAGUUCUU |
| AM06062-AS | 382 | usUfscsAfaAfgcacuUfuAfuUfgAfgusu | 767 | UUCAAAGCACUUUAUUGAGUU |
| AM06093-AS | 383 | usAfsusCfgAfaAfgUfgUfuGfaCfuCfcusu | 679 | UAUCGAAAGUGUUGACUCCUU |
| AM06094-AS | 384 | cPrpusAfsusCfgAfaAfgUfgUfuGfaCfuCfcusu | 679 | UAUCGAAAGUGUUGACUCCUU |
| AM06095-AS | 385 | usAfsusCfgAfaAfgUfgUfuGfaCfuCfcAfasg | 677 | UAUCGAAAGUGUUGACUCCAAG |
| AM06096-AS | 386 | usUfsgsUfaCfuUfaUfgCfuCfcUfuGfggsg | 784 | UUGUACUUAUGCUCCUUGGGG |
| AM06097-AS | 387 | usUfsusGfuAfcUfuAfuGfcUfcCfuUfgGfgusu | 808 | UUUGUACUUAUGCUCCUUGGGUU |
| AM06103-AS | 388 | cPrpusUfsusGfuAfcUfuAfuGfcUfcCfuUfgGfsg | 805 | UUUGUACUUAUGCUCCUUGGG |
| AM06104-AS | 389 | usUfsusGfuAfcUfuAfuGfcUfcCfuUfgusu | 811 | UUUGUACUUAUGCUCCUUGUU |
| AM06106-AS | 390 | usUfsusGfuAfcuuauGfcUfcCfuUfgGfsg | 805 | UUUGUACUUAUGCUCCUUGGG |
| AM06107-AS | 391 | usUfsusGfuAfcUfuAfuGfcUfcCfuUfgGfgsg | 806 | UUUGUACUUAUGCUCCUUGGGG |
| AM06108-AS | 392 | usUfsusGfuAfcUfuAfuGfcUfcCfuUfgGfggsu | 807 | UUUGUACUUAUGCUCCUUGGGGU |
| AM06113-AS | 393 | usUfsgsUfaCfuuaugCfuCfcUfuGfgGfsg | 784 | UUGUACUUAUGCUCCUUGGGG |
| AM06114-AS | 394 | usUfsgsUfaCfuUfaUfgCfuCfcUfuGfgGfusu | 786 | UUGUACUUAUGCUCCUUGGGUU |
| AM06117-AS | 395 | usAfsusCfgAfaAfgUfgUfuGfaCfuCfcAfagsc | 678 | UAUCGAAAGUGUUGACUCCAAGC |
| AM06334-AS | 396 | usUfsusGfuAfcUfuAfuGfcUfcCfuUfggsg | 805 | UUUGUACUUAUGCUCCUUGGG |
| AM06335-AS | 397 | usUfuGfuacUfuAfuGfcUfccuuggsg | 805 | UUUGUACUUAUGCUCCUUGGG |
| AM06336-AS | 398 | usUfsusGfuAfcUfuAfuGfcUfcCfuUfggsu | 809 | UUUGUACUUAUGCUCCUUGGU |
| AM06337-AS | 399 | usUfsusGfuAfcUfuAfuGfcUfcCfuUfgsg | 803 | UUUGUACUUAUGCUCCUUGG |
| AM06338-AS | 400 | cPrpdUsUfsusGfuAfcUfuAfuGfcUfcCfuUfgusu | 811 | UUUGUACUUAUGCUCCUUGUU |
| AM06339-AS | 401 | cPrpdUUfuGfuAfcUfuAfuGfcUfcCfuUfgu(invAb) | 810 | UUUGUACUUAUGCUCCUUGU |
| AM06343-AS | 402 | usUfsusGfuAfcUfuAfuGfcUfcCfuUfsg | 802 | UUUGUACUUAUGCUCCUUG |
| AM06344-AS | 403 | asUfsusGfuAfcUfuAfuGfcUfcCfuUfgGfsg | 669 | AUUGUACUUAUGCUCCUUGGG |
| AM07041-AS | 404 | usUfsusGfuAfcUfuAfuGfcUfcCfuUfgGfsc | 804 | UUUGUACUUAUGCUCCUUGGC |
| AM07043-AS | 405 | usUfsusGfuAfC_{UNA}UfuAfuGfcUfcCfuUfgGfsc | 804 | UUUGUACUUAUGCUCCUUGGC |
| AM07046-AS | 406 | usUfsgsUfaCfuUfaUfgCfuCfcUfuGfggsc | 783 | UUGUACUUAUGCUCCUUGGGC |
| AM07048-AS | 407 | usGfsusAfcUfuAfuGfcUfcCfuUfgGfgGfsc | 753 | UGUACUUAUGCUCCUUGGGGC |
| AM07050-AS | 408 | usUfsusUfgUfaCfuUfaUfgCfuCfcUfuGfsc | 812 | UUUUGUACUUAUGCUCCUUGC |
| AM07052-AS | 409 | usCfsusUfuGfuAfcUfuAfuGfcUfcCfucsg | 699 | UCUUUGUACUUAUGCUCCUCG |
| AM07054-AS | 410 | usCfsusUfuGfuAfcUfuAfuGfcUfcCfucsc | 698 | UCUUUGUACUUAUGCUCCUCC |
| AM07056-AS | 411 | usCfsasGfcUfuUfgUfaCfuUfaUfgCfuCfsc | 685 | UCAGCUUUGUACUUAUGCUCC |
| AM07058-AS | 412 | usGfsgsUfaCfaUfuUfgUfgGfuAfcAfgCfsu | 718 | UGGUACAUUUGUGGUACAGCU |
| AM07249-AS | 413 | usUfsusGfuAfcUfuAfuGfcUfcCfuUfgGfsu | 809 | UUUGUACUUAUGCUCCUUGGU |
| AM07253-AS | 414 | asUfscsGfaAfaguguUfgAfcUfcCfaAfsg | 665 | AUCGAAAGUGUUGACUCCAAG |
| AM07255-AS | 415 | asUfscsGfaAfaguguUfgAfcUfcCfaGfsc | 666 | AUCGAAAGUGUUGACUCCAGC |
| AM07257-AS | 416 | asAfsusCfgAfaagugUfuGfaCfuCfcAfsc | 663 | AAUCGAAAGUGUUGACUCCAC |
| AM07259-AS | 417 | usGfsgsAfaUfcgaaaGfuGfuUfgAfcUfsc | 717 | UGGAAUCGAAAGUGUUGACUC |
| AM07261-AS | 418 | usGfsusAfcAfuuuguGfgUfaCfaGfcUfsg | 752 | UGUACAUUUGUGGUACAGCUG |
| AM07263-AS | 419 | usCfsasGfcUfuuguaCfuUfaUfgCfuCfsg | 686 | UCAGCUUUGUACUUAUGCUCG |
| AM07265-AS | 420 | usCfsasGfcUfuuguaCfuUfaUfgCfuCfsu | 687 | UCAGCUUUGUACUUAUGCUCU |
| AM07267-AS | 421 | usUfsgsUfaCfuuaugCfuCfcUfuGfgGfsu | 785 | UUGUACUUAUGCUCCUUGGGU |

**Table 4. FXII RNAi Agent Sense Strand Sequences.**

| **Sense Strand ID:** | **SEQ ID NO.** | **Sense Sequence (Modified) (5' → 3')** | **SEQ ID NO.** | **Underlying Sense Base Sequence (5' → 3')** |
|---|---|---|---|---|
| AM04130-SS | 422 | (NAG25)uauaugscsccaagaAfaGfugaaagacc(invdA) | 932 | UAUAUGCCCAAGAAAGUGAAAGACCA |
| AM04554-SS | 423 | (NAG25)gcgagscccaagaAfaGfugaaagacca(invdA) | 902 | GCGAGCCCAAGAAAGUGAAAGACCAA |
| AM04557-SS | 424 | (NAG25)gcgagscccaagaAfaGfugaaagaccas(invdA) | 902 | GCGAGCCCAAGAAAGUGAAAGACCAA |
| AM04558-SS | 425 | (NAG25)gcgagscccaagaAfaGfugaaagaccAMs(invdA) | 902 | GCGAGCCCAAGAAAGUGAAAGACCAA |
| AM04559-SS | 426 | (NAG25)gcgagscccaagaAfaGfugaaagaccasas(invdA) | 903 | GCGAGCCCAAGAAAGUGAAAGACCAAA |
| AM04561-SS | 427 | (NAG25)gcgugsuuuggagCfCfCfaagaaagugAMs(invdA) | 909 | GCGUGUUUGGAGCCCAAGAAAGUGAA |
| AM04613-SS | 428 | (NAG25)gcgaugscsccaagaAfaGfugaaagacc(invdA) | 905 | GCGAUGCCCAAGAAAGUGAAAGACCA |
| AM04814-SS | 429 | (NAG25)gcgagscccaagaAfaGfugaaagacc(invdA) | 901 | GCGAGCCCAAGAAAGUGAAAGACCA |
| AM04815-SS | 430 | (NAG25)gcgugsuuuggagCfCfCfaagaaagug(invdA) | 908 | GCGUGUUUGGAGCCCAAGAAAGUGA |
| AM04836-SS | 431 | (NAG25)gcgaugscccaagaAfaGfugaaagacc(invdA) | 905 | GCGAUGCCCAAGAAAGUGAAAGACCA |
| AM04837-SS | 432 | (NAG25)gcgaugscccaagaAfaGfugaaagacc(invdT) | 906 | GCGAUGCCCAAGAAAGUGAAAGACCT |
| AM04838-SS | 433 | (NAG25)gcgaugscccaagaAfaGfugaaagacca | 905 | GCGAUGCCCAAGAAAGUGAAAGACCA |
| AM04839-SS | 434 | (NAG25)gcgaugscccaagaAfaGfugaaagaccsa | 905 | GCGAUGCCCAAGAAAGUGAAAGACCA |
| AM04840-SS | 435 | (NAG25)gcgaugscccaagaAfAfGfugaaagacc(invdA) | 905 | GCGAUGCCCAAGAAAGUGAAAGACCA |
| AM04845-SS | 436 | (NAG25)gcgaugscccaagaAfaGfugaaagacc(invAb) | 904 | GCGAUGCCCAAGAAAGUGAAAGACC |
| AM04847-SS | 437 | (NAG25)gcgaugscccaagaAfaGfugaaagacc(inva) | 905 | GCGAUGCCCAAGAAAGUGAAAGACCA |
| AM04881-SS | 438 | (NAG25)gcgaugscccaagaAfaGfugaaagaccas(invAb) | 905 | GCGAUGCCCAAGAAAGUGAAAGACCA |
| AM04882-SS | 439 | (NAG25)sugauuggagcccaAfGfAfaagugaaags(invdA) | 947 | UGAUUGGAGCCCAAGAAAGUGAAAGA |
| AM04883-SS | 440 | (NAG25)sagcccaAfGfAfaagugaaagauus(invAb) | 851 | AGCCCAAGAAAGUGAAAGAUU |
| AM04884-SS | 441 | (NAG25)saaggagcccaAfGfAfaagugaaagauus(invAb) | 836 | AAGGAGCCCAAGAAAGUGAAAGAUU |
| AM04885-SS | 442 | (NAG25)sugaggagcccaagAfAfAfgugaaagacs(invdA) | 944 | UGAGGAGCCCAAGAAAGUGAAAGACA |
| AM04886-SS | 443 | (NAG25)scccaagAfAfAfgugaaagacauus(invAb) | 867 | CCCAAGAAAGUGAAAGACAUU |
| AM04887-SS | 444 | (NAG25)saaagcccaagAfAfAfgugaaagacauus(invAb) | 826 | AAAGCCCAAGAAAGUGAAAGACAUU |
| AM04888-SS | 445 | (NAG25)sugaagcccaagaaAfGfUfgaaagaccas(invdA) | 942 | UGAAGCCCAAGAAAGUGAAAGACCAA |
| AM04889-SS | 446 | (NAG25)scaagaaAfGfUfgaaagaccaauus(invAb) | 859 | CAAGAAAGUGAAAGACCAAUU |
| AM04890-SS | 447 | (NAG25)saacccaagaaAfGfUfgaaagaccaauus(invAb) | 828 | AACCCAAGAAAGUGAAAGACCAAUU |
| AM04891-SS | 448 | (NAG25)sugaaagugcuuugAfGfCfcucagcuucs(invdA) | 938 | UGAAAGUGCUUUGAGCCUCAGCUUCA |
| AM04892-SS | 449 | (NAG25)sgcuuugAfGfCfcucagcuucauus(invAb) | 914 | GCUUUGAGCCUCAGCUUCAUU |
| AM04893-SS | 450 | (NAG25)saagugcuuugAfGfCfcucagcuucauus(invAb) | 838 | AAGUGCUUUGAGCCUCAGCUUCAUU |
| AM04894-SS | 451 | (NAG25)sugagugcuuugagCfCfUfcagcuucucs(invdA) | 946 | UGAGUGCUUUGAGCCUCAGCUUCUCA |
| AM04895-SS | 452 | (NAG25)suuugagCfCfUfcagcuucucauus(invAb) | 951 | UUUGAGCCUCAGCUUCUCAUU |
| AM04896-SS | 453 | (NAG25)saagcuuugagCfCfUfcagcuucucauus(invAb) | 835 | AAGCUUUGAGCCUCAGCUUCUCAUU |
| AM04897-SS | 454 | (NAG25)sugaacucaauaaaGfUfGfcuuugaaaas(invdA) | 939 | UGAACUCAAUAAAGUGCUUUGAAAAA |
| AM04898-SS | 455 | (NAG25)saauaaaGfUfGfcuuugaaaaauus(invAb) | 839 | AAUAAAGUGCUUUGAAAAAUU |
| AM04899-SS | 456 | (NAG25)saaucaauaaaGfUfGfcuuugaaaaauus(invAb) | 840 | AAUCAAUAAAGUGCUUUGAAAAAUU |
| AM04900-SS | 457 | (NAG25)sugagagcccaagaAfAfGfugaaagaccs(invdA) | 943 | UGAGAGCCCAAGAAAGUGAAAGACCA |
| AM04901-SS | 458 | (NAG25)sccaagaAfAfGfugaaagaccauus(invAb) | 864 | CCAAGAAAGUGAAAGACCAUU |
| AM04902-SS | 459 | (NAG25)saagcccaagaAfAfGfugaaagaccauus(invAb) | 833 | AAGCCCAAGAAAGUGAAAGACCAUU |
| AM04931-SS | 460 | (NAG25)sugagagcccaagaAfaGfugaaagaccs(invdA) | 943 | UGAGAGCCCAAGAAAGUGAAAGACCA |
| AM04933-SS | 461 | (NAG25)sugagagcccaagaAfaGfugaaagacc(invdA) | 943 | UGAGAGCCCAAGAAAGUGAAAGACCA |
| AM04934-SS | 462 | (NAG25)ugagagcccaagaAfaGfugaaagacc(invdA) | 943 | UGAGAGCCCAAGAAAGUGAAAGACCA |
| AM04935-SS | 463 | (NAG25)ugagasgcccaagaAfaGfugaaagacc(invdA) | 943 | UGAGAGCCCAAGAAAGUGAAAGACCA |
| AM04937-SS | 464 | (NAG25)uauaugscccaagaAfaGfugaaagacc(invdA) | 933 | UAUAUGCCCAAGAAAGUGAAAGACCA |
| AM04939-SS | 465 | (NAG25)ugaaugscccaagaAfaGfugaaagacc(invdA) | 943 | UGAAUGCCCAAGAAAGUGAAAGACCA |
| AM04940-SS | 466 | (NAG25)ugagugscccaagaAfaGfugaaagacc(invdA) | 945 | UGAGUGCCCAAGAAAGUGAAAGACCA |
| AM04942-SS | 467 | (NAG25)gcggugscccaagaAfaGfugaaagacc(invdA) | 907 | GCGGUGCCCAAGAAAGUGAAAGACCA |
| AM04944-SS | 468 | (NAG25)ugagugscccaagaAfAfGfugaaagacc(invdA) | 945 | UGAGUGCCCAAGAAAGUGAAAGACCA |
| AM04946-SS | 469 | (NAG25)gcggugscccaagaAfAfGfugaaagacc(invdA) | 907 | GCGGUGCCCAAGAAAGUGAAAGACCA |
| AM04948-SS | 470 | (NAG25)uauaugscccaagaAfAfGfugaaagacc(invdA) | 933 | UAUAUGCCCAAGAAAGUGAAAGACCA |
| AM04950-SS | 471 | (NAG25)uuggugscccaagaAfaGfugaaagacc(invdA) | 950 | UUGGUGCCCAAGAAAGUGAAAGACCA |
| AM04952-SS | 472 | (NAG25)aaggugscccaagaAfaGfugaaagacc(invdA) | 837 | AAGGUGCCCAAGAAAGUGAAAGACCA |
| AM04954-SS | 473 | (NAG25)guggugscccaagaAfaGfugaaagacc(invdA) | 931 | GUGGUGCCCAAGAAAGUGAAAGACCA |
| AM04956-SS | 474 | (NAG25)gaggugscccaagaAfaGfugaaagacc(invdA) | 892 | GAGGUGCCCAAGAAAGUGAAAGACCA |
| AM04958-SS | 475 | (NAG25)saagcccaagaAfaGfugaaagacc(invdA)uu | 833 | AAGCCCAAGAAAGUGAAAGACCAUU |
| AM04960-SS | 476 | (NAG25)saagcccaagaAfaGfugaaagacc(invdA)usu | 833 | AAGCCCAAGAAAGUGAAAGACCAUU |
| AM04961-SS | 477 | (NAG25)saagcccaagaAfaGfugaaagacc(invdA)usu(invAb) | 833 | AAGCCCAAGAAAGUGAAAGACCAUU |
| AM04962-SS | 478 | (NAG25)saagcccaagaAfaGfugaaagacc(invdA) | 832 | AAGCCCAAGAAAGUGAAAGACCA |
| AM04963-SS | 479 | (NAG25)saagcccaagaAfaGfugaaagaccs(invdA) | 832 | AAGCCCAAGAAAGUGAAAGACCA |
| AM04964-SS | 480 | (NAG25)saagcccaagaAfaGfugaaagacc(invdA)(invAb) | 832 | AAGCCCAAGAAAGUGAAAGACCA |
| AM04965-SS | 481 | (NAG25)saagcccaagaAfaGfugaaagacc(invdA)s(invAb) | 832 | AAGCCCAAGAAAGUGAAAGACCA |
| AM04966-SS | 482 | (NAG25)saagcccaagaAfaGfugaaagaccausu(invAb) | 833 | AAGCCCAAGAAAGUGAAAGACCAUU |
| AM04967-SS | 483 | (NAG25)saagcccaagaAfaGfugaaagacca(invAb) | 832 | AAGCCCAAGAAAGUGAAAGACCA |
| AM04968-SS | 484 | (NAG25)saagcccaagaAfaGfugaaagaccas(invAb) | 832 | AAGCCCAAGAAAGUGAAAGACCA |
| AM04974-SS | 485 | (NAG25)sccaagaAfaGfugaaagaccauus(invAb) | 864 | CCAAGAAAGUGAAAGACCAUU |
| AM04975-SS | 486 | (NAG25)saagcccaagaAfaGfugaaagaccauus(invAb) | 833 | AAGCCCAAGAAAGUGAAAGACCAUU |
| AM04980-SS | 487 | (NAG25)ugauusggagcccaAfGfAfaagugaaags(invdA) | 947 | UGAUUGGAGCCCAAGAAAGUGAAAGA |
| AM04981-SS | 488 | (NAG25)ugaggsagcccaagAfAfAfgugaaagacs(invdA) | 944 | UGAGGAGCCCAAGAAAGUGAAAGACA |
| AM04982-SS | 489 | (NAG25)ugaagscccaagaaAfGfUfgaaagaccas(invdA) | 942 | UGAAGCCCAAGAAAGUGAAAGACCAA |
| AM04983-SS | 490 | (NAG25)ugaaasgugcuuugAfGfCfcucagcuucs(invdA) | 938 | UGAAAGUGCUUUGAGCCUCAGCUUCA |
| AM04984-SS | 491 | (NAG25)ugagusgcuuugagCfCfUfcagcuucucs(invdA) | 946 | UGAGUGCUUUGAGCCUCAGCUUCUCA |
| AM04985-SS | 492 | (NAG25)ugaacsucaauaaaGfUfGfcuuugaaaas(invdA) | 939 | UGAACUCAAUAAAGUGCUUUGAAAAA |
| AM04986-SS | 493 | (NAG25)ugagasgcccaagaAfAfGfugaaagaccs(invdA) | 943 | UGAGAGCCCAAGAAAGUGAAAGACCA |
| AM04987-SS | 494 | (NAG25)ugagasgcccaagaAfaGfugaaagaccs(invdA) | 943 | UGAGAGCCCAAGAAAGUGAAAGACCA |
| AM05030-SS | 495 | (NAG25)aagcccaagaAfAfGfugaaagaccauus(invAb) | 833 | AAGCCCAAGAAAGUGAAAGACCAUU |
| AM05031-SS | 496 | (NAG25)aasgcccaagaAfAfGfugaaagaccauus(invAb) | 833 | AAGCCCAAGAAAGUGAAAGACCAUU |
| AM05032-SS | 497 | (NAG25)saasgcccaagaAfAfGfugaaagaccauus(invAb) | 833 | AAGCCCAAGAAAGUGAAAGACCAUU |
| AM05065-SS | 498 | (NAG25)sascacaagaAfAfGfugaaagaccas(invAb) | 843 | ACACAAGAAAGUGAAAGACCA |
| AM05245-SS | 499 | (NAG25)gscccaagaAfAfGfugaaagacc(invdA) | 898 | GCCCAAGAAAGUGAAAGACCA |
| AM05249-SS | 500 | (NAG25)gcggagscccaagaAfAfGfugaaagacc(invdA) | 907 | GCGGAGCCCAAGAAAGUGAAAGACCA |
| AM05251-SS | 501 | (NAG25)aagscccaagaAfAfGfugaaagacc(invdA) | 832 | AAGCCCAAGAAAGUGAAAGACCA |
| AM05252-SS | 502 | (NAG25)gagscccaagaAfAfGfugaaagacc(invdA) | 891 | GAGCCCAAGAAAGUGAAAGACCA |
| AM05254-SS | 503 | (NAG25)sgscccaagaAfAfGfugaaagacc(invdA) | 898 | GCCCAAGAAAGUGAAAGACCA |
| AM05255-SS | 504 | (NAG25)sasaccaagaAfAfGfugaaagacc(invdA) | 827 | AACCAAGAAAGUGAAAGACCA |
| AM05259-SS | 505 | (NAG31)aagscccaagaAfAfGfugaaagacc(invdA) | 832 | AAGCCCAAGAAAGUGAAAGACCA |
| AM05267-SS | 506 | (NAG25)ugaacsucaauaaaGfUfGfcuuugaaaas(invdT) | 941 | UGAACUCAAUAAAGUGCUUUGAAAAT |
| AM05269-SS | 507 | (NAG25)csucaauaaaGfUfGfcuuugaaaas(invdA) | 878 | CUCAAUAAAGUGCUUUGAAAAA |
| AM05271-SS | 508 | (NAG31)csucaauaaaGfUfGfcuuugaaaas(invdA) | 878 | CUCAAUAAAGUGCUUUGAAAAA |
| AM05272-SS | 509 | (NAG25)asucaauaaaGfUfGfcuuugaaaas(invdA) | 858 | AUCAAUAAAGUGCUUUGAAAAA |
| AM05273-SS | 510 | (NAG25)suscaauaaaGfUfGfcuuugaaaas(invdA) | 935 | UCAAUAAAGUGCUUUGAAAAA |
| AM05275-SS | 511 | (NAG25)sgscaauaaaGfUfGfcuuugaaaas(invdA) | 895 | GCAAUAAAGUGCUUUGAAAAA |
| AM05277-SS | 512 | (NAG25)suscgauaaaGfUfGfcuuugaaaas(invdA) | 937 | UCGAUAAAGUGCUUUGAAAAA |
| AM05282-SS | 513 | (NAG25)sgcuuugAfGfCfcucagcuuc(invdA) | 913 | GCUUUGAGCCUCAGCUUCA |
| AM05283-SS | 514 | (NAG25)s(invAb)sgcuuugAfGfCfcucagcuuc(invdA) | 913 | GCUUUGAGCCUCAGCUUCA |
| AM05284-SS | 515 | (NAG25)sasagcuuugAfGfCfcucagcuuc(invdA) | 834 | AAGCUUUGAGCCUCAGCUUCA |
| AM05286-SS | 516 | (NAG25)sasagcuuugAfGfCfcucagcuucas(invAb) | 834 | AAGCUUUGAGCCUCAGCUUCA |
| AM05287-SS | 517 | (NAG3 1)sasagcuuugAfGfCfcucagcuuc(invdA) | 834 | AAGCUUUGAGCCUCAGCUUCA |
| AM05288-SS | 518 | (NAG25)sgsugcuuugAfGfCfcucagcuuc(invdA) | 929 | GUGCUUUGAGCCUCAGCUUCA |
| AM05317-SS | 519 | (NAG25)sasggaacucAfAfUfaaagugcuuas(invAb) | 855 | AGGAACUCAAUAAAGUGCUUA |
| AM05318-SS | 520 | (NAG25)sgsgaacucAfAfUfaaagugcuuas(invAb) | 915 | GGAACUCAAUAAAGUGCUUA |
| AM05319-SS | 521 | (NAG25)sgsaacucaaUfAfAfagugcuuugas(invAb) | 884 | GAACUCAAUAAAGUGCUUUGA |
| AM05320-SS | 522 | (NAG25)sasgaacucaaUfAfAfagugcuuugas(invAb) | 848 | AGAACUCAAUAAAGUGCUUUGA |
| AM05321-SS | 523 | (NAG25)sasacucaauAfAfAfgugcuuugaas(invAb) | 829 | AACUCAAUAAAGUGCUUUGAA |
| AM05322-SS | 524 | (NAG25)sgsaacucaauAfAfAfgugcuuugaas(invAb) | 885 | GAACUCAAUAAAGUGCUUUGAA |
| AM05323-SS | 525 | (NAG25)sasgaacucaauAfAfAfgugcuuugaas(invAb) | 849 | AGAACUCAAUAAAGUGCUUUGAA |
| AM05324-SS | 526 | (NAG25)sascucaauaAfAfGfugcuuugaaas(invAb) | 846 | ACUCAAUAAAGUGCUUUGAAA |
| AM05325-SS | 527 | (NAG25)sascucaauaAfAfGfugcuuugaaus(invAb) | 847 | ACUCAAUAAAGUGCUUUGAAU |
| AM05326-SS | 528 | (NAG25)sgsaacucaauaAfAfGfugcuuugaaas(invAb) | 886 | GAACUCAAUAAAGUGCUUUGAAA |
| AM05366-SS | 529 | (NAG25)sasgGfaAfCfucAfaUfaAfaGfuGfcUfuas(invAb) | 855 | AGGAACUCAAUAAAGUGCUUA |
| AM05369-SS | 530 | (NAG25)sgsaAfcUfCfaaUfaAfaGfuGfcUfuUfgas(invAb) | 884 | GAACUCAAUAAAGUGCUUUGA |
| AM05372-SS | 531 | (NAG25)sasaCfuCfAfauAfaAfgUfgCfuUfuGfaas(invAb) | 829 | AACUCAAUAAAGUGCUUUGAA |
| AM05375-SS | 532 | (NAG25)sascucAfAfuaAfaGfuGfcUfuUfgAfaas(invAb) | 846 | ACUCAAUAAAGUGCUUUGAAA |
| AM05378-SS | 533 | (NAG25)sascaauaaaGfUfGfcuuugaaaaa(invAb) | 841 | ACAAUAAAGUGCUUUGAAAAA |
| AM05380-SS | 534 | (NAG25)sascAfaUfAfaaGfuGfcUfuUfgAfaAfaa(invAb) | 841 | ACAAUAAAGUGCUUUGAAAAA |
| AM05384-SS | 535 | (NAG25)ugaacsucaauaaaGfUfGfcuuugaaaas(invdA)uu | 940 | UGAACUCAAUAAAGUGCUUUGAAAAAUU |
| AM05385-SS | 536 | (NAG25)sascaauaaaGfUfGfcuuugaaaa(invdA) | 841 | ACAAUAAAGUGCUUUGAAAAA |
| AM05386-SS | 537 | (NAG25)sascaauaaaGfUfGfcuuugaaaa(invdA)uu | 842 | ACAAUAAAGUGCUUUGAAAAAUU |
| AM05389-SS | 538 | (NAG25)sasggaacucAfAfUfaaagugcuu(invdA)uu | 856 | AGGAACUCAAUAAAGUGCUUAUU |
| AM05390-SS | 539 | (NAG25)sasacucaauAfAfAfgugcuuuga(invdA)uu | 831 | AACUCAAUAAAGUGCUUUGAAUU |
| AM05534-SS | 540 | (NAG31)aagscccaagaAfAfGfugaaagaccas(invAb) | 832 | AAGCCCAAGAAAGUGAAAGACCA |
| AM05535-SS | 541 | (NAG25)sgscccaagaAfAfGfugaaagaccas(invAb) | 898 | GCCCAAGAAAGUGAAAGACCA |
| AM05536-SS | 542 | (NAG25)sasaccaagaAfAfGfugaaagaccas(invAb) | 827 | AACCAAGAAAGUGAAAGACCA |
| AM05537-SS | 543 | (NAG25)csucaauaaaGfUfGfcuuugaaaaas(invAb) | 878 | CUCAAUAAAGUGCUUUGAAAAA |
| AM05538-SS | 544 | (NAG31)csucaauaaaGfUfGfcuuugaaaaas(invAb) | 878 | CUCAAUAAAGUGCUUUGAAAAA |
| AM05539-SS | 545 | (NAG25)asucaauaaaGfUfGfcuuugaaaaas(invAb) | 858 | AUCAAUAAAGUGCUUUGAAAAA |
| AM05540-SS | 546 | (NAG25)suscaauaaaGfUfGfcuuugaaaaas(invAb) | 935 | UCAAUAAAGUGCUUUGAAAAA |
| AM05541-SS | 547 | (NAG25)sgscaauaaaGfUfGfcuuugaaaaas(invAb) | 895 | GCAAUAAAGUGCUUUGAAAAA |
| AM05542-SS | 548 | (NAG25)suscgauaaaGfUfGfcuuugaaaaas(invAb) | 937 | UCGAUAAAGUGCUUUGAAAAA |
| AM05587-SS | 549 | (NAG25)s(invAb)sgcccaagaAfAfGfugaaagaccas(invAb) | 898 | GCCCAAGAAAGUGAAAGACCA |
| AM05588-SS | 550 | (NAG25)sgscccaagaAfAfGfugaaagaccAMs(invAb) | 898 | GCCCAAGAAAGUGAAAGACCA |
| AM05589-SS | 551 | (NAG25)sgscccaagaAfAfGfugaaagacmCMAMs(invAb) | 898 | GCCCAAGAAAGUGAAAGACCA |
| AM05592-SS | 552 | (NAG25)s(invAb)saaccaagaAfAfGfugaaagaccas(invAb) | 827 | AACCAAGAAAGUGAAAGACCA |
| AM05593-SS | 553 | (NAG25)sasaccaagaAfAfGfugaaagaccAMs(invAb) | 827 | AACCAAGAAAGUGAAAGACCA |
| AM05594-SS | 554 | (NAG25)sasaccaagaAfAfGfugaaagacmCMAMs(invAb) | 827 | AACCAAGAAAGUGAAAGACCA |
| AM05595-SS | 555 | (NAG25)sgsaccaagaAfAfGfugaaagaccas(invAb) | 888 | GACCAAGAAAGUGAAAGACCA |
| AM05678-SS | 556 | (NAG25)sgscucaauaAfAfGfugcuuugaaas(invAb) | 910 | GCUCAAUAAAGUGCUUUGAAA |
| AM05680-SS | 557 | (NAG25)sgsacucaauAfAfAfgugcuuugaas(invAb) | 889 | GACUCAAUAAAGUGCUUUGAA |
| AM05682-SS | 558 | (NAG25)sasgcucaauAfAfAfgugcuuugaas(invAb) | 852 | AGCUCAAUAAAGUGCUUUGAA |
| AM05684-SS | 559 | (NAG25)sgsgcucaauAfAfAfgugcuuugaas(invAb) | 926 | GGCUCAAUAAAGUGCUUUGAA |
| AM05688-SS | 560 | (NAG25)sgsgacucaauAfAfAfgugcuuugaas(invAb) | 918 | GGACUCAAUAAAGUGCUUUGAA |
| AM05690-SS | 561 | (NAG25)s(invAb)scucaauAfAfAfgugcuuugaas(invAb) | 877 | CUCAAUAAAGUGCUUUGAA |
| AM05693-SS | 562 | (NAG25)s(invAb)scucaauAfAfAfgugcuuugaausu(invAb) | 879 | CUCAAUAAAGUGCUUUGAAUU |
| AM05694-SS | 563 | (NAG25)s(invAb)sCfuCfaAfuAfAfAfgUfgCfuUfuGfaausu (invAb) | 879 | CUCAAUAAAGUGCUUUGAAUU |
| AM05697-SS | 564 | (NAG25)sasacucaauaAfAfGfugcuuugaaas(invAb) | 830 | AACUCAAUAAAGUGCUUUGAAA |
| AM05699-SS | 565 | (NAG25)sasgcucaauaAfAfGfugcuuugaaas(invAb) | 853 | AGCUCAAUAAAGUGCUUUGAAA |
| AM05701-SS | 566 | (NAG25)sgsacucaauaAfAfGfugcuuugaaas(invAb) | 890 | GACUCAAUAAAGUGCUUUGAAA |
| AM05703-SS | 567 | (NAG25)sgsgacucaauaAfAfGfugcuuugaaas(invAb) | 919 | GGACUCAAUAAAGUGCUUUGAAA |
| AM05706-SS | 568 | (NAG25)s(invAb)sucaauaAfAfGfugcuuugaaas(invAb) | 934 | UCAAUAAAGUGCUUUGAAA |
| AM05709-SS | 569 | (NAG25)s(invAb)sucaauaAfAfGfugcuuugaaausu(invAb) | 936 | UCAAUAAAGUGCUUUGAAAUU |
| AM05710-SS | 570 | (NAG25)sgsgcucaauaAfAfGfugcuuugaaas(invAb) | 927 | GGCUCAAUAAAGUGCUUUGAAA |
| AM05712-SS | 571 | (NAG25)s(invAb)sUfcAfaUfaAfAfGfuGfcUfuUfgAfaausu (invAb) | 936 | UCAAUAAAGUGCUUUGAAAUU |
| AM05716-SS | 572 | (NAG25)s(invAb)sucaauaAfAfGfugcuuugaaauus(invAb) | 936 | UCAAUAAAGUGCUUUGAAAUU |
| AM05717-SS | 573 | (NAG25)s(invAb)sUfcAfaUfaAfAfGfuGfcUfuUfgAfaauus (invAb) | 936 | UCAAUAAAGUGCUUUGAAAUU |
| AM05719-SS | 574 | (NAG25)sascucaAfuaAfAfGfuGfcuuUfgAfaas(invAb) | 846 | ACUCAAUAAAGUGCUUUGAAA |
| AM05721-SS | 575 | (NAG25)sgsaacucaAfuaAfAfGfuGfcuuUfgAfaas(invAb) | 886 | GAACUCAAUAAAGUGCUUUGAAA |
| AM05723-SS | 576 | (NAG31)sascucaauaAfAfGfugcuuugaaas(invAb) | 846 | ACUCAAUAAAGUGCUUUGAAA |
| AM05724-SS | 577 | (NAG25)s(invAb)sacucaauaAfAfGfugcuuugaaas(invAb) | 846 | ACUCAAUAAAGUGCUUUGAAA |
| AM05725-SS | 578 | (NAG25)sgsggaacucAfAfUfaaagugcuuas(invAb) | 928 | GGGAACUCAAUAAAGUGCUUA |
| AM05727-SS | 579 | (NAG25)sasggaacucAfAfUfaaagugcuuus(invAb) | 857 | AGGAACUCAAUAAAGUGCUUU |
| AM05730-SS | 580 | (NAG25)scsaggaacucAfAfUfaaagugcuuas(invAb) | 863 | CAGGAACUCAAUAAAGUGCUUA |
| AM05732-SS | 581 | (NAG25)s(invAb)sgaacucAfAfUfaaagugcuuauus(invAb) | 883 | GAACUCAAUAAAGUGCUUAUU |
| AM05734-SS | 582 | (NAG25)s(invAb)sGfaacucAfAfUfaaagugcUfuausu(invAb ) | 883 | GAACUCAAUAAAGUGCUUAUU |
| AM05735-SS | 583 | (NAG25)s(invAb)sGfaAfcUfcAfAfUfaAfagugcUfuausu(in vAb) | 883 | GAACUCAAUAAAGUGCUUAUU |
| AM05738-SS | 584 | (NAG31)sasggaacucAfAfUfaaagugcuuas(invAb) | 855 | AGGAACUCAAUAAAGUGCUUA |
| AM05739-SS | 585 | (NAG25)s(invAb)saggaacucAfAfUfaaagugcuuas(invAb) | 855 | AGGAACUCAAUAAAGUGCUUA |
| AM05740-SS | 586 | (NAG25)saggaacucAfAfUfaaagugcuuas(invAb) | 855 | AGGAACUCAAUAAAGUGCUUA |
| AM05741-SS | 587 | (NAG25)sasggaaCfucAfAfUfaAfaguGfcUfuas(invAb) | 855 | AGGAACUCAAUAAAGUGCUUA |
| AM05743-SS | 588 | (NAG25)sasgGfaacucAfAfUfaaagugcuuas(invAb) | 855 | AGGAACUCAAUAAAGUGCUUA |
| AM05748-SS | 589 | (NAG31)sasacucaauAfAfAfgugcuuugaas(invAb) | 829 | AACUCAAUAAAGUGCUUUGAA |
| AM05749-SS | 590 | (NAG25)sasacucAfauAfAfAfgUfgcuGfuGfaas(invAb) | 1027 | AACUCAAUAAAGUGCUGUGAA |
| AM05751-SS | 591 | (NAG31)sgsaacucaauAfAfAfgugcuuugaas(invAb) | 885 | GAACUCAAUAAAGUGCUUUGAA |
| AM05752-SS | 592 | (NAG25)sgsaacucAfauAfAfAfgUfgcuGfuGfaas(invAb) | 882 | GAACUCAAUAAAGUGCUGUGAA |
| AM05770-SS | 593 | (NAG25)sasacucAfauAfAfAfgUfgcuUfuGfaas(invAb) | 829 | AACUCAAUAAAGUGCUUUGAA |
| AM05771-SS | 594 | (NAG25)sgsaacucAfauAfAfAfgUfgcuUfuGfaas(invAb) | 885 | GAACUCAAUAAAGUGCUUUGAA |
| AM05821-SS | 595 | (NAG25)sgscccaaGfaAfAfGfugaaagaccas(invAb) | 898 | GCCCAAGAAAGUGAAAGACCA |
| AM05822-SS | 596 | (NAG25)sgscCfcaaGfaAfAfGfugaaagaccas(invAb) | 898 | GCCCAAGAAAGUGAAAGACCA |
| AM05830-SS | 597 | (NAG25)saacucaauAfAfAfgugcuuugaas(invAb) | 829 | AACUCAAUAAAGUGCUUUGAA |
| AM05831-SS | 598 | (NAG25)saaCfuCfaauAfAfAfgugcuuugaas(invAb) | 829 | AACUCAAUAAAGUGCUUUGAA |
| AM05832-SS | 599 | (NAG25)saacucaAfuAfAfAfgugcuuugaas(invAb) | 829 | AACUCAAUAAAGUGCUUUGAA |
| AM05843-SS | 600 | (NAG25)susuggagccCfAfAfgaaagugaaas(invAb) | 948 | UUGGAGCCCAAGAAAGUGAAA |
| AM05844-SS | 601 | (NAG25)susuggagCfcCfAfAfgaaagugaaas(invAb) | 948 | UUGGAGCCCAAGAAAGUGAAA |
| AM05845-SS | 602 | (NAG25)susuGfgAfgCfcCfAfAfgaaagugaaas(invAb) | 948 | UUGGAGCCCAAGAAAGUGAAA |
| AM05846-SS | 603 | (NAG25)sgsgagccCfAfAfgaaagugaaas(invAb) | 921 | GGAGCCCAAGAAAGUGAAA |
| AM05847-SS | 604 | (NAG25)sgsgagccCfAfAfgaaagugaaauus(invAb) | 922 | GGAGCCCAAGAAAGUGAAAUU |
| AM05848-SS | 605 | (NAG25)(invAb)ggagccCfAfAfgaaagugaaauus(invAb) | 922 | GGAGCCCAAGAAAGUGAAAUU |
| AM05907-SS | 606 | (NAG25)scsccaaggaGfCfAfuaaguacaaas(invAb) | 868 | CCCAAGGAGCAUAAGUACAAA |
| AM05908-SS | 607 | (NAG25)s(invAb)suuggagucAfAfCfacuuucgauas(invAb) | 949 | UUGGAGUCAACACUUUCGAUA |
| AM05909-SS | 608 | (NAG25)s(invAb)sccaaggAfGfCfauaaguacaauus(invAb) | 866 | CCAAGGAGCAUAAGUACAAUU |
| AM05910-SS | 609 | (NAG25)s(invAb)scgcaagAfGfUfcugucuucgauus(invAb) | 876 | CGCAAGAGUCUGUCUUCGAUU |
| AM05911-SS | 610 | (NAG37)sasacucaauAfAfAfgugcuuugaas(invAb) | 829 | AACUCAAUAAAGUGCUUUGAA |
| AM05912-SS | 611 | (NAG37)scsccaaggaGfCfAfuaaguacaaas(invAb) | 868 | CCCAAGGAGCAUAAGUACAAA |
| AM05913-SS | 612 | (NAG37)s(invAb)suuggagucAfAfCfacuuucgauas(invAb) | 949 | UUGGAGUCAACACUUUCGAUA |
| AM05914-SS | 613 | (NAG37)s(invAb)sccaaggAfGfCfauaaguacaauus(invAb) | 866 | CCAAGGAGCAUAAGUACAAUU |
| AM05915-SS | 614 | (NAG37)s(invAb)scgcaagAfGfUfcugucuucgauus(invAb) | 876 | CGCAAGAGUCUGUCUUCGAUU |
| AM05949-SS | 615 | (NAG3 8)sasacucaauAfAfAfgugcuuugaas(invAb) | 829 | AACUCAAUAAAGUGCUUUGAA |
| AM05951-SS | 616 | (NAG33)sasacucaauAfAfAfgugcuuugaas(invAb) | 829 | AACUCAAUAAAGUGCUUUGAA |
| AM06063-SS | 617 | (NAG25)sgsgaacucaauAfAfAfgugcuuugas(invdA) | 917 | GGAACUCAAUAAAGUGCUUUGAA |
| AM06064-SS | 618 | (NAG25)sgsaacucaauAfAfAfgugcuuugas(invdA) | 885 | GAACUCAAUAAAGUGCUUUGAA |
| AM06065-SS | 619 | (NAG25)s(invAb)sgaacucaauAfAfAfgugcuuugaauus(invA b) | 887 | GAACUCAAUAAAGUGCUUUGAAUU |
| AM06066-SS | 620 | (NAG25)s(invAb)scucaauAfAfAfgugcuuugaauus(invAb) | 879 | CUCAAUAAAGUGCUUUGAAUU |
| AM06067-SS | 621 | (NAG25)sgsgaacucaauAfAfAfgugcuuugs(invdA) | 916 | GGAACUCAAUAAAGUGCUUUGA |
| AM06098-SS | 622 | (NAG3 7)s(invAb)sggagucAfAfCfacuuucgauauus(invAb) | 925 | GGAGUCAACACUUUCGAUAUU |
| AM06099-SS | 623 | (NAG3 7)s(invAb)sggagucAfAfCfacuuucgauas(invAb) | 924 | GGAGUCAACACUUUCGAUA |
| AM06100-SS | 624 | (NAG37)scsuuggagucAfAfCfacuuucgauas(invAb) | 881 | CUUGGAGUCAACACUUUCGAUA |
| AM06101-SS | 625 | (NAG37)scscccaaggAfGfCfauaaguacaas(invAb) | 872 | CCCCAAGGAGCAUAAGUACAA |
| AM06105-SS | 626 | (NAG37)s(invAb)scaaggaGfCfAfuaaguacaaauus(invAb) | 861 | CAAGGAGCAUAAGUACAAAUU |
| AM06110-SS | 627 | (NAG37)s(invAb)scccaaggaGfCfAfuaaguacaaas(invAb) | 868 | CCCAAGGAGCAUAAGUACAAA |
| AM06111-SS | 628 | (NAG37)s(invAb)sccccaaggaGfCfAfuaaguacaaas(invAb) | 873 | CCCCAAGGAGCAUAAGUACAAA |
| AM06112-SS | 629 | (NAG37)s(invAb)scccaaggaGfCfAfuaaguacaaauus(invAb) | 869 | CCCAAGGAGCAUAAGUACAAAUU |
| AM06115-SS | 630 | (NAG37)s(invAb)sccccaaggAfGfCfauaaguacaas(invAb) | 872 | CCCCAAGGAGCAUAAGUACAA |
| AM06116-SS | 631 | (NAG37)s(invAb)scccaaggAfGfCfauaaguacaauus(invAb) | 871 | CCCAAGGAGCAUAAGUACAAUU |
| AM06118-SS | 632 | (NAG37)s(invAb)scuuggagucAfAfCfacuuucgauas(invAb) | 881 | CUUGGAGUCAACACUUUCGAUA |
| AM06119-SS | 633 | (NAG37)s(invAb)sgcuuggagucAfAfCfacuuucgauas(invAb) | 912 | GCUUGGAGUCAACACUUUCGAUA |
| AM06345-SS | 634 | (NAG37)scsccaaggaGfCfAfuaaguacaas(invdA) | 868 | CCCAAGGAGCAUAAGUACAAA |
| AM06346-SS | 635 | (NAG37)cccaaggagcAfuaaguacaa(invdA) | 868 | CCCAAGGAGCAUAAGUACAAA |
| AM06347-SS | 636 | (NAG37)cccaaggagcauaaguacaa(invdA) | 868 | CCCAAGGAGCAUAAGUACAAA |
| AM06348-SS | 637 | (NAG3 7)s(invAb)sccaaggaGfCfAfuaaguacaas(invdA) | 865 | CCAAGGAGCAUAAGUACAAA |
| AM06349-SS | 638 | (NAG37)s(invAb)scaaggaGfCfAfuaaguacaas(invdA) | 860 | CAAGGAGCAUAAGUACAAA |
| AM06351-SS | 639 | (NAG37)scsccaaggaGfCfAfuaaguacaaus(invAb) | 870 | CCCAAGGAGCAUAAGUACAAU |
| AM06587-SS | 640 | (NAG3 7)sascucaauaAfAfGfugcuuugaaas(invAb) | 846 | ACUCAAUAAAGUGCUUUGAAA |
| AM07040-SS | 641 | (NAG37)s(invAb)sgccaaggaGfCfAfuaaguacaaas(invAb) | 896 | GCCAAGGAGCAUAAGUACAAA |
| AM07042-SS | 642 | (NAG37)s(invAb)sgccaaggaGfCfAfuaaiuacaaas(invAb) | 897 | GCCAAGGAGCAUAAIUACAAA |
| AM07044-SS | 643 | (NAG37)gsccaaggaGfCfAfuaaguacaaas(invAb) | 896 | GCCAAGGAGCAUAAGUACAAA |
| AM07045-SS | 644 | (NAG37)s(invAb)sgcccaaggAfGfCfauaaguacaas(invAb) | 899 | GCCCAAGGAGCAUAAGUACAA |
| AM07047-SS | 645 | (NAG37)s(invAb)sgccccaagGfAfGfcauaaguacas(invAb) | 900 | GCCCCAAGGAGCAUAAGUACA |
| AM07049-SS | 646 | (NAG37)s(invAb)sgcaaggagCfAfUfaaguacaaaas(invAb) | 894 | GCAAGGAGCAUAAGUACAAAA |
| AM07051-SS | 647 | (NAG37)s(invAb)scgaggagcAfUfAfaguacaaagas(invAb) | 875 | CGAGGAGCAUAAGUACAAAGA |
| AM07053-SS | 648 | (NAG37)s(invAb)sggaggagcAfUfAfaguacaaagas(invAb) | 923 | GGAGGAGCAUAAGUACAAAGA |
| AM07055-SS | 649 | (NAG37)s(invAb)sggagcauaAfGfUfacaaagcugas(invAb) | 920 | GGAGCAUAAGUACAAAGCUGA |
| AM07057-SS | 650 | (NAG37)s(invAb)sagcuguacCfAfCfaaauguaccas(invAb) | 854 | AGCUGUACCACAAAUGUACCA |
| AM07248-SS | 651 | (NAG37)s(invAb)saccaaggaGfCfAfuaaguacaaas(invAb) | 844 | ACCAAGGAGCAUAAGUACAAA |
| AM07250-SS | 652 | (NAG37)asccaaggaGfCfAfuaaguacaaas(invAb) | 844 | ACCAAGGAGCAUAAGUACAAA |
| AM07251-SS | 653 | (NAG37)gsccaaggaGfCfAfuAaguacaaas(invAb) | 896 | GCCAAGGAGCAUAAGUACAAA |
| AM07252-SS | 654 | (NAG37)s(invAb)scuuggaguCfAfAfcacuuucgaus(invAb) | 880 | CUUGGAGUCAACACUUUCGAU |
| AM07254-SS | 655 | (NAG37)s(invAb)sgcuggaguCfAfAfcacuuucgaus(invAb) | 911 | GCUGGAGUCAACACUUUCGAU |
| AM07256-SS | 656 | (NAG37)s(invAb)sguggagucAfAfCfacuuucgauus(invAb) | 930 | GUGGAGUCAACACUUUCGAUU |
| AM07258-SS | 657 | (NAG37)s(invAb)sgagucaacAfCfUfuucgauuccas(invAb) | 893 | GAGUCAACACUUUCGAUUCCA |
| AM07260-SS | 658 | (NAG37)s(invAb)scagcuguaCfCfAfcaaauguacas(invAb) | 862 | CAGCUGUACCACAAAUGUACA |
| AM07262-SS | 659 | (NAG37)s(invAb)scgagcauaAfGfUfacaaagcugas(invAb) | 874 | CGAGCAUAAGUACAAAGCUGA |
| AM07264-SS | 660 | (NAG37)s(invAb)sagagcauaAfGfUfacaaagcugas(invAb) | 850 | AGAGCAUAAGUACAAAGCUGA |
| AM07266-SS | 661 | (NAG37)s(invAb)sacccaaggAfGfCfauaaguacaas(invAb) | 845 | ACCCAAGGAGCAUAAGUACAA |

The FXII RNAi agents described herein are formed by annealing an antisense strand with a sense strand. A sense strand containing a sequence listed in Table 2, Table 4, or Table 6 can be hybridized to any antisense strand containing a sequence listed in Table 2, Table 3, or Table 6, provided the two sequences have a region of at least 85% complementarity over a contiguous 16, 17, 18, 19, 20, or 21 nucleotide sequence.

In some embodiments, the antisense strand of an FXII RNAi agent disclosed herein differs by 0, 1, 2, or 3 nucleotides from any of the antisense strand sequences in Table 3. In some embodiments, the sense strand of an FXII RNAi agent disclosed herein differs by 0, 1, 2, or 3 nucleotides from any of the sense strand sequences in Table 4.

In some embodiments, an FXII RNAi agent antisense strand comprises a nucleotide sequence of any of the sequences in Table 2, Table 3, or Table 6. In some embodiments, an FXII RNAi agent antisense strand comprises the sequence of nucleotides (from 5' end → 3' end) 1-17, 2-17, 1-18, 2-18, 1-19, 2-19, 1-20, 2-20, 1-21, 2-21, 1-22, 2-22, 1-23, 2-23, 1-24, or 2-24, 1-25, 2-25, 1-16, or 2-16 of any of the sequences in Table 2, Table 3, or Table 6. In certain embodiments, an FXII RNAi agent antisense strand comprises or consists of a modified sequence of any one of the modified sequences in Table 3. In certain embodiments, an FXII RNAi agent sense strand comprises or consists of a modified sequence of any one of the modified sequences in Table 6.

In some embodiments, an FXII RNAi agent sense strand comprises the nucleotide sequence of any of the sequences in Table 2, Table 4, or Table 6. In some embodiments, an FXII RNAi agent sense strand comprises the sequence of nucleotides (from 5' end → 3' end) 1-17, 2-17, 3-17, 4-17, 1-18, 2-18, 3-18, 4-18, 1-19, 2-19, 3-19, 4-19, 1-20, 2-20, 3-20, 4-20, 1-21, 2-21, 3-21, 4-21, 1-22, 2-22, 3-22, 4-22, 1-23, 2-23, 3-23, 4-23, 1-24, 2-24, 3-24, 4-24, 1-25, 2-25, 3-25, 4-25, 1-26, 2-26, 3-26, or 4-26, of any of the sequences in Table 2, Table 4, or Table 6. In certain embodiments, an FXII RNAi agent sense strand comprises or consists of a modified sequence of any one of the modified sequences in Table 5. In certain embodiments, an FXII RNAi agent sense strand comprises or consists of a modified sequence of any one of the modified sequences in Table 6.

For the FXII RNAi agents disclosed herein, the nucleotide at position 1 of the antisense strand (from 5' end → 3' end) can be perfectly complementary to an FXII gene, or can be non-complementary to an FXII gene. In some embodiments, the nucleotide at position 1 of the antisense strand (from 5' end + 3' end) is a U, A, or dT (or a modified version of U, A or dT). In some embodiments, the nucleotide at position 1 of the antisense strand (from 5' end → 3' end) forms an A:U or U:A base pair with the sense strand.

In some embodiments, an FXII RNAi agent antisense strand comprises the sequence of nucleotides (from 5' end → 3' end) 2-18 or 2-19 of any of the antisense strand sequences in Table 2, Table 3, or Table 6. In some embodiments, an FXII RNAi sense strand comprises the sequence of nucleotides (from 5' end → 3' end) 1-17 or 1-18 of any of the sense strand sequences in Table 2, Table 4, or Table 6.

In some embodiments, an FXII RNAi agent includes (i) an antisense strand comprising the sequence of nucleotides (from 5' end + 3' end) 2-18 or 2-19 of any of the antisense strand sequences in Table 2, Table 3, or Table 6, and (ii) a sense strand comprising the sequence of nucleotides (from 5' end → 3' end) 1-17 or 1-18 of any of the sense strand sequences in Table 2, Table 4, or Table 6.

A sense strand containing a sequence listed in Table 2, Table 4, or Table 6 can be hybridized to any antisense strand containing a sequence listed in Table 2, Table 3, or Table 6, provided the two sequences have a region of at least 85% complementarity over a contiguous 16, 17, 18, 19, 20, or 21 nucleotide sequence. In some embodiments, the FXII RNAi agent has a sense strand consisting of the modified sequence of any of the modified sequences in Table 4, and an antisense strand consisting of the modified sequence of any of the modified sequences in Table 3. Certain representative sequence pairings are exemplified by the Duplex ID Nos. shown in Table 5 and Table 6.

In some embodiments, an FXII RNAi agent comprises any of the duplexes represented by any of the Duplex ID Nos. presented herein. In some embodiments, an FXII RNAi agent consists of any of the duplexes represented by any of the Duplex ID Nos. presented herein. In some embodiments, an FXII RNAi agent comprises the sense strand and antisense strand nucleotide sequences of any of the duplexes represented by any of the Duplex ID Nos. presented herein. In some embodiments, an FXII RNAi agent includes the sense strand and antisense strand nucleotide sequences of any of the duplexes represented by any of the Duplex ID Nos. presented herein and a targeting group and/or linking group, wherein the targeting group and/or linking group is covalently linked (i.e., conjugated) to the sense strand or the antisense strand. In some embodiments, an FXII RNAi agent includes the sense strand and antisense strand modified nucleotide sequences of any of the duplexes represented by any of the Duplex ID Nos. presented herein. In some embodiments, an FXII RNAi agent comprises the sense strand and antisense strand modified nucleotide sequences of any of the duplexes represented by any of the Duplex ID Nos. presented herein and a targeting group and/or linking group, wherein the targeting group and/or linking group is covalently linked to the sense strand or the antisense strand.

In some embodiments, an FXII RNAi agent comprises an antisense strand and a sense strand having the nucleotide sequences of any of the antisense strand/sense strand duplexes of Table 2, Table 5, or Table 6, and comprises an asialoglycoprotein receptor ligand targeting group.

In some embodiments, an FXII RNAi agent comprises an antisense strand and a sense strand having the nucleotide sequences of any of the antisense strand/sense strand duplexes of Table 2, Table 5, or Table 6, and further comprises a targeting group selected from the group consisting of (PAZ), (NAG13), (NAG13)s, (NAG18), (NAG18)s, (NAG24), (NAG24)s, (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s, (NAG38), (NAG38)s, (NAG39), (NAG39)s. In some embodiments, the targeting group is (NAG25) or (NAG25)s as defined in Table 7. In other embodiments, the targeting group is (NAG37) or (NAG37)s as defined in Table 7.

In some embodiments, an FXII RNAi agent comprises an antisense strand and a sense strand having the modified nucleotide sequence of any of the antisense strand and/or sense strand nucleotide sequences of any of the duplexes of Table 5.

In some embodiments, an FXII RNAi agent comprises an antisense strand and a sense strand having a modified nucleotide sequence of any of the antisense strand and/or sense strand nucleotide sequences of any of the duplexes of Table 5, and comprises an asialoglycoprotein receptor ligand targeting group.

In some embodiments, an FXII RNAi agent comprises, consists of, or consists essentially of, the duplex structure of any of the duplexes in Table 5.

**Table 5. FXII RNAi Agents Identified by Duplex ID No. with Corresponding Sense and Antisense Strands.**

| **Duplex ID** | **Antisense Strand ID** | **Sense Strand ID** |
|---|---|---|
| AD03224 | AM04048-AS | AM03844-SS |
| AD03592 | AM04568-AS | AM04558-SS |
| AD03593 | AM03965-AS | AM04558-SS |
| AD03594 | AM04569-AS | AM04558-SS |
| AD03595 | AM04570-AS | AM04558-SS |
| AD03596 | AM04571-AS | AM04558-SS |
| AD03600 | AM04575-AS | AM04561-SS |
| AD03601 | AM04576-AS | AM04561-SS |
| AD03602 | AM04577-AS | AM04561-SS |
| AD03632 | AM04048-AS | AM04613-SS |
| AD03635 | AM03157-AS | AM04130-SS |
| AD03637 | AM04619-AS | AM04558-SS |
| AD03638 | AM04620-AS | AM04558-SS |
| AD03773 | AM04619-AS | AM04554-SS |
| AD03774 | AM04620-AS | AM04554-SS |
| AD03782 | AM04568-AS | AM04814-SS |
| AD03783 | AM03965-AS | AM04814-SS |
| AD03784 | AM04569-AS | AM04814-SS |
| AD03785 | AM04570-AS | AM04814-SS |
| AD03786 | AM04571-AS | AM04814-SS |
| AD03790 | AM04575-AS | AM04815-SS |
| AD03791 | AM04576-AS | AM04815-SS |
| AD03792 | AM04577-AS | AM04815-SS |
| AD03807 | AM04048-AS | AM04836-SS |
| AD03808 | AM04048-AS | AM04837-SS |
| AD03809 | AM04048-AS | AM04845-SS |
| AD03810 | AM04048-AS | AM04838-SS |
| AD03811 | AM04048-AS | AM04839-SS |
| AD03812 | AM04048-AS | AM04840-SS |
| AD03813 | AM04841-AS | AM04840-SS |
| AD03814 | AM04842-AS | AM04836-SS |
| AD03815 | AM04842-AS | AM04840-SS |
| AD03816 | AM04843-AS | AM04840-SS |
| AD03817 | AM04844-AS | AM04836-SS |
| AD03819 | AM04048-AS | AM04847-SS |
| AD03820 | AM04568-AS | AM04554-SS |
| AD03865 | AM04048-AS | AM04881-SS |
| AD03866 | AM04903-AS | AM04882-SS |
| AD03868 | AM04905-AS | AM04883-SS |
| AD03869 | AM04906-AS | AM04884-SS |
| AD03870 | AM04907-AS | AM04885-SS |
| AD03872 | AM04909-AS | AM04886-SS |
| AD03873 | AM04910-AS | AM04887-SS |
| AD03874 | AM04911-AS | AM04888-SS |
| AD03876 | AM04913-AS | AM04889-SS |
| AD03877 | AM04914-AS | AM04890-SS |
| AD03878 | AM04915-AS | AM04891-SS |
| AD03879 | AM04916-AS | AM04891-SS |
| AD03880 | AM04917-AS | AM04892-SS |
| AD03881 | AM04918-AS | AM04893-SS |
| AD03882 | AM04919-AS | AM04894-SS |
| AD03884 | AM04921-AS | AM04895-SS |
| AD03885 | AM04922-AS | AM04896-SS |
| AD03886 | AM04923-AS | AM04897-SS |
| AD03888 | AM04925-AS | AM04898-SS |
| AD03889 | AM04926-AS | AM04899-SS |
| AD03890 | AM04927-AS | AM04900-SS |
| AD03892 | AM04929-AS | AM04901-SS |
| AD03893 | AM04930-AS | AM04902-SS |
| AD03894 | AM04932-AS | AM04931-SS |
| AD03895 | AM04932-AS | AM04933-SS |
| AD03896 | AM04932-AS | AM04934-SS |
| AD03897 | AM04932-AS | AM04935-SS |
| AD03898 | AM04936-AS | AM04935-SS |
| AD03899 | AM04938-AS | AM04937-SS |
| AD03900 | AM04047-AS | AM04939-SS |
| AD03901 | AM04941-AS | AM04940-SS |
| AD03902 | AM04943-AS | AM04942-SS |
| AD03903 | AM04945-AS | AM04944-SS |
| AD03904 | AM04947-AS | AM04946-SS |
| AD03905 | AM04949-AS | AM04948-SS |
| AD03906 | AM04951-AS | AM04950-SS |
| AD03907 | AM04953-AS | AM04952-SS |
| AD03908 | AM04955-AS | AM04954-SS |
| AD03909 | AM04957-AS | AM04956-SS |
| AD03910 | AM04959-AS | AM04958-SS |
| AD03911 | AM04959-AS | AM04960-SS |
| AD03912 | AM04959-AS | AM04961-SS |
| AD03913 | AM04959-AS | AM04962-SS |
| AD03914 | AM04959-AS | AM04963-SS |
| AD03915 | AM04959-AS | AM04964-SS |
| AD03916 | AM04959-AS | AM04965-SS |
| AD03917 | AM04959-AS | AM04966-SS |
| AD03918 | AM04959-AS | AM04967-SS |
| AD03919 | AM04959-AS | AM04968-SS |
| AD03927 | AM03977-AS | AM04974-SS |
| AD03928 | AM04959-AS | AM04975-SS |
| AD03934 | AM04903-AS | AM04980-SS |
| AD03936 | AM04907-AS | AM04981-SS |
| AD03938 | AM04911-AS | AM04982-SS |
| AD03940 | AM04915-AS | AM04983-SS |
| AD03942 | AM04919-AS | AM04984-SS |
| AD03944 | AM04923-AS | AM04985-SS |
| AD03946 | AM04927-AS | AM04986-SS |
| AD03948 | AM04932-AS | AM04987-SS |
| AD03989 | AM04930-AS | AM05031-SS |
| AD03990 | AM04930-AS | AM05032-SS |
| AD04014 | AM05066-AS | AM05065-SS |
| AD04120 | AM05248-AS | AM05245-SS |
| AD04121 | AM05256-AS | AM05249-SS |
| AD04124 | AM05257-AS | AM05251-SS |
| AD04125 | AM05257-AS | AM05259-SS |
| AD04126 | AM05246-AS | AM05252-SS |
| AD04127 | AM05246-AS | AM05253-SS |
| AD04130 | AM05246-AS | AM05254-SS |
| AD04131 | AM05261-AS | AM05255-SS |
| AD04132 | AM05262-AS | AM05254-SS |
| AD04135 | AM05265-AS | AM04985-SS |
| AD04136 | AM05266-AS | AM04985-SS |
| AD04137 | AM05268-AS | AM05267-SS |
| AD04138 | AM05270-AS | AM05269-SS |
| AD04139 | AM05270-AS | AM05271-SS |
| AD04140 | AM04926-AS | AM05272-SS |
| AD04141 | AM05274-AS | AM05273-SS |
| AD04142 | AM05276-AS | AM05275-SS |
| AD04143 | AM05278-AS | AM05277-SS |
| AD04144 | AM05279-AS | AM04892-SS |
| AD04147 | AM04917-AS | AM05282-SS |
| AD04148 | AM04917-AS | AM05283-SS |
| AD04149 | AM04917-AS | AM05284-SS |
| AD04151 | AM04917-AS | AM05286-SS |
| AD04152 | AM04917-AS | AM05287-SS |
| AD04153 | AM05289-AS | AM05288-SS |
| AD04157 | AM05327-AS | AM05317-SS |
| AD04158 | AM05327-AS | AM05318-SS |
| AD04159 | AM05328-AS | AM05318-SS |
| AD04160 | AM05329-AS | AM05319-SS |
| AD04161 | AM05330-AS | AM05320-SS |
| AD04162 | AM05331-AS | AM05321-SS |
| AD04163 | AM05332-AS | AM05322-SS |
| AD04164 | AM05333-AS | AM05323-SS |
| AD04165 | AM05334-AS | AM05324-SS |
| AD04166 | AM05335-AS | AM05325-SS |
| AD04167 | AM05336-AS | AM05326-SS |
| AD04191 | AM05367-AS | AM05366-SS |
| AD04193 | AM05370-AS | AM05369-SS |
| AD04195 | AM05373-AS | AM05372-SS |
| AD04197 | AM05376-AS | AM05375-SS |
| AD04199 | AM05379-AS | AM05378-SS |
| AD04200 | AM05382-AS | AM05380-SS |
| AD04203 | AM04923-AS | AM05384-SS |
| AD04204 | AM05379-AS | AM05385-SS |
| AD04205 | AM05379-AS | AM05386-SS |
| AD04208 | AM05327-AS | AM05389-SS |
| AD04209 | AM05331-AS | AM05390-SS |
| AD04254 | AM05478-AS | AM05324-SS |
| AD04255 | AM05479-AS | AM05325-SS |
| AD04256 | AM05480-AS | AM05326-SS |
| AD04258 | AM05482-AS | AM05375-SS |
| AD04321 | AM05257-AS | AM05534-SS |
| AD04324 | AM05257-AS | AM05535-SS |
| AD04327 | AM05246-AS | AM05535-SS |
| AD04330 | AM05261-AS | AM05536-SS |
| AD04333 | AM05270-AS | AM05537-SS |
| AD04336 | AM05270-AS | AM05538-SS |
| AD04339 | AM04926-AS | AM05539-SS |
| AD04342 | AM05274-AS | AM05540-SS |
| AD04345 | AM05276-AS | AM05541-SS |
| AD04348 | AM05278-AS | AM05542-SS |
| AD04351 | AM05274-AS | AM05543-SS |
| AD04352 | AM05274-AS | AM05544-SS |
| AD04353 | AM05274-AS | AM05545-SS |
| AD04354 | AM05274-AS | AM05546-SS |
| AD04355 | AM05274-AS | AM05547-SS |
| AD04356 | AM05274-AS | AM05548-SS |
| AD04357 | AM05274-AS | AM05549-SS |
| AD04358 | AM05274-AS | AM05550-SS |
| AD04395 | AM05246-AS | AM05587-SS |
| AD04396 | AM05246-AS | AM05588-SS |
| AD04397 | AM05246-AS | AM05589-SS |
| AD04398 | AM05590-AS | AM05535-SS |
| AD04399 | AM05591-AS | AM05535-SS |
| AD04400 | AM05261-AS | AM05592-SS |
| AD04401 | AM05261-AS | AM05593-SS |
| AD04402 | AM05261-AS | AM05594-SS |
| AD04403 | AM05596-AS | AM05595-SS |
| AD04404 | AM05597-AS | AM05536-SS |
| AD04443 | AM05641-AS | AM05324-SS |
| AD04465 | AM05681-AS | AM05680-SS |
| AD04466 | AM05683-AS | AM05682-SS |
| AD04467 | AM05685-AS | AM05684-SS |
| AD04468 | AM05686-AS | AM05321-SS |
| AD04469 | AM05687-AS | AM05321-SS |
| AD04470 | AM05689-AS | AM05688-SS |
| AD04471 | AM05691-AS | AM05690-SS |
| AD04472 | AM05692-AS | AM05690-SS |
| AD04473 | AM05691-AS | AM05693-SS |
| AD04474 | AM05692-AS | AM05693-SS |
| AD04475 | AM05695-AS | AM05694-SS |
| AD04476 | AM05679-AS | AM05678-SS |
| AD04477 | AM05696-AS | AM05324-SS |
| AD04478 | AM05698-AS | AM05697-SS |
| AD04479 | AM05700-AS | AM05699-SS |
| AD04480 | AM05702-AS | AM05701-SS |
| AD04481 | AM05704-AS | AM05703-SS |
| AD04482 | AM05705-AS | AM05324-SS |
| AD04483 | AM05707-AS | AM05706-SS |
| AD04484 | AM05708-AS | AM05706-SS |
| AD04485 | AM05707-AS | AM05709-SS |
| AD04486 | AM05708-AS | AM05709-SS |
| AD04487 | AM05711-AS | AM05710-SS |
| AD04488 | AM05713-AS | AM05712-SS |
| AD04489 | AM05708-AS | AM05716-SS |
| AD04490 | AM05718-AS | AM05717-SS |
| AD04491 | AM05720-AS | AM05719-SS |
| AD04492 | AM05722-AS | AM05721-SS |
| AD04493 | AM05478-AS | AM05723-SS |
| AD04494 | AM05478-AS | AM05724-SS |
| AD04495 | AM05726-AS | AM05725-SS |
| AD04496 | AM05728-AS | AM05727-SS |
| AD04497 | AM05729-AS | AM05317-SS |
| AD04498 | AM05731-AS | AM05730-SS |
| AD04499 | AM05733-AS | AM05732-SS |
| AD04500 | AM05327-AS | AM05743-SS |
| AD04501 | AM05733-AS | AM05734-SS |
| AD04502 | AM05733-AS | AM05735-SS |
| AD04503 | AM05736-AS | AM05735-SS |
| AD04505 | AM05327-AS | AM05738-SS |
| AD04506 | AM05327-AS | AM05739-SS |
| AD04507 | AM05327-AS | AM05740-SS |
| AD04508 | AM05742-AS | AM05741-SS |
| AD04512 | AM05331-AS | AM05748-SS |
| AD04513 | AM05750-AS | AM05749-SS |
| AD04514 | AM05332-AS | AM05751-SS |
| AD04515 | AM05753-AS | AM05752-SS |
| AD04524 | AM05750-AS | AM05770-SS |
| AD04525 | AM05753-AS | AM05771-SS |
| AD04547 | AM05818-AS | AM05535-SS |
| AD04548 | AM05818-AS | AM05254-SS |
| AD04549 | AM05819-AS | AM05821-SS |
| AD04550 | AM05247-AS | AM05535-SS |
| AD04551 | AM05247-AS | AM05822-SS |
| AD04552 | AM05820-AS | AM05535-SS |
| AD04553 | AM05820-AS | AM05822-SS |
| AD04554 | AM05823-AS | AM05321-SS |
| AD04555 | AM05824-AS | AM05321-SS |
| AD04556 | AM05825-AS | AM05830-SS |
| AD04557 | AM05825-AS | AM05831-SS |
| AD04558 | AM05826-AS | AM05832-SS |
| AD04559 | AM05836-AS | AM05843-SS |
| AD04560 | AM05837-AS | AM05843-SS |
| AD04561 | AM05838-AS | AM05844-SS |
| AD04562 | AM05837-AS | AM05845-SS |
| AD04563 | AM05839-AS | AM05843-SS |
| AD04564 | AM05837-AS | AM05844-SS |
| AD04565 | AM05840-AS | AM05843-SS |
| AD04566 | AM05841-AS | AM05846-SS |
| AD04567 | AM05841-AS | AM05847-SS |
| AD04568 | AM05842-AS | AM05847-SS |
| AD04569 | AM05842-AS | AM05848-SS |
| AD04618 | AM05902-AS | AM05321-SS |
| AD04619 | AM05903-AS | AM05907-SS |
| AD04620 | AM05904-AS | AM05908-SS |
| AD04621 | AM05905-AS | AM05909-SS |
| AD04622 | AM05906-AS | AM05910-SS |
| AD04623 | AM05331-AS | AM05911-SS |
| AD04624 | AM05902-AS | AM05911-SS |
| AD04625 | AM05903-AS | AM05912-SS |
| AD04626 | AM05904-AS | AM05913-SS |
| AD04627 | AM05905-AS | AM05914-SS |
| AD04628 | AM05906-AS | AM05915-SS |
| AD04649 | AM05950-AS | AM05321-SS |
| AD04650 | AM05331-AS | AM05951-SS |
| AD04651 | AM05331-AS | AM05949-SS |
| AD04722 | AM05825-AS | AM06063-SS |
| AD04723 | AM05825-AS | AM06064-SS |
| AD04724 | AM06060-AS | AM06064-SS |
| AD04725 | AM06061-AS | AM06065-SS |
| AD04726 | AM06062-AS | AM06066-SS |
| AD04727 | AM05825-AS | AM06067-SS |
| AD04743 | AM06093-AS | AM06098-SS |
| AD04744 | AM06093-AS | AM06099-SS |
| AD04745 | AM06094-AS | AM06099-SS |
| AD04746 | AM06095-AS | AM06100-SS |
| AD04747 | AM06096-AS | AM06101-SS |
| AD04748 | AM06097-AS | AM05912-SS |
| AD04749 | AM06103-AS | AM05912-SS |
| AD04750 | AM06104-AS | AM06105-SS |
| AD04751 | AM06097-AS | AM06110-SS |
| AD04752 | AM06106-AS | AM05912-SS |
| AD04753 | AM06107-AS | AM06111-SS |
| AD04754 | AM06108-AS | AM06111-SS |
| AD04755 | AM06097-AS | AM06112-SS |
| AD04757 | AM06113-AS | AM06115-SS |
| AD04758 | AM06114-AS | AM06116-SS |
| AD04759 | AM06095-AS | AM06118-SS |
| AD04760 | AM06117-AS | AM06119-SS |
| AD04899 | AM06334-AS | AM06345-SS |
| AD04900 | AM06335-AS | AM06345-SS |
| AD04901 | AM06334-AS | AM06346-SS |
| AD04902 | AM06334-AS | AM06347-SS |
| AD04903 | AM06336-AS | AM06348-SS |
| AD04904 | AM06337-AS | AM06348-SS |
| AD04905 | AM06104-AS | AM06349-SS |
| AD04906 | AM06338-AS | AM06349-SS |
| AD04907 | AM06339-AS | AM06350-SS |
| AD04911 | AM06343-AS | AM06349-SS |
| AD04912 | AM06344-AS | AM06351-SS |
| AD05055 | AM05478-AS | AM06587-SS |
| AD05330 | AM07041-AS | AM07040-SS |
| AD05331 | AM07041-AS | AM07042-SS |
| AD05332 | AM07043-AS | AM07040-SS |
| AD05333 | AM07041-AS | AM07044-SS |
| AD05334 | AM07046-AS | AM07045-SS |
| AD05335 | AM07048-AS | AM07047-SS |
| AD05336 | AM07050-AS | AM07049-SS |
| AD05337 | AM07052-AS | AM07051-SS |
| AD05338 | AM07054-AS | AM07053-SS |
| AD05339 | AM07056-AS | AM07055-SS |
| AD05340 | AM07058-AS | AM07057-SS |
| AD05496 | AM07249-AS | AM07248-SS |
| AD05497 | AM07249-AS | AM07250-SS |
| AD05498 | AM07041-AS | AM07251-SS |
| AD05499 | AM07253-AS | AM07252-SS |
| AD05500 | AM07255-AS | AM07254-SS |
| AD05501 | AM07257-AS | AM07256-SS |
| AD05502 | AM07259-AS | AM07258-SS |
| AD05503 | AM07261-AS | AM07260-SS |
| AD05504 | AM07263-AS | AM07262-SS |
| AD05505 | AM07265-AS | AM07264-SS |
| AD05506 | AM07267-AS | AM07266-SS |

**Table 6. Additional Modified FXII RNAi Agent Duplexes (With Corresponding Antisense Strand and Sense Strand Sequences).**

| **DUPLEX ID:** | **Antisense Sequence (5' → 3')** | **SEQ ID NO:** | **Sense Sequence (5' → 3')** | **SEQ ID NO:** |
|---|---|---|---|---|
| 91_2 | uAfuCfgAfaAfgUfgUfuGfaCfuCfcuu | 952 | ggAfgUfcAfAfCfaCfuUfuCfgAfuauu | 989 |
| 91_3 | uAfuCfgAfaAfGfUfgUfuGfaCfuCfcuu | 953 | ggAfgUfcAfAfCfaCfuUfuCfgAfuauu | 990 |
| 91_4 | uAfuCfgAfaAfgUfgUfuGfaCfuCfcuu | 954 | ggAfgUfcAfAfCfacuuucgauauu | 991 |
| 91_5 | uAfuCfgAfaAfgUfgUfuGfaCfuCfcuu | 955 | ggagucAfAfCfacuuucgauauu | 992 |
| 91_6 | uAfuCfgAfaagugUfuGfaCfuCfcuu | 955 | ggagucAfAfCfacuuucgauauu | 993 |
| 91_7 | uAfuCfgAfaagugUfuGfaCfuCfcaa | 956 | ggagucAfAfCfacuuucgauauu | 994 |
| 91_8 | uAfucgAfaAfGfuguuGfaCfuccuu | 957 | ggagUfcAfAfCfacuuucgauauu | 995 |
| 91_9 | uAfuCfgAfaagugUfuGfaCfuCfcAfa | 958 | uuggagucAfAfCfacuuucgaua | 996 |
| 91_10 | uAfuCfgAfaagugUfuGfaCfuCfcAfacg | 959 | uuggagucAfAfCfacuuucgaua | 997 |
| 125_2 | uGfuAfcUfuAfUfGfcUfcCfuUfgGfguu | 960 | ccCfaAfgGfAfGfcAfuAfaGfuAfcauu | 998 |
| 125_3 | uGfuAfcUfuaugcUfcCfuUfgGfguu | 961 | cccaagGfAfGfcauaaguacauu | 999 |
| 125_4 | uGfuAfcUfuAfuGfcUfcCfuUfgGfguu | 962 | cccaagGfAfGfcauaaguacauu | 1000 |
| 125_5 | uGfuacUfuAfUfgcUfcCfuuggguu | 963 | cccaAfgGfAfGfcauaaguacauu | 1001 |
| 126_2 | uUfgUfaCfuUfAfUfgCfuCfcUfuGfguu | 964 | ccAfaGfgAfGfCfaUfaAfgUfaCfaauu | 1002 |
| 126_3 | uUfgUfaCfuuaugCfuCfcUfuGfguu | 965 | ccaaggAfGfCfauaaguacaauu | 1003 |
| 126_4 | uUfgUfaCfuUfaUfgCfuCfcUfuGfguu | 966 | ccaaggAfGfCfauaaguacaauu | 1004 |
| 126_5 | uUfguaCfuUfAfugcuCfcUfugguu | 967 | ccaaGfgAfGfCfauaaguacaauu | 1005 |
| 126_6 | uUfgUfaCfuuaugCfuCfcUfuGfggu | 968 | acccaaggAfGfCfauaaguacaa | 1006 |
| 126_7 | uUfgUfaCfuuaugCfuCfcUfuGfggg | 969 | ccccaaggAfGfCfauaaguacaa | 1007 |
| 127_2 | uUfuGfuAfcUfUfAfuGfcUfcCfuUfguu | 970 | caAfgGfaGfCfAfuAfaGfuAfcAfaauu | 1008 |
| 127_3 | uUfuGfuAfcuuauGfcUfcCfuUfguu | 971 | caaggaGfCfAfuaaguacaaauu | 1009 |
| 127_4 | uUfuGfuAfcUfuAfuGfcUfcCfuUfguu | 972 | caaggaGfCfAfuaaguacaaauu | 1010 |
| 127_5 | uUfuGfuAfcuuauGfcUfcCfuUfgcc | 973 | ggcaaggaGfCfAfuaaguacaaa | 1011 |
| 127_6 | uUfuGfuAfcUfuAfuGfcUfcCfuUfgcc | 974 | ggcaaggaGfCfAfuaaguacaaa | 1012 |
| 132_2 | uCfaGfcUfuUfgUfaCfuUfaUfgCfuuu | 975 | agcauaAfGfUfacaaagcugauu | 1013 |
| 132_3 | uCfaGfcUfuUfgUfaCfuUfaUfgCfuCfc | 976 | ggagcauaAfGfUfacaaagcuga | 1014 |
| 223_2 | uUfuGfuGfgGfuAfcAfuUfuGfuGfguu | 977 | ccacaaAfUfGfuacccacaaauu | 1015 |
| 849_2 | uUfgUfcGfuUfgUfcCfgGfgUfuCfcuu | 978 | ggaaccCfGfGfacaacgacaauu | 1016 |
| 849_3 | aUfgUfcGfuUfgUfcCfgGfgUfuCfcuu | 979 | ggaaccCfGfGfacaacgacauuu | 1017 |
| 1139_2 | uCfgAfaGfaCfAfGfaCfuCfuUfgCfguu | 980 | CfgCfaAfgAfGfUfcUfgUfcUfuCfgauu | 1018 |
| 1139_3 | uCfgAfaGfacagaCfuCfuUfgCfguu | 981 | cgcaagAfGfUfcugucuucgauu | 1019 |
| 1139_4 | uCfgAfaGfaCfaGfaCfuCfuUfgCfguu | 982 | cgcaagAfGfUfcugucuucgauu | 1020 |
| 1139_5 | uCfgaaGfaCfAfgacuCfuUfgcguu | 983 | cgcaAfgAfGfUfcugucuucgauu | 1021 |
| 1476_2 | aCfgUfaAfgGfCfGfaCfaGfgAfgCfguu | 984 | cgCfuCfcUfGfUfcGfcCfuUfaCfguuu | 1022 |
| 1476_3 | uCfgUfaAfgGfCfGfaCfaGfgAfgCfguu | 985 | cgCfuCfcUfGfUfcGfcCfuUfaCfgauu | 1023 |
| 1476_4 | uCfgUfaAfggcgaCfaGfgAfgCfguu | 986 | cgcuccUfGfUfcgccuuacgauu | 1024 |
| 1476_5 | uCfgUfaAfgGfcGfaCfaGfgAfgCfguu | 987 | cgcuccUfGfUfcgccuuacgauu | 1025 |
| 1476_6 | uCfgUfaAfgGfCfgacaGfgAfgcguu | 988 | cgcuCfcUfGfUfcgccuuacgauu | 1026 |

An FXII RNAi agent can be prepared or provided as a salt, mixed salt, or a free-acid. The RNAi agents described herein, upon delivery to a cell expressing an FXII gene, inhibit or knockdown expression of one or more FXII genes *in vivo.*

### Targeting Groups, Linking Groups, and Delivery Vehicles

An FXII RNAi agent can be conjugated to one or more non-nucleotide groups including, but not limited to, a targeting group, linking group, delivery polymer, or a delivery vehicle. The non-nucleotide group can enhance targeting, delivery or attachment of the RNAi agent. Examples of targeting groups and linking groups are provided in Table 7. The non-nucleotide group can be covalently linked to the 3' and/or 5' end of either the sense strand and/or the antisense strand. In some embodiments, an FXII RNAi agent contains a non-nucleotide group linked to the 3' and/or 5' end of the sense strand. In some embodiments, a non-nucleotide group is linked to the 5' end of an FXII RNAi agent sense strand. A non-nucleotide group can be linked directly or indirectly to the RNAi agent via a linker/linking group. In some embodiments, a non-nucleotide group is linked to the RNAi agent via a labile, cleavable, or reversible bond or linker.

A non-nucleotide group can enhance the pharmacokinetic or biodistribution properties of an RNAi agent or conjugate to which it is attached to improve cell- or tissue-specific distribution and cell-specific uptake of the RNAi agent or conjugate. In some embodiments, a non-nucleotide group enhances endocytosis of the RNAi agent.

Targeting groups or targeting moieties can enhance the pharmacokinetic or biodistribution properties of a conjugate or RNAi agent to which they are attached to improve cell-specific distribution and cell-specific uptake of the conjugate or RNAi agent. A targeting group can be monovalent, divalent, trivalent, tetravalent, or have higher valency for the target to which it is directed. Representative targeting groups include, without limitation, compounds with affinity to cell surface molecules, cell receptor ligands, haptens, antibodies, monoclonal antibodies, antibody fragments, and antibody mimics with affinity to cell surface molecules. In some embodiments, a targeting group is linked to an RNAi agent using a linker, such as a PEG linker or one, two, or three abasic and/or ribitol (abasic ribose) residues, which in some instances can serve as linkers. In some embodiments, a targeting group comprises a galactose-derivative cluster.

The FXII RNAi agents described herein can be synthesized having a reactive group, such as an amine group, at the 5'-terminus. The reactive group can be used to subsequently attach a targeting group using methods typical in the art.

A targeting group can comprise an asialoglycoprotein receptor ligand. An asialoglycoprotein receptor ligand can include or consists of one or more galactose derivatives. As used herein, the term galactose derivative includes both galactose and derivatives of galactose having affinity for the asialoglycoprotein receptor that is equal to or greater than that of galactose. Galactose derivatives include, but are not limited to: galactose, galactosamine, N-formylgalactosamine, N-acetyl-galactosamine, N-propionyl-galactosamine, N-n-butanoyl-galactosamine, and N-iso-butanoylgalactos-amine (see for example: S.T. Iobst and K. Drickamer, J.B.C., 1996, 271, 6686). Galactose derivatives, and clusters of galactose derivatives, that are useful for *in vivo* targeting of oligonucleotides and other molecules to the liver are known in the art (see, for example, Baenziger and Fiete, 1980, Cell, 22, 611-620; Connolly et al., 1982, J. Biol. Chem., 257, 939-945).

Galactose derivatives have been used to target molecules to hepatocytes *in vivo* through their binding to the asialoglycoprotein receptor expressed on the surface of hepatocytes. Binding of asialoglycoprotein receptor ligands to the asialoglycoprotein receptor(s) facilitates cell-specific targeting to hepatocytes and endocytosis of the molecule into hepatocytes. Asialoglycoprotein receptor ligands can be monomeric (e.g., having a single galactose derivative) or multimeric (e.g., having multiple galactose derivatives). The galactose derivative or galactose derivative cluster can be attached to the 3' or 5' end of the sense or antisense strand of the RNAi agent using methods known in the art. The preparation of targeting groups, such as galactose derivative clusters, is described in, for example, U.S. Patent Application Serial No. 15/452,324 and U.S. Patent Publication No. US 2017/0253875.

As used herein, a galactose derivative cluster comprises a molecule having two to four terminal galactose derivatives. A terminal galactose derivative is attached to a molecule through its C-1 carbon. In some embodiments, the galactose derivative cluster is a galactose derivative trimer (also referred to as tri-antennary galactose derivative or tri-valent galactose derivative). The galactose derivative cluster can comprise N-acetyl-galactosamines. The galactose derivative cluster can comprise three N-acetyl-galactosamines. The galactose derivative cluster can be a galactose derivative tetramer (also referred to as tetra-antennary galactose derivative or tetra-valent galactose derivative). The galactose derivative cluster can comprise four N-acetyl-galactosamines.

As used herein, a galactose derivative trimer contains three galactose derivatives, each linked to a central branch point. As used herein, a galactose derivative tetramer contains four galactose derivatives, each linked to a central branch point. The galactose derivatives can be attached to the central branch point through the C-1 carbons of the saccharides. In some embodiments, the galactose derivatives are linked to the branch point via linkers or spacers. In some embodiments, the linker or spacer is a flexible hydrophilic spacer, such as a PEG group (see, for example, U.S. Patent No. 5,885,968; Biessen et al. J. Med. Chem. 1995 Vol. 39 p. 1538-1546). The PEG spacer can be a PEG₃ spacer. The branch point can be any small molecule which permits attachment of three galactose derivatives and further permits attachment of the branch point to the RNAi agent. An example of branch point group is a di-lysine or di-glutamate. Attachment of the branch point to the RNAi agent can occur through a linker or spacer. The linker or spacer can comprise a flexible hydrophilic spacer, such as, but not limited to, a PEG spacer. The linker can comprise a rigid linker, such as a cyclic group. A galactose derivative can comprise or consists of N-acetyl-galactosamine. The galactose derivative cluster can be comprised of a galactose derivative tetramer, which can be, for example, an N-acetyl-galactosamine tetramer.

Embodiments of the present disclosure include pharmaceutical compositions for use in delivering an FXII RNAi agent to a liver cell *in vivo.* Such pharmaceutical compositions can include, for example, an FXII RNAi agent conjugated to a galactose derivative cluster.

Targeting groups include for example (PAZ), (NAG13), (NAG13)s, (NAG18), (NAG18)s, (NAG24), (NAG24)s, (NAG25), (NAG25)s, (NAG26), (NAG26)s, (NAG27), (NAG27)s, (NAG28), (NAG28)s, (NAG29), (NAG29)s, (NAG30), (NAG30)s, (NAG31), (NAG31)s, (NAG32), (NAG32)s, (NAG33), (NAG33)s, (NAG34), (NAG34)s, (NAG35), (NAG35)s, (NAG36), (NAG36)s, (NAG37), (NAG37)s, (NAG38), (NAG38)s, (NAG39), and (NAG39)s as defined in Table 7. Other targeting groups, including galactose cluster targeting ligands, are known in the art.

A linking group can be conjugated to the RNAi agent. The linking group facilitates covalent linkage of the agent to a targeting group or delivery polymer or delivery vehicle. The linking group can be linked to the 3' or the 5' end of the RNAi agent sense strand or antisense strand. In some embodiments, the linking group is linked to the RNAi agent sense strand. The linking group can be conjugated to the 5' or 3' end of an RNAi agent sense strand. In some embodiments, a linking group is conjugated to the 5' end of an RNAi agent sense strand. Examples of linking groups, can include, but are not limited to: reactive groups such a primary amines and alkynes, alkyl groups, abasic nucleotides, ribitol (abasic ribose), and/or PEG groups.

A linker or linking group is a connection between two atoms that links one chemical group (such as an RNAi agent) or segment of interest to another chemical group (such as a targeting group or delivery polymer) or segment of interest via one or more covalent bonds. A labile linkage contains a labile bond. A linkage may optionally include a spacer that increases the distance between the two joined atoms. A spacer can further add flexibility and/or length to the linkage. Spacers can include, but are not be limited to, alkyl groups, alkenyl groups, alkynyl groups, aryl groups, aralkyl groups, aralkenyl groups, and aralkynyl groups; each of which can contain one or more heteroatoms, heterocycles, amino acids, nucleotides, and saccharides. Spacer groups are well known in the art and the preceding list is not meant to limit the scope of the description.

Any of the FXII RNAi agent nucleotide sequences listed in Tables 2, 3, 4, or 6, whether modified or unmodified, may contain 3' or 5' targeting groups or linking groups. Any of the FXII RNAi agent sequences listed in Tables 3 or 4 which contain a 3' or 5' targeting group or linking group, may alternatively contain no 3' or 5' targeting group or linking group, or may contain a different 3' or 5' targeting group or linking group including, but not limited to, those depicted in Table 7. Any of the FXII RNAi agent duplexes listed in Table 2, Table 5, or Table 6, whether modified or unmodified, may further comprise a targeting group or linking group, including, but not limited to, those depicted in Table 7, and the targeting group or linking group may be attached to the 3' or 5' terminus of either the sense strand or the antisense strand of the FXII RNAi agent duplex.

Examples of targeting groups and linking groups are provided in Table 7. Table 4 provides several examples of FXII RNAi agent sense strands having a targeting group or linking group linked to the 5' or 3' end.

**Table 7. Structures Representing Various Modified Nucleotides, Targeting Groups, and Linking Groups**

| | |
|---|---|
| 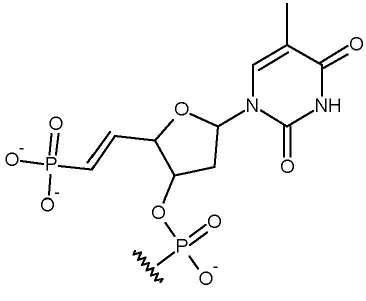 vpdT | 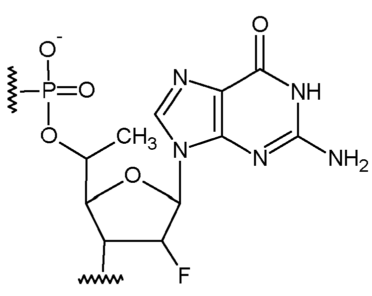 5Me-Gf |
| 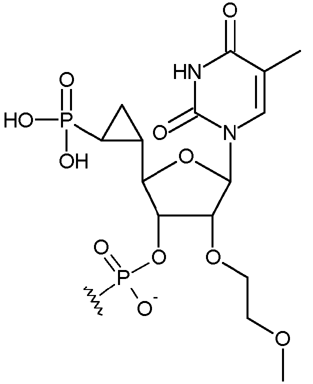 cPrpTM | 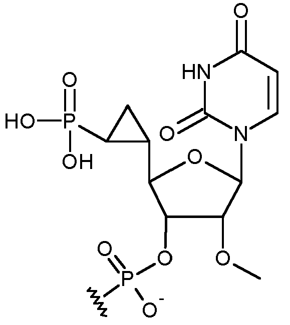 (cPrp)u |
| 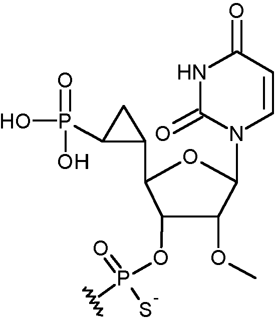 | |
| cPrpus | |
| 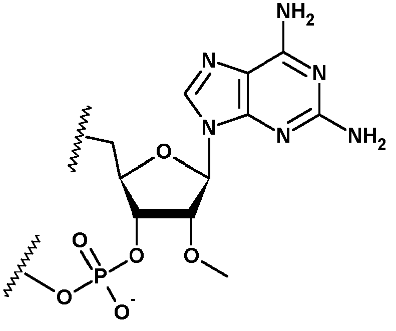 a_2N | 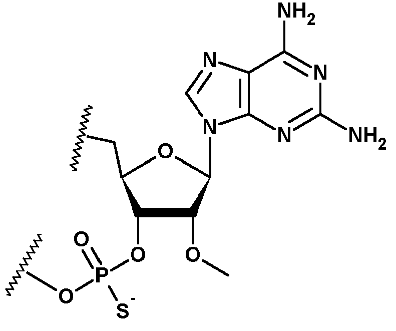 a_2Ns |
| 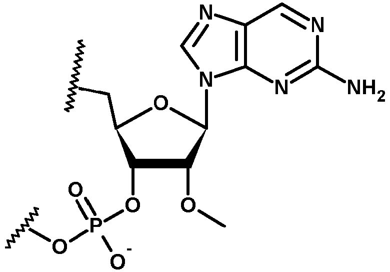 pu_2N | 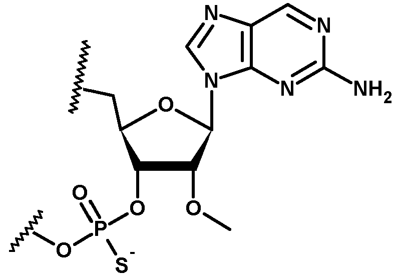 pu_2Ns |
| 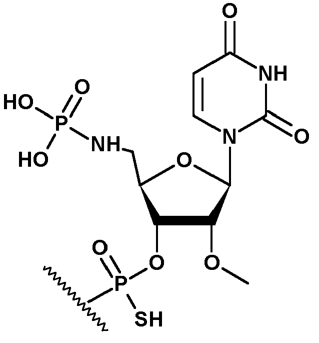 Npus | 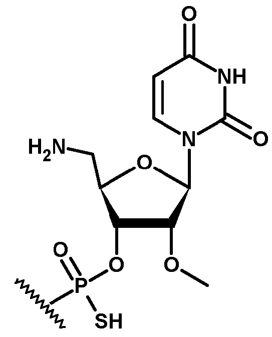 Nus |
| 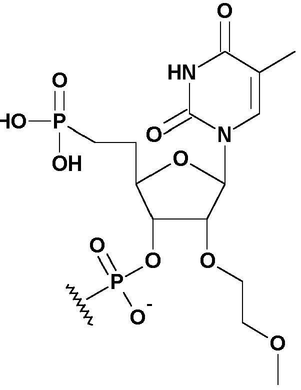 epTM | 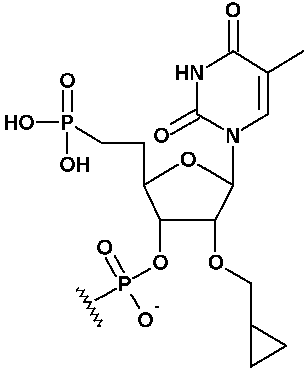 epTcPr |
| When positioned internally on oligonucleotide: | |
| linkage towards 5' end of oligonucleotide | |
| 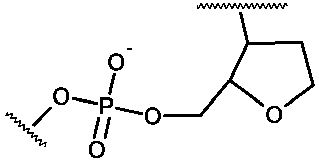 | |
| linkage towards 3' end of oligonucleotide (invAb) | |
| When positioned internally on oligonucleotide: | |
| linkage towards 5' end of oligonucleotide | |
| 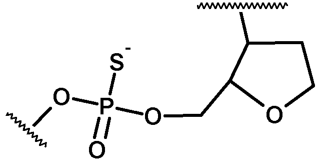 | |
| linkage towards 3' end of oligonucleotide (invAb)s | |
| When positioned at the 3' terminal end of oligonucleotide: | |
| linkage towards 5' end of oligonucleotide | |
| 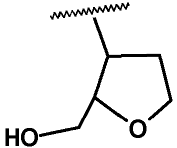 | |
| (invAb) | |
| 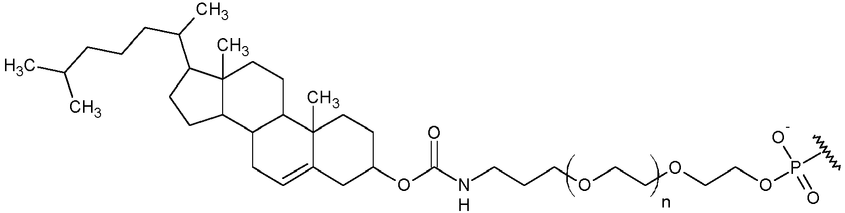 | |
| (Chol-TEG) | |
| 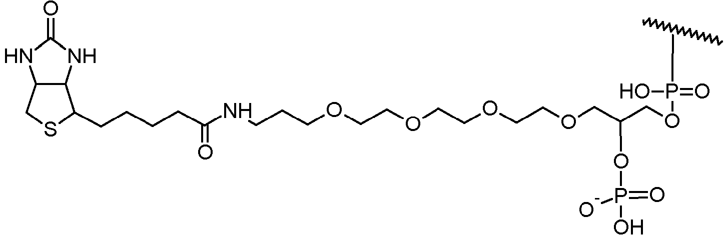 (TEG-Biotin) | |
| 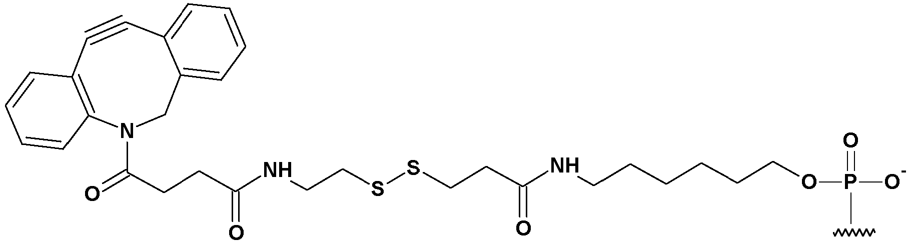 (C6-SS-Alk) or (Alk-SS-C6) | |
| 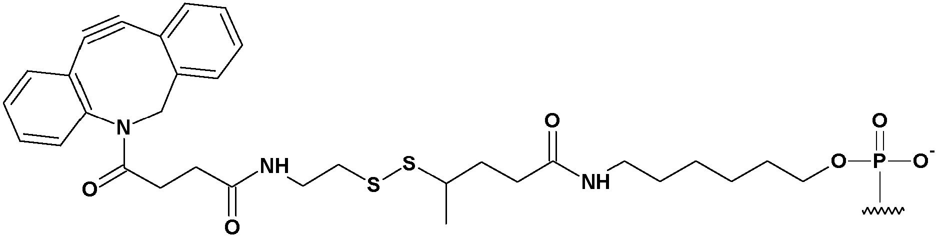 (C6-SS-Alk-Me) | |
| 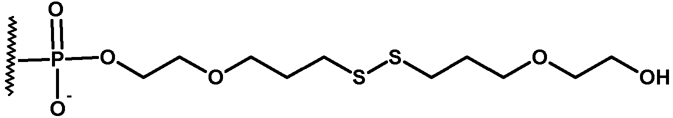 (PEG-C3-SS) | |
| 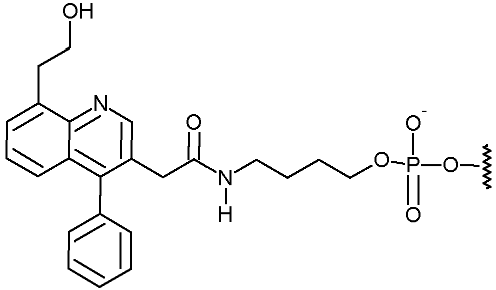 (PAZ) | |
| 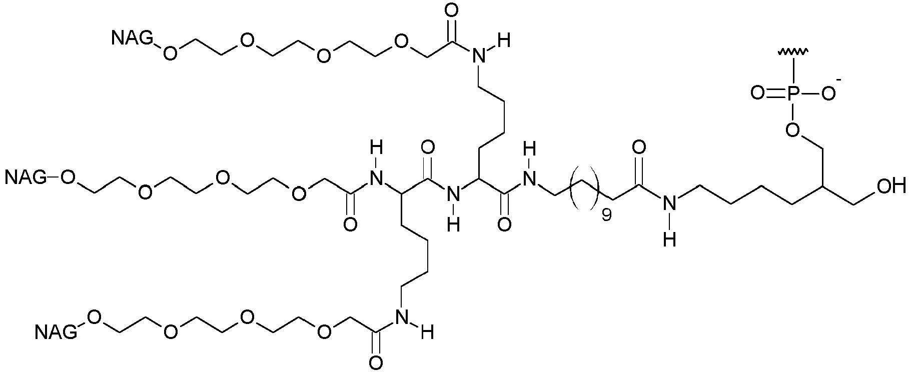 (NAG13) | |
| 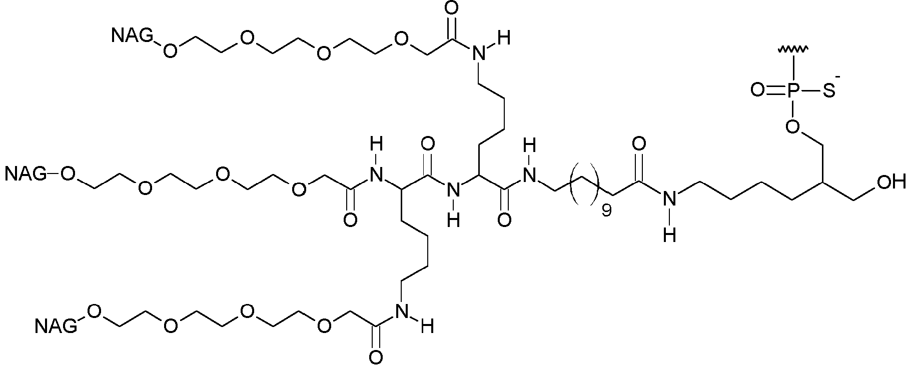 (NAG13)s | |
| 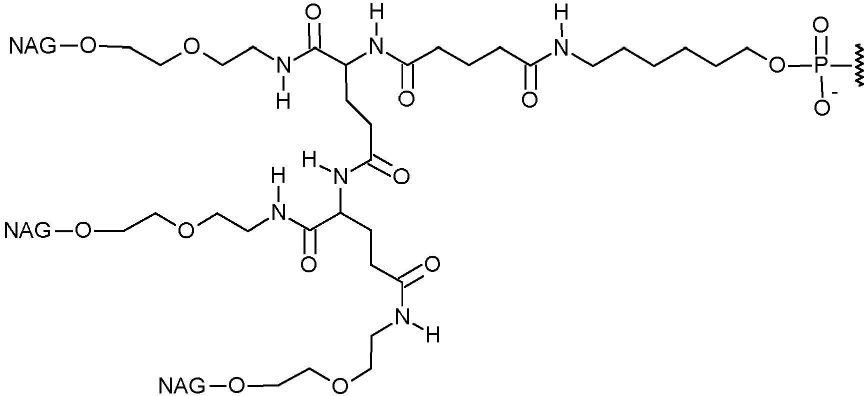 (NAG18) | |
| 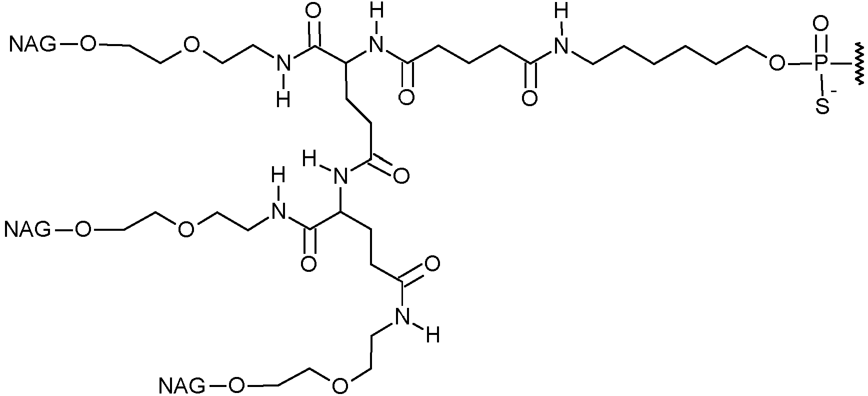 (NAG18)s | |
| 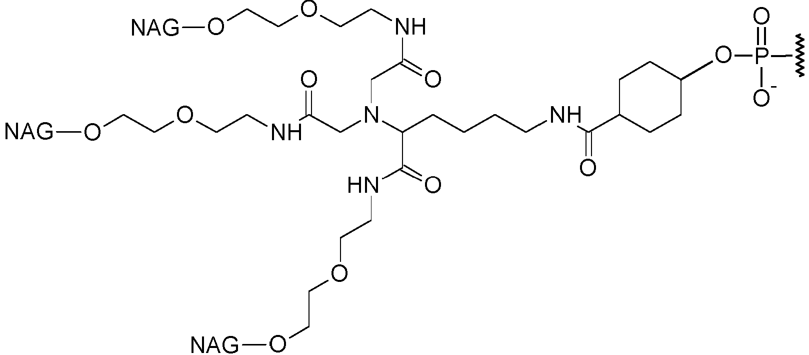 (NAG24) | |
| 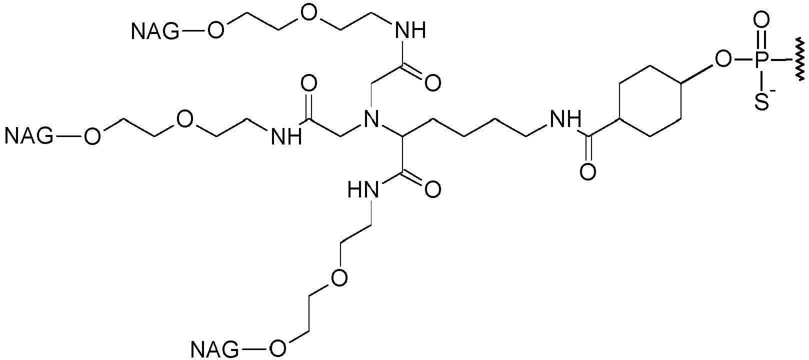 (NAG24)s | |
| 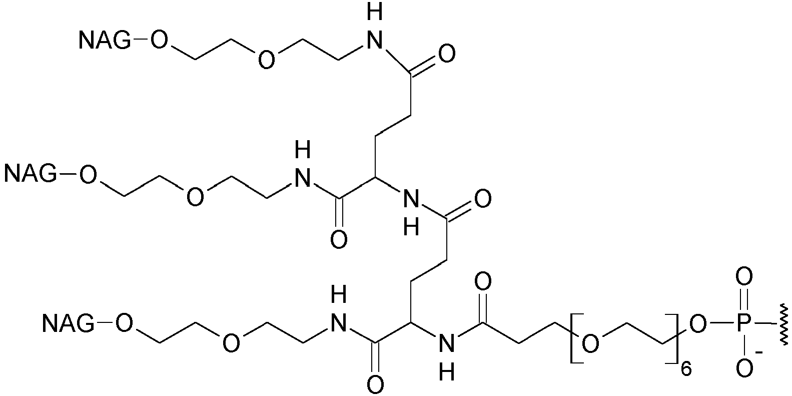 (NAG25) | |
| 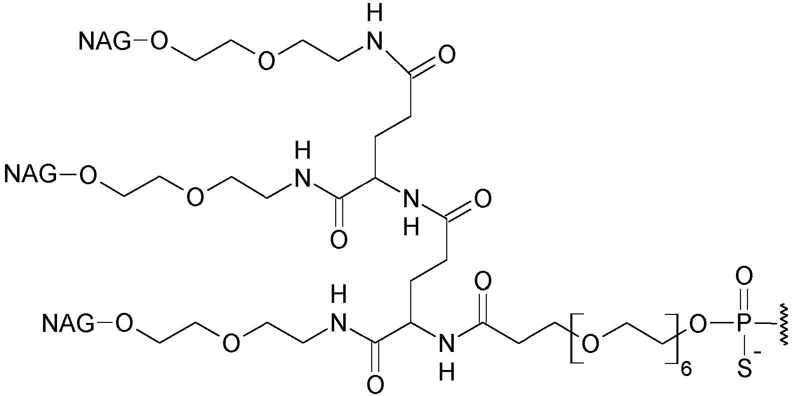 (NAG25)s | |
| 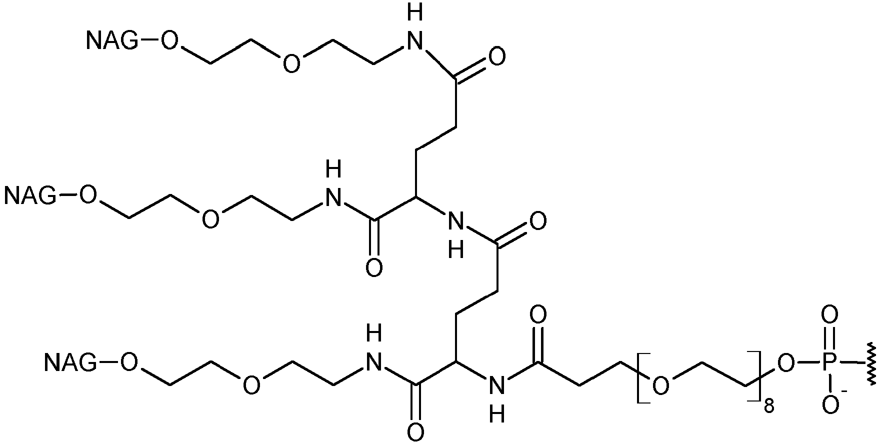 (NAG26) | |
| 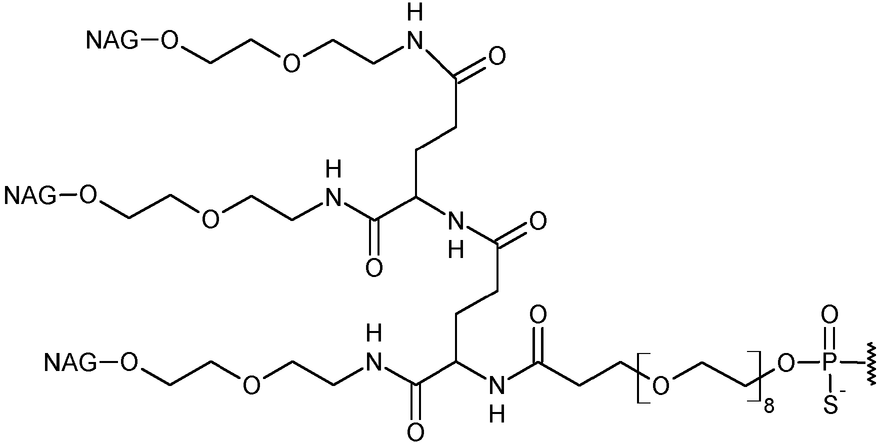 (NAG26)s | |
| 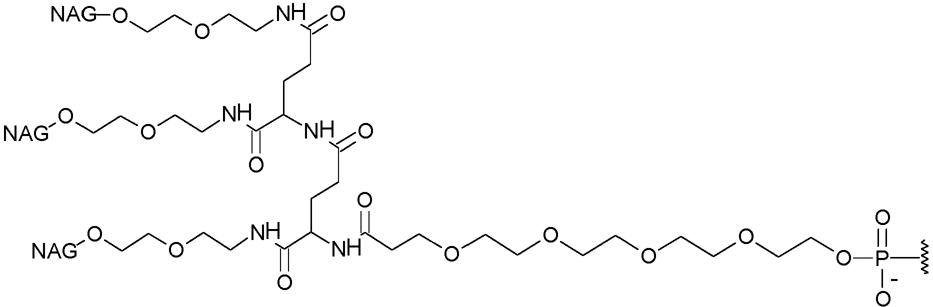 (NAG27) | |
| 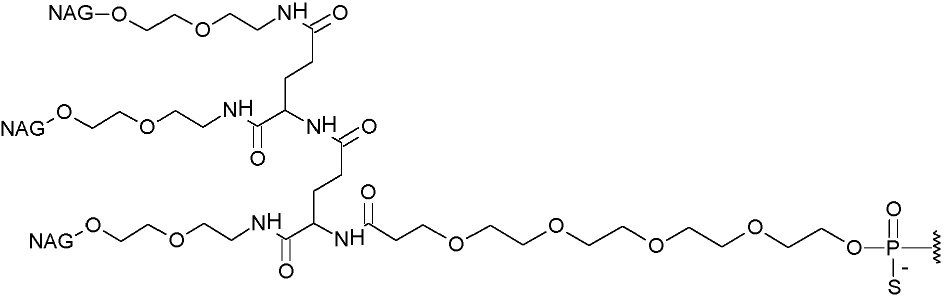 (NAG27)s | |
| 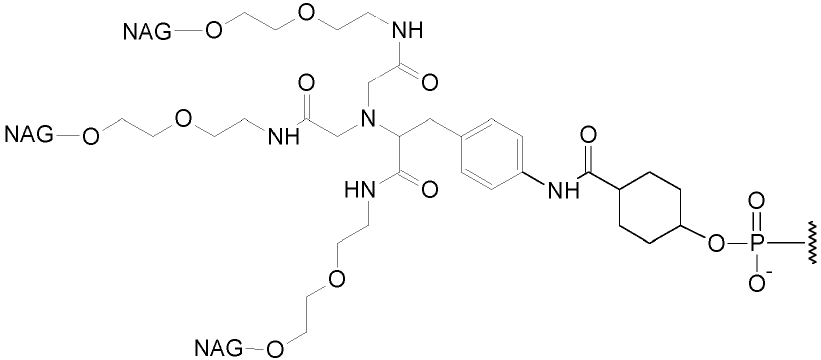 (NAG28) | |
| 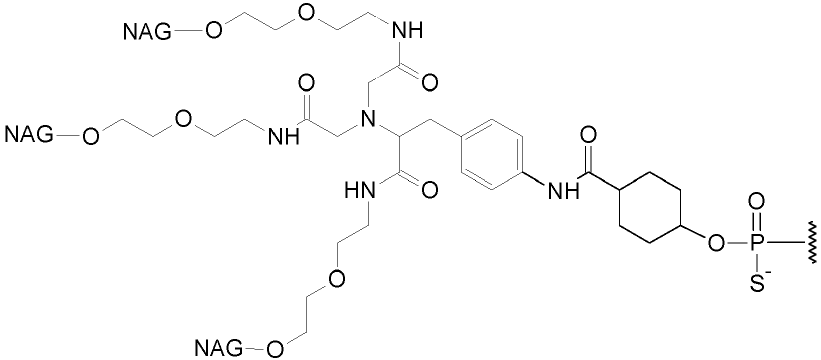 (NAG28)s | |
| 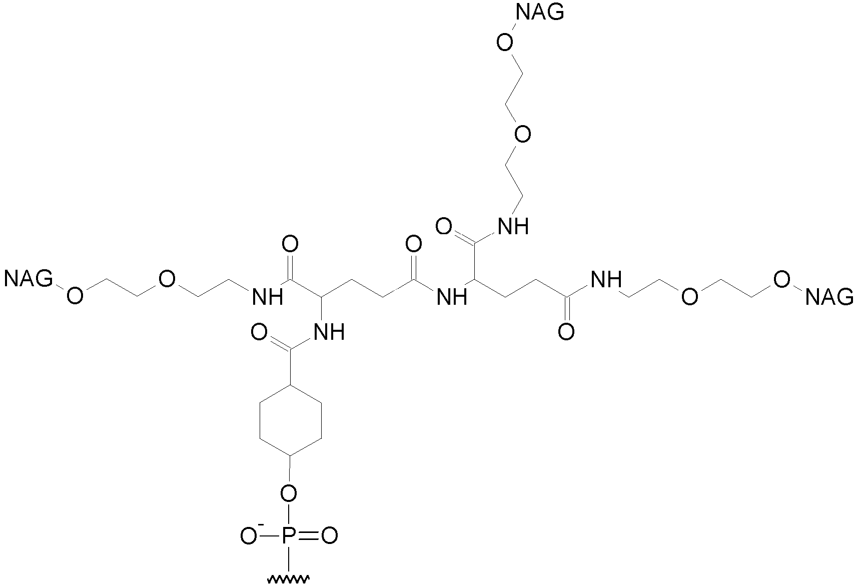 (NAG29) | |
| 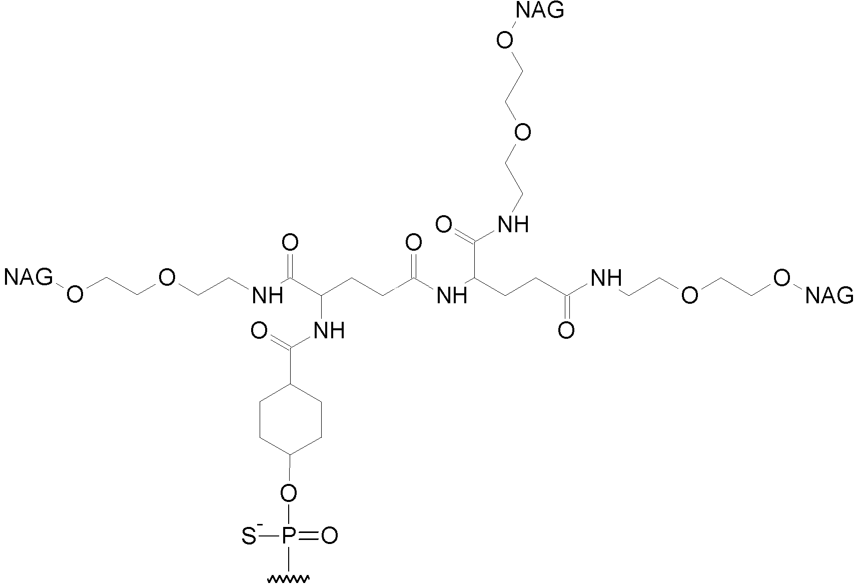 (NAG29)s | |
| 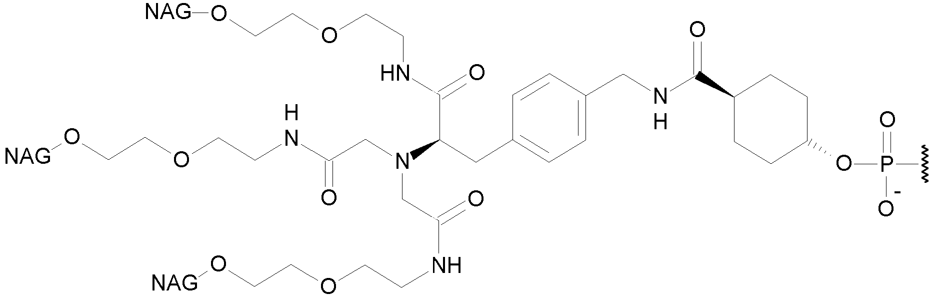 (NAG30) | |
| 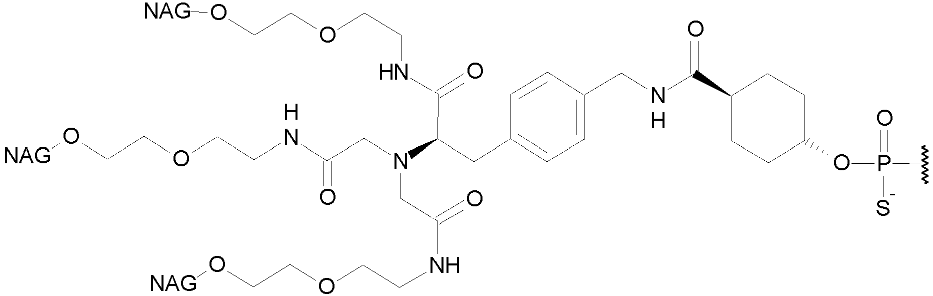 (NAG30)s | |
| 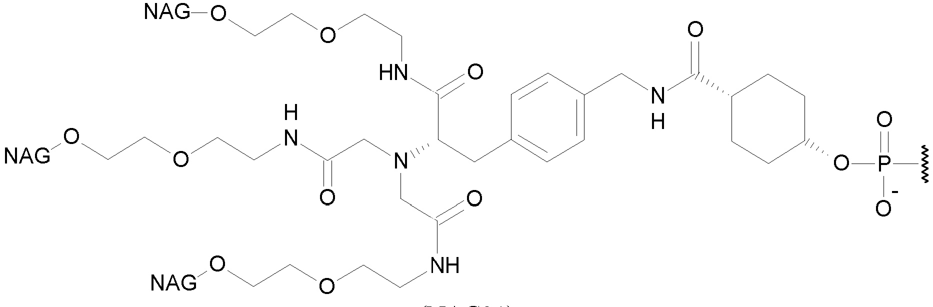 (NAG31) | |
| 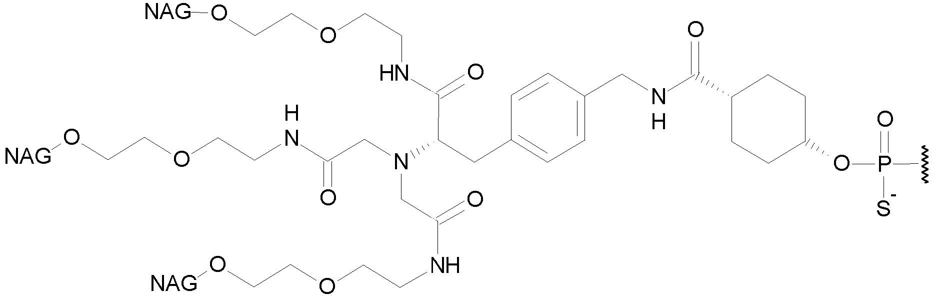 (NAG31)s | |
| 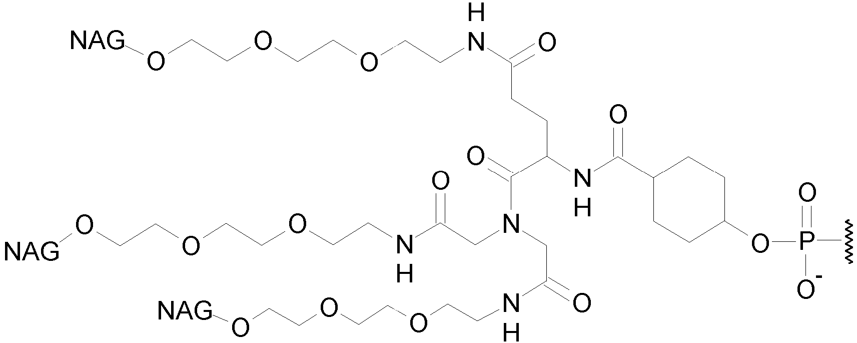 (NAG32) | |
| 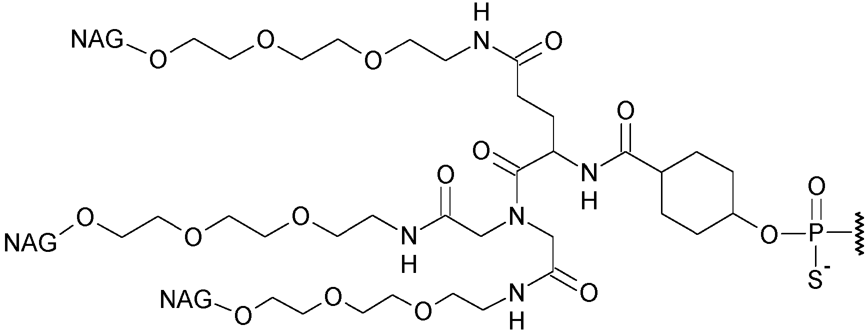 (NAG32)s | |
| 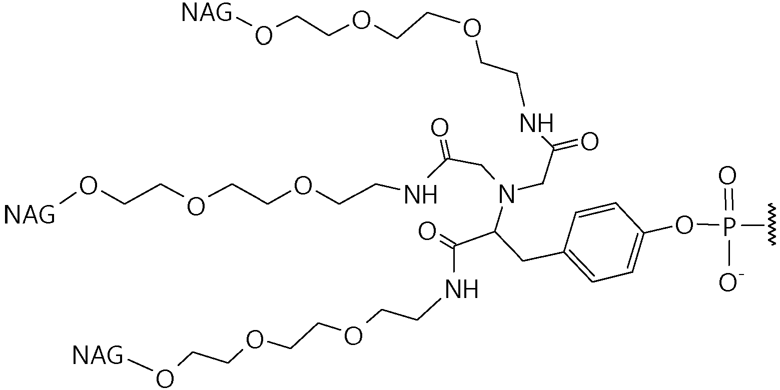 (NAG33) | |
| 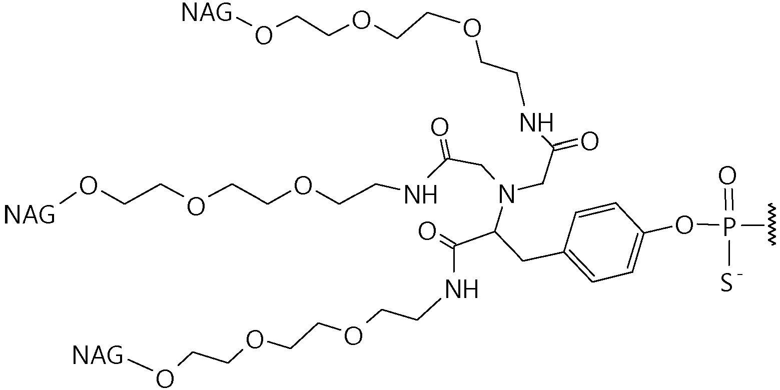 (NAG33)s | |
| 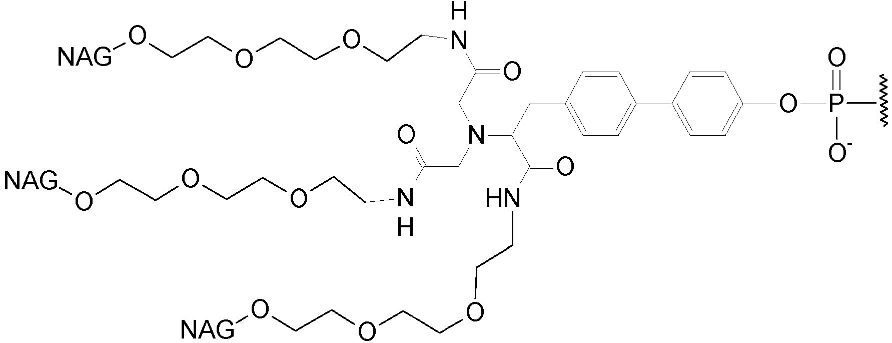 (NAG34) | |
| 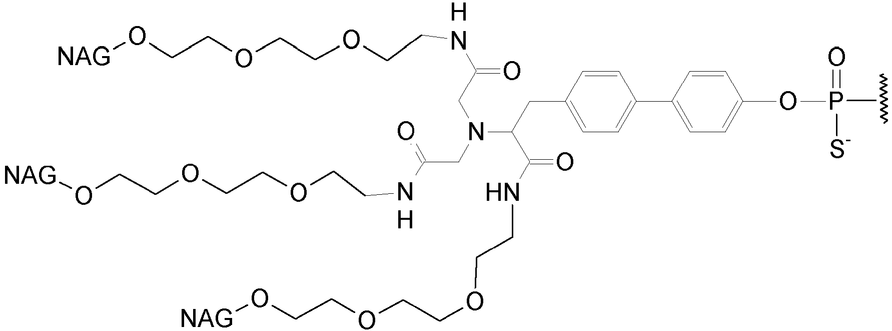 (NAG34)s | |
| 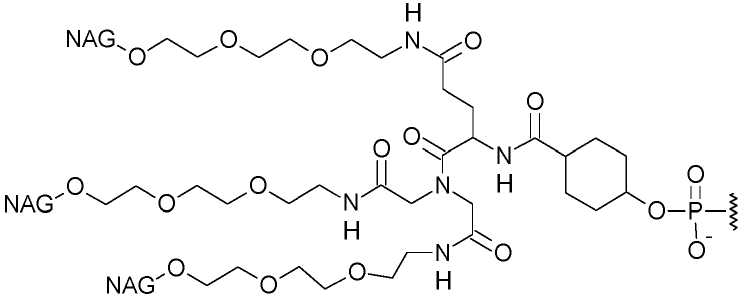 (NAG35) | |
| 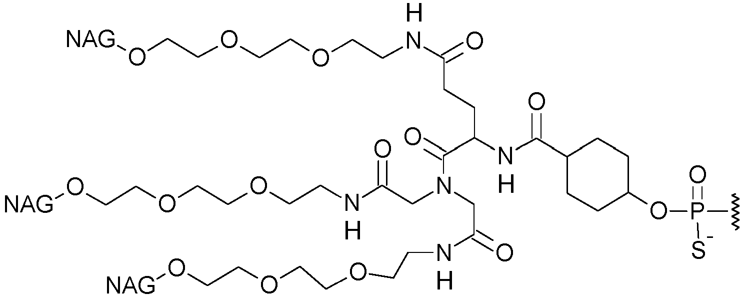 (NAG35)s | |
| 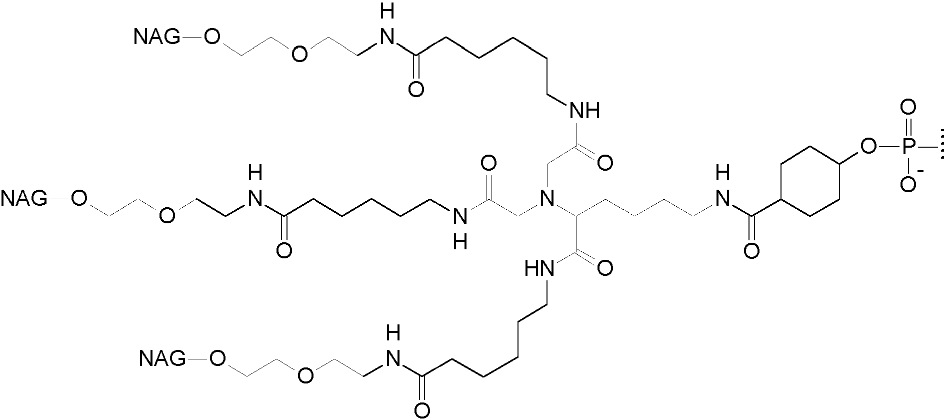 (NAG36) | |
| 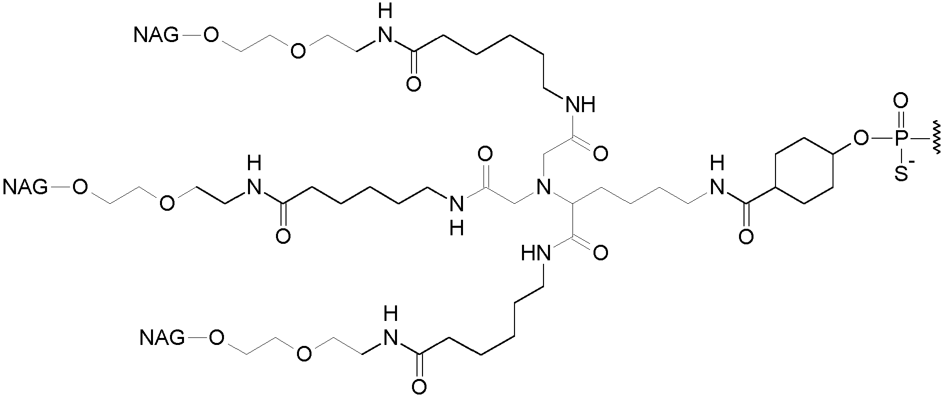 (NAG36)s | |
| 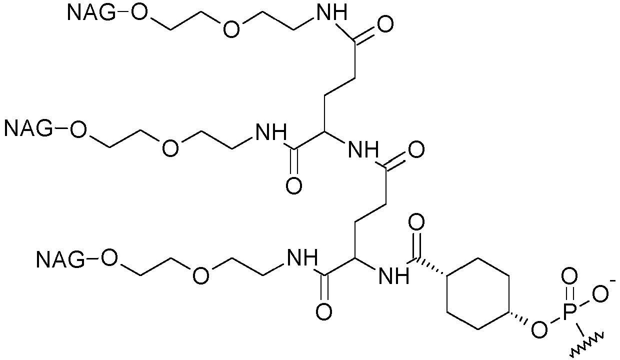 (NAG37) | |
| 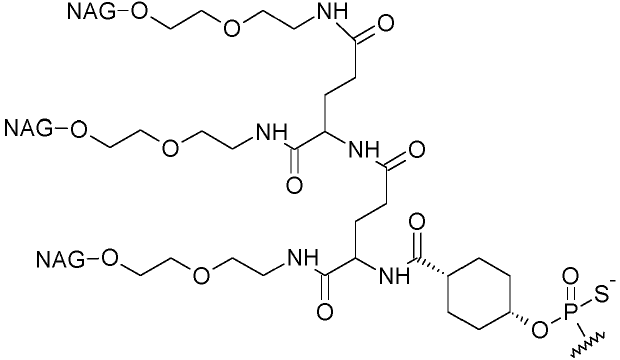 (NAG37)s | |
| 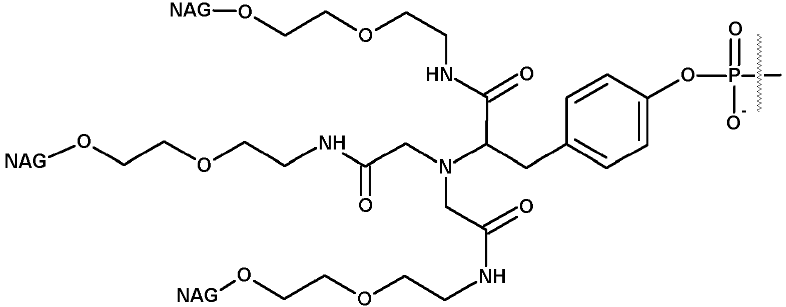 (NAG 38) | |
| 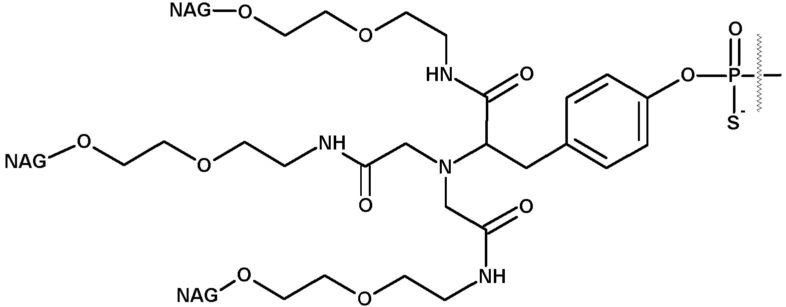 (NAG 38)s | |
| 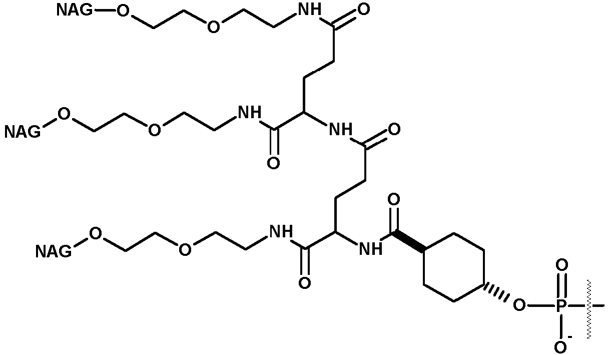 (NAG39) | |
| 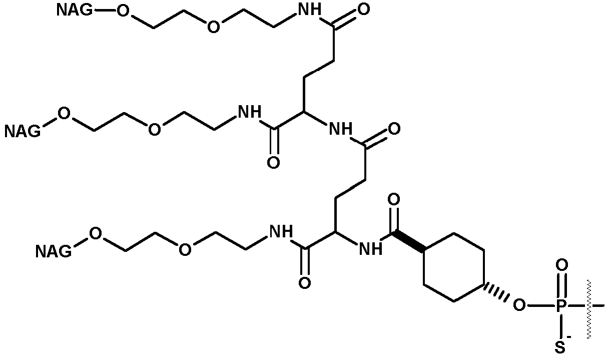 (NAG39)s | |

In each of the above structures in Table 7, NAG comprises an N-acetyl-galactosamine or another asialoglycoprotein receptor ligand, as would be understood by a person of ordinary skill in the art to be attached in view of the structures above and description provided herein. For example, in some embodiments, NAG in the structures provided in Table 7 is represented by the following structure:

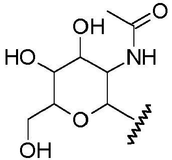

### (N-acetyl-galactosamine)

Each (NAGx) can be attached to an FXII RNAi agent via a phosphate group (as in (NAG25), (NAG30), and (NAG31)), or a phosphorothioate group, (as is (NAG25)s, (NAG29)s, (NAG30)s, (NAG31)s, or (NAG37)s), or another linking group.

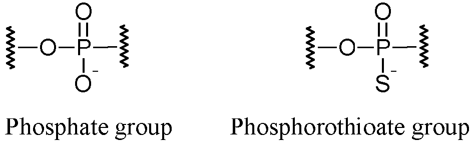

Other linking groups known in the art may be used.

A delivery vehicle can be used to deliver an RNAi agent to a cell or tissue. A delivery vehicle is a compound that improves delivery of the RNAi agent to a cell or tissue. A delivery vehicle can include, or consist of, but is not limited to: a polymer, such as an amphipathic polymer, a membrane active polymer, a peptide, a melittin peptide, a melittin-like peptide (MLP), a lipid, a reversibly modified polymer or peptide, or a reversibly modified membrane active polyamine. In some embodiments, the RNAi agents can be combined with lipids, nanoparticles, polymers, liposomes, micelles, DPCs or other delivery systems available in the art. The RNAi agents can also be chemically conjugated to targeting groups, lipids (including, but not limited to cholesterol and cholesteryl derivatives), nanoparticles, polymers, liposomes, micelles, DPCs (see, for example WO 2000/053722, WO 2008/0022309, WO 2011/104169, and WO 2012/083185, WO 2013/032829, WO 2013/158141) or other delivery systems available in the art.

### Pharmaceutical Compositions and Formulations

The FXII RNAi agents disclosed herein can be prepared as pharmaceutical compositions or formulations. In some embodiments, pharmaceutical compositions include at least one FXII RNAi agent. These pharmaceutical compositions are particularly useful in the inhibition of the expression of the target mRNA in a target cell, a group of cells, a tissue, or an organism. The pharmaceutical compositions can be used to treat a subject having a disease or disorder that would benefit from reduction in the level of the target mRNA, or inhibition in expression of the target gene. The pharmaceutical compositions can be used to treat a subject at risk of developing a disease, disorder, or condition that would benefit from reduction of the level of the target mRNA or an inhibition in expression the target gene. In some embodiments, one or more pharmaceutically acceptable excipients (including vehicles, carriers, diluents, and/or delivery polymers) are added to the pharmaceutical compositions including an FXII RNAi agent, thereby forming a pharmaceutical formulation suitable for *in vivo* delivery to a subject, including a human.

The described pharmaceutical compositions including an FXII RNAi agent can be used for treating or managing clinical presentations in a subject that would benefit from the inhibition of expression of a FXII gene, such as include HAE, AAE, ACE inhibitor associated angioedema, allergic angioedema, INAE, idiopathic angioedema, thrombosis, VTE, thrombotic occlusive disease, and peri-operative venous occlusive disease prophylaxis.

The described pharmaceutical compositions including an FXII RNAi agent can be used to treat at least one symptom in a subject having a disease or disorder that would benefit from reduction or inhibition in expression of FXII mRNA.

The route of administration is the path by which an FXII RNAi agent is brought into contact with the body. In general, methods of administering drugs and oligonucleotides and nucleic acids for treatment of a mammal are well known in the art and can be applied to administration of the compositions described herein. The FXII RNAi agents disclosed herein can be administered via any suitable route in a preparation appropriately tailored to the particular route. Thus, herein described pharmaceutical compositions can be administered by injection, for example, intravenously, intramuscularly, intracutaneously, subcutaneously, intraarticularly, or intraperitoneally.

The pharmaceutical compositions including an FXII RNAi agent described herein can be delivered to a cell, group of cells, tissue, or subject using oligonucleotide delivery technologies known in the art. In general, any suitable method recognized in the art for delivering a nucleic acid molecule (*in vitro* or *in vivo*) can be adapted for use with the compositions described herein. For example, delivery can be by local administration, (e.g., direct injection, implantation, or topical administering), systemic administration, or subcutaneous, intravenous, intraperitoneal, or parenteral routes, including intracranial (e.g., intraventricular, intraparenchymal and intrathecal), intramuscular, transdermal, airway (aerosol), nasal, oral, rectal, or topical (including buccal and sublingual) administration.

Accordingly, in some embodiments, the pharmaceutical compositions described herein comprise one or more pharmaceutically acceptable excipients. The pharmaceutical compositions described herein are formulated for administration to a subject.

As used herein, a pharmaceutical composition or medicament includes a pharmacologically effective amount of at least one of the described FXII RNAi agents and one or more pharmaceutically acceptable excipients. Pharmaceutically acceptable excipients (excipients) are substances other than the Active Pharmaceutical Ingredient (API, therapeutic product, e.g., FXII RNAi agent) that are intentionally included in the drug delivery system. Excipients do not exert or are not intended to exert a therapeutic effect at the intended dosage. Excipients can act to a) aid in processing of the drug delivery system during manufacture, b) protect, support, or enhance stability, bioavailability or patient acceptability of the API, c) assist in product identification, and/or d) enhance any other attribute of the overall safety, effectiveness, of delivery of the API during storage or use. A pharmaceutically acceptable excipient may or may not be an inert substance.

Excipients include, but are not limited to: absorption enhancers, anti-adherents, antifoaming agents, anti-oxidants, binders, buffering agents, carriers, coating agents, colors, delivery enhancers, delivery polymers, dextran, dextrose, diluents, disintegrants, emulsifiers, extenders, fillers, flavors, glidants, humectants, lubricants, oils, polymers, preservatives, saline, salts, solvents, sugars, suspending agents, sustained release matrices, sweeteners, thickening agents, tonicity agents, vehicles, water-repelling agents, and wetting agents.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor^{®} ELTM (BASF, Parsippany, NJ) or phosphate buffered saline (PBS). It should be stable under the conditions of manufacture and storage and should be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filter sterilization. Generally, dispersions are prepared by incorporating the active compound into a sterile vehicle, which contains a basic dispersion medium and the required other ingredients from those enumerated above. In the case of sterile powders for the preparation of sterile injectable solutions, methods of preparation include vacuum drying and freeze-drying which yields a powder of the active ingredient plus any additional desired ingredient from a previously sterile-filtered solution thereof.

Formulations suitable for intra-articular administration can be in the form of a sterile aqueous preparation of the drug that can be in microcrystalline form, for example, in the form of an aqueous microcrystalline suspension. Liposomal formulations or biodegradable polymer systems can also be used to present the drug for both intra-articular and ophthalmic administration.

The active compounds can be prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Liposomal suspensions can also be used as pharmaceutically acceptable carriers. These can be prepared according to methods known to those skilled in the art, for example, as described in U.S. Patent No. 4,522,811.

The FXII RNAi agents can be formulated in compositions in dosage unit form for ease of administration and uniformity of dosage. Dosage unit form refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the therapeutic effect to be achieved, and the limitations inherent in the art of compounding such an active compound for the treatment of individuals.

A pharmaceutical composition can contain other additional components commonly found in pharmaceutical compositions. Such additional components include, but are not limited to: anti-pruritics, astringents, local anesthetics, or anti-inflammatory agents (e.g., antihistamine, diphenhydramine, etc.). It is also envisioned that cells, tissues, or isolated organs that express or comprise the herein defined RNAi agents may be used as "pharmaceutical compositions." As used herein, "pharmacologically effective amount," "therapeutically effective amount," or simply "effective amount" refers to that amount of an RNAi agent to produce a pharmacological, therapeutic or preventive result.

Generally, an effective amount of an active compound will be in the range of from about 0.1 to about 100 mg/kg of body weight/day, e.g., from about 1.0 to about 50 mg/kg of body weight/day. In some embodiments, an effective amount of an active compound will be in the range of from about 0.25 to about 5 mg/kg of body weight per dose. In some embodiments, an effective amount of an active ingredient will be in the range of from about 0.5 to about 4 mg/kg of body weight per dose. The amount administered will also likely depend on such variables as the overall health status of the patient, the relative biological efficacy of the compound delivered, the formulation of the drug, the presence and types of excipients in the formulation, and the route of administration. Also, it is to be understood that the initial dosage administered can, in some instances, be increased beyond the above upper level to rapidly achieve the desired blood-level or tissue level, or the initial dosage can, in some instances, be smaller than the optimum.

For treatment of disease or for formation of a medicament or composition for treatment of a disease, the pharmaceutical compositions described herein including an FXII RNAi agent can be combined with an excipient or with a second therapeutic agent or treatment including, but not limited to: a second or other RNAi agent, a small molecule drug, an antibody, an antibody fragment, peptide and/or aptamer.

The described FXII RNAi agents, when added to pharmaceutically acceptable excipients or adjuvants, can be packaged into kits, containers, packs, or dispensers. The pharmaceutical compositions described herein can be packaged in pre-filled syringes or vials.

### Methods of Treatment and Inhibition of Expression

The FXII RNAi agents disclosed herein can be used to treat a subject (e.g., a human or other mammal) having a disease or disorder that would benefit from administration of the compound. In some embodiments, the RNAi agents disclosed herein can be used to treat a subject (e.g., a human) that would benefit from a reduction and/or inhibition of FXII gene expression, for example, a subject that has been diagnosed with or is at risk of developing symptoms related to hereditary angioedema (HAE), acquired angioedema (AAE), ACE inhibitor associated angioedema, allergic angioedema, nonhistaminergic angioedema (INAE), idiopathic angioedema, thrombosis, venous thromboembolism (VTE), thrombotic occlusive disease, and/or peri-operative venous occlusive disease prophylaxis. Treatment of a subject may include therapeutic and/or prophylactic treatment. The subject is administered a therapeutically effective amount of any one or more of the FXII RNAi agents described herein. The subject can be a human, patient, or human patient. The subject may be an adult, adolescent, child, or infant. Administration of a pharmaceutical composition can be to a human being or animal.

In some embodiments, the FXII RNAi agents described herein are used to treat a subject that would benefit from a reduction and/or inhibition of FXII gene expression. In some embodiments, the described FXII RNAi agents are used to treat (including prophylactically) at least one symptom or pathological mediated at least in part by FXII expression.

In some embodiments, the FXII RNAi agents are used to treat or manage a pathological state (such as a condition or disease) of a subject, for which the pathological state is mediated at least in part by FXII expression.

The gene expression level and/or mRNA level of an FXII gene in a subject to whom a described FXII RNAi agent is administered can be reduced by at least about 5%, 10%, 15%, 20%, 25%, 30%, 35%, 40%, 45%, 50%, 55%, 60%, 65%, 70%, 75%, 80%, 85%, 95%, 96%, 97%, 98%, 99%, or greater than 99% relative to the subject prior to being administered the FXII RNAi agent or to a subject not receiving the FXII RNAi agent. The gene expression level and/or mRNA level in the subject is reduced in a cell, group of cells, and/or tissue of the subject.

The protein level of FXII in a subject to whom a described FXII RNAi agent has been administered is reduced by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or greater than 99% relative to the subject prior to being administered the FXII RNAi agent or to a subject not receiving the FXII RNAi agent. The protein level in the subject is reduced in a cell, group of cells, tissue, blood, and/or other fluid of the subject.

A reduction in FXII gene expression, FXII mRNA, or FXII protein levels can be assessed and quantified by general methods known in the art. The Examples disclosed herein set forth generally known methods for assessing inhibition of FXII gene expression and reduction in FXII protein levels. The reduction or decrease in FXII mRNA level and/or protein level are collectively referred to herein as a reduction or decrease in FXII or inhibiting or reducing the expression of FXII.

### Cells, Tissues, and non-Human Organisms

*In vitro* cells or tissues that include at least one of the FXII RNAi agents described herein is contemplated. The cell or issue is made by delivering the RNAi agent to the cell or tissue.

The above provided embodiments and items are now illustrated with the following, nonlimiting examples.

### EXAMPLES

### Example 1. Identification of FXII RNAi Agent Sequences and Synthesis of FXII RNAi Agents

A selection process for identifying lead sequences targeting FXII began with *in silico* methods to identify conserved sequences across variants of the FXII gene (SEQ ID NO: 1). The FXII sequence was initially screened using bioinformatics for 19-nucleotide sequences having a complementary sequence cross-reactive with human and non-human primate. Sequences known to have manufacturing challenges and those predicted to have poor RNAi activity based on known parameters were eliminated. The sequences were also evaluated for specificity to avoid off-target effects against the human and cynomolgus monkey genomes. Seventeen (17) sequence families of 19-mers were initially selected as potential candidates. An additional sequence was identified by changing the nucleotide at antisense strand position 1 (5' → 3') and the corresponding base pair on the sense strand to form a U:A base pair. Modification patterns were selected and modified RNAi agent sense strands and antisense strands were synthesized according to phosphoramidite technology on solid phase using methods known in the art for oligonucleotide synthesis. The duplexes in Table 5 and Table 6 herein, for example, were synthesized in accordance with the following:

### Synthesis

The sense and antisense strands of the FXII RNAi agents were synthesized according to phosphoramidite technology on solid phase used in oligonucleotide synthesis. Depending on the scale, a MerMade96E^{®} (Bioautomation), a MerMade12^{®} (Bioautomation), or a comparable commercially-available synthesizer was used. Syntheses were performed on a solid support made of controlled pore glass (CPG, 500 Å or 600Å, obtained from Prime Synthesis, Aston, PA, USA). All RNA and 2'-modified RNA phosphoramidites were purchased from Thermo Fisher Scientific (Milwaukee, WI, USA). Specifically, the following 2'-O-methyl phosphoramidites were used: (5'-O-dimethoxytrityl-N6-(benzoyl)-2'-O-methyl-adenosine-3'-O-(2-cyanoethyl-N,N-diisopropylamino) phosphoramidite, (5'-O-dimethoxytrityl-N4-(acetyl)-2'-O-methyl-cytidine-3'-O-(2-cyanoethyl-N,N-diisopropylamino) phosphoramidite, (5'-O-dimethoxytrityl-N2-(isobutyryl)-2'-O-methyl-guanosine-3'-O-(2-cyanoethyl-N,N-diisopropylamino)phosphoramidite, and (5'-O-dimethoxytrityl-2'-O-methyl-uridine-3'-O-(2-cyanoethyl-N,N-diisopropylamino)phosphoramidite. The 2'-deoxy-2'-fluoro-phosphoramidites carried the same protecting groups as the 2'-O-methyl RNA amidites. The following UNA phosphoramidites were used: 5'-(4,4'-Dimethoxytrityl)-N6-(benzoyl)-2',3'-seco-adenosine, 2'-benzoyl-3'-[(2-cyanoethyl)-(N,N-diisopropyl)]-phosphoramidite, 5'-(4,4'-Dimethoxytrityl)-N-acetyl-2',3'-seco-cytosine, 2'-benzoyl-3'-[(2-cyanoethyl)-(N,N-diiso-propyl)]-phosphoramidite, 5'-(4,4'-Dimethoxytrityl)-N-isobutyryl-2',3'-seco-guanosine, 2'-benzoyl-3'-[(2-cyanoethyl)-(N,N-diisopropyl)]-phosphoramidite, and 5'-(4,4'-Dimethoxy-trityl)-2',3'-seco-uridine, 2'-benzoyl-3'-[(2-cyanoethyl)-(N,N- diisopropyl)]-phosphoramidite.

Targeting ligand-containing phosphoramidites were dissolved in anhydrous dichloromethane or anhydrous acetonitrile (50 mM), while all other amidites were dissolved in anhydrous acetonitrile (50 mM) and molecular sieves (3Å) were added. 5-Benzylthio-1H-tetrazole (BTT, 250 mM in acetonitrile) or 5-Ethylthio-1H-tetrazole (ETT, 250 mM in acetonitrile) was used as activator solution. Coupling times were 10 min (RNA), 15 min (targeting ligand), 90 sec (2'OMe), and 60 sec (2'F). In order to introduce phosphorothioate linkages, a 100 mM solution of 3-phenyl 1,2,4-dithiazoline-5-one (POS, obtained from PolyOrg, Inc., Leominster, MA, USA) in anhydrous acetonitrile was employed.

### Cleavage and Deprotection of Support Bound Oligomers.

After finalization of the solid phase synthesis, the dried solid support was treated with a 1:1 volume solution of 40 wt. % methylamine in water and 28% to 31% ammonium hydroxide solution (Aldrich) for 1.5 hours at 30°C. The solution was evaporated and the solid residue was reconstituted in water (see below).

### Purification

Crude oligomers were purified by anionic exchange HPLC using a TKSgel SuperQ-5PW 13u column and Shimadzu LC-8 system. Buffer A was 20 mM Tris, 5 mM EDTA, pH 9.0 and contained 20% Acetonitrile and buffer B was the same as buffer A with the addition of 1.5 M sodium chloride. UV traces at 260 nm were recorded. Appropriate fractions were pooled then run on size exclusion HPLC using a GE Healthcare XK 16/40 column packed with Sephadex G-25 medium with a running buffer of 100mM ammonium bicarbonate, pH 6.7 and 20% Acetonitrile or filtered water.

### Annealing

Complementary strands were mixed by combining equimolar RNA solutions (sense and antisense) in 1× PBS (Phosphate-Buffered Saline, 1×, Corning, Cellgro) to form the RNAi agents. Some RNAi agents were lyophilized and stored at -15 to -25°C. Duplex concentration was determined by measuring the solution absorbance on a UV-Vis spectrometer in 1× PBS. The solution absorbance at 260 nm was then multiplied by a conversion factor and the dilution factor to determine the duplex concentration. Unless otherwise stated, all conversion factor was 0.037 mg/(mL·cm). For some experiments, a conversion factor was calculated from an experimentally determined extinction coefficient.

### Example 2. In vitro Testing of RNAi Agent Candidates

To test modified FXII RNAi agent constructs *in vitro,* the human FXII cDNA sequence (accession # NM_000505.3 (SEQ ID NO: 1)) was synthesized and cloned into a commercially-available reporter-based screening plasmid, psiCHECK2 (Promega, Madison, WI), which generated a Renilla luciferase/FXII fusion mRNA. For siRNA efficacy in the human background, Huh7 cells, a human hepatocyte-derived carcinoma cell line, were plated at ~7,500 cells per well in 96-well format. Each of the FXII siRNAs selected for *in vitro* study was co-transfected at three concentrations, 10 nM, 1 nM, and 0.1 nM, with 25 ng FXII-psiCHECK2 plasmid DNA per well and 0.2 µL LipoFectamine 2000 per well. Gene knockdown was determined by measuring Renilla luciferase levels normalized to the levels of constitutively-expressed firefly luciferase, also present on the psiCHECK2 plasmid, using the Dual Luciferase Reporter Assay (Promega, Madison, WI). Candidate sequence duplexes set forth in Table 6 were tested *in vitro.* Data are reported in the following Table 8.

**Table 8: Results of FXII RNAi Agents in Human Background, as Determined by Dual-Luciferase Reporter Assay.**

| **Duplex ID No:** | **Average Relative Rluc-FXII Expression** | | | | | |
|---|---|---|---|---|---|---|
| | **10 nM** | **St. Dev. (+/-)** | **1 nM** | **St. Dev. (+/-)** | **0.1 nM** | **St. Dev. (+/-)** |
| 91_2 | 0.078 | 0.000 | 0.113 | 0.010 | 0.595 | 0.036 |
| 91_3 | 0.079 | 0.004 | 0.132 | 0.005 | 0.655 | 0.003 |
| 91_4 | 0.074 | 0.003 | 0.157 | 0.006 | 0.675 | 0.043 |
| 91_5 | 0.069 | 0.003 | 0.135 | 0.017 | 0.593 | 0.023 |
| 91_6 | 0.068 | 0.002 | 0.136 | 0.004 | 0.660 | 0.023 |
| 91_7 | 0.109 | 0.004 | 0.179 | 0.012 | 0.756 | 0.058 |
| 91_8 | 0.081 | 0.009 | 0.166 | 0.009 | 0.805 | 0.039 |
| 91_9 | 0.082 | 0.003 | 0.184 | 0.010 | 0.748 | 0.031 |
| 91_10 | 0.090 | 0.005 | 0.124 | 0.010 | 0.706 | 0.016 |
| 125_2 | 0.155 | 0.005 | 0.515 | 0.050 | 1.044 | 0.025 |
| 125_3 | 0.154 | 0.010 | 0.378 | 0.020 | 0.897 | 0.069 |
| 125_4 | 0.153 | 0.018 | 0.298 | 0.007 | 0.844 | 0.047 |
| 125_5 | 0.212 | 0.007 | 0.616 | 0.050 | 1.074 | 0.076 |
| 126_2 | 0.123 | 0.010 | 0.192 | 0.005 | 0.940 | 0.089 |
| 126_3 | 0.114 | 0.017 | 0.194 | 0.049 | 0.853 | 0.018 |
| 126_4 | 0.124 | 0.016 | 0.167 | 0.011 | 0.819 | 0.049 |
| 126_5 | 0.128 | 0.013 | 0.213 | 0.027 | 0.868 | 0.050 |
| 126_6 | 0.133 | 0.008 | 0.177 | 0.011 | 0.858 | 0.028 |
| 126_7 | 0.202 | 0.012 | 0.286 | 0.020 | 0.869 | 0.054 |
| 127_2 | 0.110 | 0.003 | 0.167 | 0.008 | 0.828 | 0.045 |
| 127_3 | 0.120 | 0.001 | 0.161 | 0.004 | 0.687 | 0.042 |
| 127_4 | 0.102 | 0.008 | 0.136 | 0.005 | 0.804 | 0.062 |
| 127_5 | 0.146 | 0.019 | 0.182 | 0.018 | 0.900 | 0.160 |
| 127_6 | 0.135 | 0.024 | 0.128 | 0.007 | 0.824 | 0.045 |
| 132_2 | 0.322 | 0.025 | 0.397 | 0.003 | 0.977 | 0.167 |
| 132_3 | 0.156 | 0.015 | 0.211 | 0.017 | 1.001 | 0.148 |
| 223_2 | 0.232 | 0.008 | 0.374 | 0.061 | 1.013 | 0.115 |
| 849_2 | 0.561 | 0.046 | 0.697 | 0.030 | 1.151 | 0.049 |
| 849_3 | 0.583 | 0.032 | 0.632 | 0.120 | 1.133 | 0.252 |
| 1139_2 | 0.290 | 0.043 | 0.377 | 0.007 | 1.111 | 0.247 |
| 1139_3 | 0.339 | 0.007 | 0.397 | 0.073 | 1.159 | 0.204 |
| 1139_4 | 0.270 | 0.031 | 0.370 | 0.029 | 1.086 | 0.108 |
| 1139_5 | 0.259 | 0.015 | 0.365 | 0.041 | 1.122 | 0.077 |
| 1476_2 | 0.433 | 0.028 | 0.716 | 0.030 | 1.186 | 0.032 |
| 1476_3 | 0.430 | 0.001 | 0.732 | 0.092 | 1.004 | 0.020 |
| 1476_4 | 0.672 | 0.022 | 0.841 | 0.061 | 1.128 | 0.171 |
| 1476_5 | 0.636 | 0.102 | 0.794 | 0.041 | 1.119 | 0.110 |
| 1476_6 | 0.661 | 0.081 | 0.801 | 0.050 | 1.312 | 0.237 |

### Example 3. FXII Knockdown In Wild Type Mice Following FXII RNAi Agent Delivery

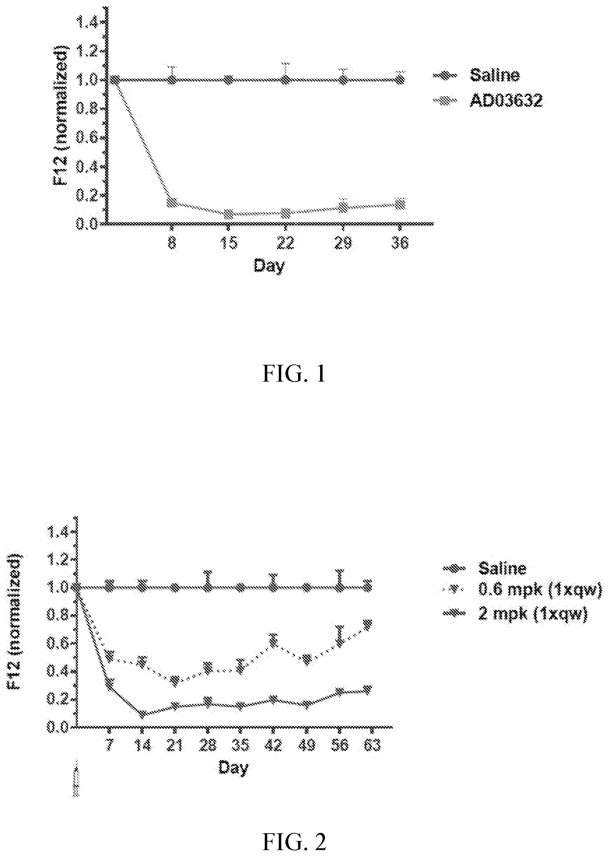

NAG-conjugated FXII RNAi agents were made and combined in a pharmaceutically acceptable buffer as described above for subcutaneous (SQ) injection. On day 1, three mice in each group (n=3) were injected with either saline or 3 mg/kg AD03632 *(see* Tables 3-5 and 7 for the modified FXII RNAi agent and NAG ligand structure). Blood samples were drawn and analyzed for FXII protein levels at days 8, 15, 22, 29 and 36. FXII protein levels were measured using an internally developed mF12 alphaLISA^{®} (Perkin Elmer) that utilized antibodies commercially acquired (Molecular Innovations), which allowed for increased throughput; alternatively, commercially available mF12 ELISA kits may be used. For normalization, FXII levels for each animal at a respective time point was divided by the pre-treatment level of expression in that animal (in this case at day 1) to determine the ratio of expression "normalized" to day 1. Knockdown of FXII is reported in FIG. 1.

### Example 4. Factor 12 (FXII) In Wild Type Mice Following FXII RNAi Agent Delivery

NAG-conjugated FXII RNAi agents were made and combined in a pharmaceutically acceptable buffer as described above for subcutaneous (SQ) injection. On day 1, four mice in each group were injected with either (i) 0.6 mg/kg AD03632; (ii) 2 mg/kg AD03632; or (iii) saline. *(see* Tables 3-5 and 7 for the modified FXII RNAi agents and NAG ligand structures). Blood samples were drawn and analyzed for FXII protein levels at days 7, 14, 21, 28, 35, 42, 49, 56, and 63. FXII protein levels were measured using an internally developed mF12 alphaLISA^{®} (Perkin Elmer) that utilized antibodies commercially acquired (Molecular Innovations), which allowed for increased throughput; alternatively, commercially available mF12 ELISA kits may be used. For normalization, FXII levels for each animal at a respective time point was divided by the pre-treatment level of expression in that animal (in this case at day 1) to determine the ratio of expression "normalized" to day 1. Knockdown of FXII is reported in FIG. 2.

### Example 5. Factor 12 (FXII) Dose Response In Mice Following FXII RNAi Agent Delivery

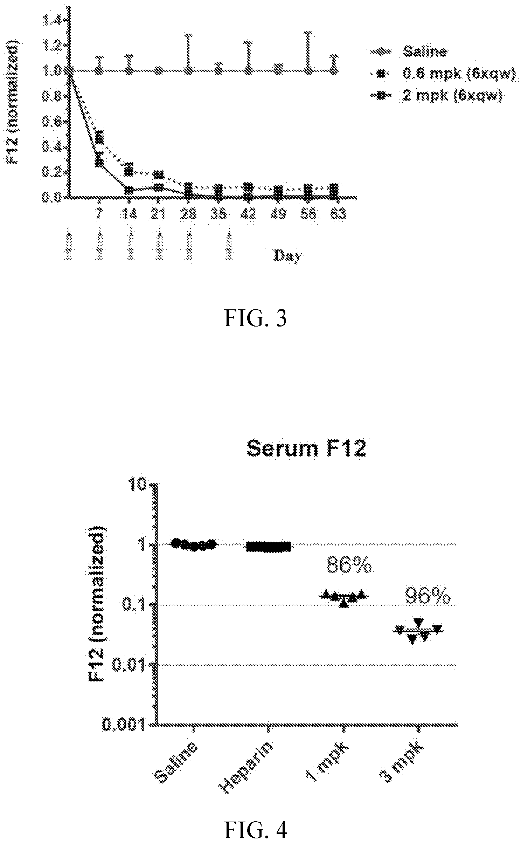

NAG-conjugated FXII RNAi agents were made and combined in a pharmaceutically acceptable buffer as described above for subcutaneous (SQ) injection. On day 1, six mice in each group were injected with either (i) 0.6 mg/kg AD03632; (ii) 2 mg/kg AD03632; or (iii) saline. *(see* Tables 3-5 and 7 for the modified FXII RNAi agent and NAG ligand structure). The mice were again injected once each week over the next six weeks with the same dose of either AD03632 or saline that was administered on day 1. Blood samples were drawn and analyzed for FXII protein levels the day prior to the next injection (i.e., blood samples drawn on days 7, 14, 21, 28, 35, 42, 49, 56, and 63). FXII protein levels were measured using an internally developed mF12 alphaLISA^{®} (Perkin Elmer) that utilized antibodies commercially acquired (Molecular Innovations), which allowed for incrased throughput; alternatively, commercially available mF12 ELISA kits may be used. For normalization, FXII levels for each animal at a respective time point was divided by the pre-treatment level of expression in that animal (in this case at day 1) to determine the ratio of expression "normalized" to day 1. Knockdown of FXII is reported in FIG. 3.

### Example 6. Factor 12 (FXII) Serum Protein Levels and Clot Weigh In Arterio-Venus Shunt Model In Rat Following FXII RNAi Agent Delivery

NAG-conjugated FXII RNAi agents were made and combined in a pharmaceutically acceptable buffer as described above for subcutaneous (SQ) injection. On day 1, five rats in each group were injected with either (i) saline; (ii) saline on day 1, followed by 1000 U/kg heparin at cannulation on day 7, 8, or 9; (iii) 1 mg/kg FXII RNAi agent AD03224; or (iv) 3 mg/kg FXII RNAi agent AD03224. *(see* Tables 3-5 and 7 for the modified FXII RNAi agent and NAG ligand structure). Blood samples were drawn on the day of procedure (i.e., day 7, 8, or 9). For normalization, FXII levels for each animal at a respective time point was divided by the pre-treatment serum level in that animal (in this case at day 1) to determine the ratio of expression "normalized" to day 1. FXII serum protein levels are reported in FIG. 4. FXII protein levels were measured using an internally developed mF12 alphaLISA^{®} (Perkin Elmer) that utilized antibodies commercially acquired (Molecular Innovations), which allowed for increased throughput; alternatively, commercially available mF12 ELISA kits may be used.

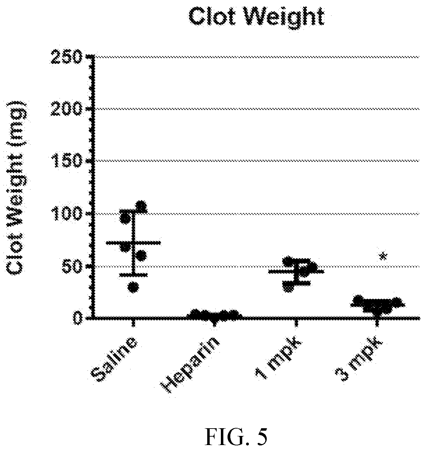

Additionally, an arterio-venous shunt model was conducted on these mice to measure. On day the day of the shunt experiment (i.e., day 7, 8, or 9), a polyurethane tubing (with silk thread) was implanted to connect the carotid artery to the jugular vein (cannulated). After 15 minutes, the shunt was removed and net thrombus weight was measured. Clot weight (in mg) is reported in FIG. 5.

### Example 7. Administration of NAG-conjugated FXII RNAi Agents In Cynomolgus Monkeys

NAG-conjugated FXII RNAi agents were made and combined in a pharmaceutically acceptable saline buffer as known in the art for subcutaneous (SC) injection. On day 1, cynomolgus macaque (*Macaca fascicularis*) primates (referred to herein as "cynos" or "monkeys") were injected subcutaneously with 3 mg/kg of AD03635, AD04131, AD04157, AD04162, AD04254, or AD04443. *(see* Tables 3-5 and 7 for the modified FXII RNAi agents and NAG ligand structures). Three monkeys in each group were tested (n=3) for groups AD03635, AD04131, AD04157, AD04162, AD04254. Only two monkeys were tested (n=2) for group AD04443. On day 29, two (2) of the monkeys in each respective group were administered a subsequent dose of 1.5 mg/kg of the same respective FXII RNAi agent that was administered on day 1.

Serum samples from treated cynomolgus monkeys were collected on day -29, day -7, and day 1 (pre-treatment), and on days 8, 15, 22, 29, 36, 43, 50, 57, 64, and 78. At the indicated time points, blood samples were drawn and serum was analyzed for FXII protein levels. FXII protein levels were determined using a commercially purchased ELISA for human F12 (Molecular Innovations) according to the manufacturer's recommendations. Standard clinical chemistry, including blood urea nitrogen (BUN), alanine transaminase (ALT), aspartate aminotransferase (AST), and creatinine were also assessed on an automated chemistry analyzer according to the manufacturer's recommendations. Activated Partial Thromboplastin Time (aPTT) was also determined using STA Compact MAX^{®} (Stago). Functional readout of F12 knockdown can be observed through elongation of the activated partial thromboplastin time (aPTT) compared to pre-treatment. The samples were also assessed for FXII activity by using an FXII activity assay. The FXII activity assay was performed using the aPTT method (standard techniques known in the art) and a factor deficient substrate. Subject plasma was combined and incubated with a FXII deficient substrate (normal plasma depleted of FXII by immunoadsorption) and an aPTT reagent. After a specified incubation time, calcium was added to trigger the coagulation process and clot formation was measured. Results are then compared to normal human results (100%) and a percent activity is calculated.

FXII protein levels for each animal at a respective time point was divided by the pre-treatment level (average of day -29, day -7 and day 1 (pre-treatment)) of expression in that animal to determine the ratio of expression "normalized to pre-treatment." Additionally, mean knockdown levels at the respective nadir (i.e., the mean lowest expression level of the days in which serum was collected) for both FXII protein and FXII activity were calculated.

**Table 9. Normalized FXII Protein from ELISA (Normalized to Pre-Treatment) in Example 7.**

| | **Day 8** | **Day 15** | **Day 22** | **Day 29** |
|---|---|---|---|---|
| **Group ID** | **FXII** | **FXII** | **FXII** | **FXII** |
| **Group 1, Cyno A** (3.0 mg/kg AD03635) | 0.61 | 0.27 | 0.21 | 0.20 |
| **Group 1, Cyno B** (3.0 mg/kg AD03635) | 0.60 | 0.45 | 0.47 | 0.39 |
| **Group 1, Cyno C** (3.0 mg/kg AD03635) | 0.57 | 0.34 | 0.24 | 0.21 |
| **Group 2, Cyno A** (3.0 mg/kg AD04131) | 0.45 | 0.27 | 0.20 | 0.19 |
| **Group 2, Cyno B** (3.0 mg/kg AD04131) | 1.00 | 0.39 | 0.27 | 0.26 |
| **Group 2, Cyno C** (3.0 mg/kg AD04131) | 0.46 | 0.33 | 0.22 | 0.21 |
| **Group 3, Cyno A** (3.0 mg/kg AD04157) | 0.45 | 0.25 | 0.20 | 0.17 |
| **Group 3, Cyno B** (3.0 mg/kg AD04157) | 0.39 | 0.28 | 0.24 | 0.26 |
| **Group 3, Cyno C** (3.0 mg/kg AD04157) | 0.64 | 0.21 | 0.17 | 0.11 |
| **Group 4, Cyno A** (3.0 mg/kg AD04162) | 0.34 | 0.13 | 0.09 | 0.09 |
| **Group 4, Cyno B** (3.0 mg/kg AD04162) | 0.33 | 0.17 | 0.12 | 0.10 |
| **Group 4, Cyno C** (3.0 mg/kg AD04162) | 0.41 | 0.20 | 0.16 | 0.12 |
| **Group 5, Cyno A** (3.0 mg/kg AD04254) | 0.51 | 0.33 | 0.25 | 0.18 |
| **Group 5, Cyno B** (3.0 mg/kg AD04254) | 0.47 | 0.19 | 0.15 | 0.12 |
| **Group 5, Cyno C** (3.0 mg/kg AD04254) | 0.68 | 0.12 | 0.14 | 0.10 |
| **Group 6, Cyno A** (3.0 mg/kg AD04443) | 0.33 | 0.20 | 0.15 | 0.15 |
| **Group 6, Cyno B** (3.0 mg/kg AD04443) | 0.35 | 0.15 | 0.11 | 0.09 |

**Table 10. Mean FXII Protein Knockdown Percentages at Nadir from Example 7.**

| **Group ID** | **Mean FXII knockdown at nadir (lowest measured date) after first dose** | **Mean FXII knockdown at nadir (lowest measured timepoint) after second dose** |
|---|---|---|
| **Group 1** (AD03635) | 73% | 86% |
| **Group 2,** (AD04131) | 78% | 86% |
| **Group 3** (AD04157) | 82% | 88% |
| **Group 4** (AD04162) | 90% | 93.5% |
| **Group 5** (AD04254) | 87% | 90% |
| **Group 6** (AD04443) | 88% | 93.5% |

**Table 11. Mean FXII Activity Percentages (Normalized to Pre-Treatment) from Example 7.**

| **Group ID** | **Mean FXII Activity knockdown at nadir (lowest measured timepoint) after first dose** | **Mean FXII Activity knockdown at nadir (lowest measured timepoint) after second dose** |
|---|---|---|
| **Group 1** (AD03635) | 67.6% | 80.5% |
| **Group 2,** (AD04131) | 65.7% | 76% |
| **Group 3** (AD04157) | 73.3% | 83% |
| **Group 4** (AD04162) | 85% | 89% |
| **Group 5** (AD04254) | 76.3% | 84% |
| **Group 6** (AD04443) | 80% | 88% |

**Table 12. Normalized aPTT (seconds) (Normalized to Pre-Treatment) from Example 7.**

| **Group ID** | **Mean aPTT at apex (highest measured timepoint) after first dose** | **Mean aPTT at apex (highest measured timepoint) after second dose** |
|---|---|---|
| **Group 1** (AD03635) | 1.27 | 1.76 |
| **Group 2,** (AD04131) | 1.27 | 1.52 |
| **Group 3** (AD04157) | 1.39 | 1.73 |
| **Group 4** (AD04162) | 1.44 | 1.73 |
| **Group 5** (AD04254) | 1.34 | 1.56 |
| **Group 6** (AD04443) | 1.59 | 1.92 |

Each of the FXII RNAi agents tested in Example 7 showed substantial knockdown of FXII in cynos. Additionally, administration of a second dose of FXII RNAi agents showed further improvement in knockdown of FXII protein and FXII activity, as well as elongation of aPTT in cynos. For example, AD04162, which includes an antisense strand having a sequence at least partially complementary to positions 127-145 of the FXII gene (SEQ ID NO: 1) exhibited 93.5% inhibition of FXII protein expression at nadir after a second dose.

### Example 8. Administration of NAG-conjugated FXII RNAi agents in Cynomolgus Monkeys

NAG-conjugated FXII RNAi agents were made and combined in a pharmaceutically acceptable saline buffer as known in the art for subcutaneous (SC) injection. On day 1, cynomolgus macaque (*Macaca fascicularis*) primates (referred to herein as "cynos" or "monkeys") were injected subcutaneously with 3 mg/kg of AD04623, AD04624, AD04625, AD04626, AD04627, or AD04628. *(see* Tables 3-5 and 7 for the modified FXII RNAi agents and NAG ligand structures). Two monkeys in each group were tested (n=2), one male and one female in each group. On day 24, a single 3 mg/kg SC injection of the same respective FXII RNAi agent that was administered to the respective animal on day 1 was administered to each cyno (i.e., a second dose).

Serum samples from treated cynomolgus monkeys were collected on day -6, and day 1 (pre-treatment), and on days 8, 15, 24, 29, 36 and 43. At the indicated time points, blood samples were drawn and serum was analyzed for FXII protein levels. FXII protein levels were determined using a commercially purchased ELISA for human F12 (Molecular Innovations) according to the manufacturer's recommendations. Standard clinical chemistry, including blood urea nitrogen (BUN), alanine transaminase (ALT), aspartate aminotransferase (AST), and creatinine were also assessed on an automated chemistry analyzer according to the manufacturer's recommendations. The samples were also assessed for FXII activity by using an FXII activity assay. The FXII activity assay was performed using the aPTT method (standard techniques known in the art) and a factor deficient substrate. Subject plasma was combined and incubated with a FXII deficient substrate (normal plasma depleted of FXII by immunoadsorption) and an aPTT reagent. After a specified incubation time, calcium was added to trigger the coagulation process and clot formation was measured. Results are then compared to normal human results (100%) and a percent activity is calculated.

FXII protein levels for each animal at a respective time point was divided by the pre-treatment level (average of day -6 and day 1 (pre-treatment)) of expression in that animal to determine the ratio of expression "normalized to pre-treatment." Additionally, mean knockdown levels at the respective nadir (i.e., the mean lowest expression level of the days in which serum was collected) for both FXII protein and FXII activity were calculated.

**Table 13. Normalized FXII Protein from ELISA (Normalized to Pre-Treatment) in Example 8.**

| | **Day 8** | **Day 15** | **Day 24** | **Day 29** | **Day 36** | **Day 43** |
|---|---|---|---|---|---|---|
| **Group ID** | **FXII** | **FXII** | **FXII** | **FXII** | **FXII** | **FXII** |
| **Group 1, Cyno A** (3.0 mg/kg AD04623) | 0.38 | 0.19 | 0.14 | 0.10 | 0.07 | 0.08 |
| **Group 1, Cyno B** (3.0 mg/kg AD04623) | 0.38 | 0.23 | 0.20 | 0.17 | 0.11 | 0.12 |
| **Group 2, Cyno A** (3.0 mg/kg AD04624) | 0.65 | 0.45 | 0.40 | 0.32 | 0.28 | 0.24 |
| **Group 2, Cyno B** (3.0 mg/kg AD04624) | 0.60 | 0.53 | 0.63 | 0.50 | 0.38 | 0.46 |
| **Group 3, Cyno A** (3.0 mg/kg AD04625) | 0.36 | 0.18 | 0.12 | 0.09 | 0.07 | 0.08 |
| **Group 3, Cyno B** (3.0 mg/kg AD04625) | 0.33 | 0.16 | 0.13 | 0.09 | 0.07 | 0.08 |
| **Group 4, Cyno A** (3.0 mg/kg AD04626) | 0.68 | 0.57 | 0.73 | 0.56 | 0.54 | 0.57 |
| **Group 4, Cyno B** (3.0 mg/kg AD04626) | 0.77 | 0.64 | 0.77 | 0.61 | 0.62 | 0.60 |
| **Group 5, Cyno A** (3.0 mg/kg AD04627) | 0.44 | 0.38 | 0.29 | 0.25 | 0.21 | 0.21 |
| **Group 5, Cyno B** (3.0 mg/kg AD04627) | 0.55 | 0.40 | 0.35 | 0.32 | 0.16 | 0.24 |
| **Group 6, Cyno A** (3.0 mg/kg AD04628) | 0.71 | 0.81 | 0.75 | 0.79 | 0.66 | 0.69 |
| **Group 6, Cyno B** (3.0 mg/kg AD04628) | 0.81 | 0.76 | 0.85 | 0.75 | 0.69 | 0.72 |

**Table 14. Average Normalized FXII Protein (Normalized to Pre-Treatment) from Example 8.**

| | **Day 8** | | **Day 15** | | **Day 24** | | **Day 29** | | **Day 36** | |
|---|---|---|---|---|---|---|---|---|---|---|
| **Group ID** | **Avg FXII** | **Std Dev (+/-)** | **Avg FXII** | **Std Dev (+/-)** | **Avg FXII** | **Std Dev (+/-)** | **Avg FXII** | **Std Dev (+/-)** | **Avg FXII** | **Std Dev (+/-)** |
| **Group 1** (3.0 mg/kg AD04623) | 0.378 | 0.004 | 0.210 | 0.025 | 0.166 | 0.043 | 0.133 | 0.049 | 0.090 | 0.029 |
| **Group 2** (3.0 mg/kg AD04624) | 0.626 | 0.033 | 0.489 | 0.052 | 0.513 | 0.162 | 0.411 | 0.124 | 0.330 | 0.073 |
| **Group 3** (3.0 mg/kg AD04625) | 0.348 | 0.024 | 0.165 | 0.014 | 0.125 | 0.004 | 0.090 | 0.007 | 0.070 | 0.003 |
| **Group 4** (3.0 mg/kg AD04626) | 0.726 | 0.068 | 0.606 | 0.052 | 0.752 | 0.031 | 0.584 | 0.040 | 0.579 | 0.060 |
| **Group 5** (3.0 mg/kg AD04627) | 0.492 | 0.077 | 0.389 | 0.011 | 0.320 | 0.037 | 0.285 | 0.054 | 0.188 | 0.036 |
| **Group 6** (3.0 mg/kg AD04628) | 0.757 | 0.070 | 0.785 | 0.040 | 0.797 | 0.069 | 0.770 | 0.021 | 0.676 | 0.025 |

**Table 15. Normalized FXII Activity (Normalized to Pre-Treatment) from Example 8.**

| | **Day 8** | **Day 15** | **Day 24** | **Day 29** | **Day 36** | **Day 43** |
|---|---|---|---|---|---|---|
| **Group ID** | **FXII** | **FXII** | **FXII** | **FXII** | **FXII** | **FXII** |
| **Group 1, Cyno A** (3.0 mg/kg AD04623) | 0.55 | 0.26 | 0.22 | 0.13 | 0.12 | 0.10 |
| **Group 1, Cyno B** (3.0 mg/kg AD04623) | 0.54 | 0.29 | 0.27 | 0.21 | 0.18 | 0.20 |
| **Group 2, Cyno A** (3.0 mg/kg AD04624) | 0.82 | 0.59 | 0.55 | 0.40 | 0.44 | 0.39 |
| **Group 2, Cyno B** (3.0 mg/kg AD04624) | 0.84 | 0.59 | 0.64 | 0.56 | 0.56 | 0.59 |
| **Group 3, Cyno A** (3.0 mg/kg AD04625) | 0.55 | 0.26 | 0.15 | 0.14 | 0.11 | 0.10 |
| **Group 3, Cyno B** (3.0 mg/kg AD04625) | 0.49 | 0.26 | 0.20 | 0.14 | 0.13 | 0.14 |
| **Group 4, Cyno A** (3.0 mg/kg AD04626) | 0.84 | 0.62 | 0.77 | 0.62 | 0.58 | 0.62 |
| **Group 4, Cyno B** (3.0 mg/kg AD04626) | 0.86 | 0.78 | 0.72 | 0.67 | 0.62 | 0.65 |
| **Group 5, Cyno A** (3.0 mg/kg AD04627) | 0.64 | 0.46 | 0.46 | 0.38 | 0.34 | 0.32 |
| **Group 5, Cyno B** (3.0 mg/kg AD04627) | 0.59 | 0.44 | 0.47 | 0.40 | 0.22 | 0.43 |
| **Group 6, Cyno A** (3.0 mg/kg AD04628) | 1.06 | 0.82 | 0.82 | 0.99 | 0.83 | 0.80 |
| **Group 6, Cyno B** (3.0 mg/kg AD04628) | 1.10 | 0.89 | 0.89 | 0.81 | 0.86 | 0.88 |

Each of the FXII RNAi agents administered in Example 8 showed substantial inhibition of FXII expression in cynos. For example, AD04625, which includes an antisense strand designed to target positions 127-145 of the FXII gene (SEQ ID NO: 1), showed mean normalized knockdown of 91.0% of FXII protein on Day 29 (0.090 +/- 0.007) (*See* Table 14, group 3).

### Example 9. Administration of NAG-conjugated FXII RNAi agents in Cynomolgus Monkeys

NAG-conjugated FXII RNAi agents were mad and combined in a pharmaceutically acceptable saline buffer as known in the art for subcutaneous (SC) injection. On day 1, cynomolgus macaque (*Macaca fascicularis*) primates (referred to herein as "cynos" or "monkeys") were injected subcutaneously with 4.0 mg/kg of AD04623, AD04625, AD04753, or AD04757. *(see* Tables 3-5 and 7 for the modified FXII RNAi agents and NAG ligand structures). A second subcutaneous injection of 4.0 mg/kg of the same FXII RNAi agent was administered on day 29. Two female monkeys in each group were tested (n=2).

Serum samples from treated cynomolgus monkeys were collected on day -14, day -7, and day 1 (pre-treatment), and on days 8, 15, 22, 29, 30, 36 and 43. At the indicated time points, blood samples were drawn and serum was analyzed for FXII protein levels. FXII protein levels were determined using a commercially purchased ELISA for human F12 (Molecular Innovations) according to the manufacturer's recommendations. Standard clinical chemistry, including blood urea nitrogen (BUN), alanine transaminase (ALT), aspartate aminotransferase (AST), and creatinine were also assessed on an automated chemistry analyzer according to the manufacturer's recommendations. Activated Partial Thromboplastin Time (aPTT) was also determined using STA^{®}-PTT Automate 5 (Stago). Functional readout of F12 knockdown can be observed through elongation of the activated partial thromboplastin time (aPTT) compared to pre-treatment. The samples were also assessed for FXII activity by using an FXII activity assay. The FXII activity assay was performed using the aPTT method (standard techniques known in the art) and a factor deficient substrate. Subject plasma was combined and incubated with a FXII deficient substrate (normal plasma depleted of FXII by immunoadsorption) and an aPTT reagent. After a specified incubation time, calcium was added to trigger the coagulation process and clot formation was measured. Results are then compared to normal human results (100%) and a percent activity is calculated.

FXII protein levels for each animal at a respective time point was divided by the pre-treatment level (average of day -6 and day 1 (pre-treatment)) of expression in that animal to determine the ratio of expression "normalized to pre-treatment." Additionally, mean knockdown levels at the respective nadir (i.e., the mean lowest expression level of the days in which serum was collected) for both FXII protein and FXII activity were calculated.

**Table 16. Normalized FXII Protein from ELISA (Normalized to Pre-Treatment) in Example 9.**

| **Group ID** | **Day 8** | **Day 15** | **Day 22** | **Day 29** | **Day 30** | **Day 36** | **Day 43** |
|---|---|---|---|---|---|---|---|
| **Group 1, Cyno A** (4.0 mg/kg AD04625) | 0.306 | 0.126 | 0.084 | 0.087 | 0.070 | 0.062 | 0.050 |
| **Group 1, Cyno B** (4.0 mg/kg AD04625) | 0.346 | 0.179 | 0.117 | 0.097 | 0.102 | 0.077 | 0.058 |
| **Group 2, Cyno A** (4.0 mg/kg AD04623) | 0.455 | 0.269 | 0.219 | 0.238 | 0.212 | 0.205 | 0.159 |
| **Group 2, Cyno B** (4.0 mg/kg AD04623) | 0.384 | 0.203 | 0.142 | 0.161 | 0.156 | 0.101 | 0.083 |
| **Group 3, Cyno A** (4.0 mg/kg AD04753) | 0.411 | 0.174 | 0.129 | 0.176 | 0.155 | 0.144 | 0.112 |
| **Group 3, Cyno B** (4.0 mg/kg AD04753) | 0.433 | 0.213 | 0.167 | 0.117 | 0.109 | 0.109 | 0.099 |
| **Group 4, Cyno A** (4.0 mg/kg AD04757) | 0.429 | 0.212 | 0.170 | 0.166 | 0.184 | 0.147 | 0.108 |
| **Group 4, Cyno B** (4.0 mg/kg AD04757) | 0.428 | 0.222 | 0.161 | 0.146 | 0.143 | 0.145 | 0.134 |

**Table 17. Average Normalized FXII Protein (Normalized to Pre-Treatment) from Example 9**

| | **Day 8** | | **Day 15** | | **Day 22** | | **Day 29** | |
|---|---|---|---|---|---|---|---|---|
| **Group ID** | **Avg FXII** | **Std Dev (+/-)** | **Avg FXII** | **Std Dev (+/-)** | **Avg FXII** | **Std Dev (+/-)** | **Avg FXII** | **Std Dev (+/-)** |
| **Group 1** (4.0 mg/kg AD04625) | 0.326 | 0.028 | 0.153 | 0.038 | 0.100 | 0.023 | 0.092 | 0.007 |
| **Group 2** (4.0 mg/kg AD04623) | 0.420 | 0.050 | 0.236 | 0.047 | 0.181 | 0.054 | 0.199 | 0.054 |
| **Group 3** (4.0 mg/kg AD04753) | 0.422 | 0.015 | 0.193 | 0.027 | 0.148 | 0.027 | 0.147 | 0.042 |
| **Group 4** (4.0 mg/kg AD04757) | 0.429 | 0.001 | 0.217 | 0.007 | 0.166 | 0.007 | 0.156 | 0.014 |

| | **Day 30** | | **Day 36** | | **Day 43** | | **Day 50** | |
|---|---|---|---|---|---|---|---|---|
| **Group ID** | **Avg FXII** | **Std Dev (+/-)** | **Avg FXII** | **Std Dev (+/-)** | **Avg FXII** | **Std Dev (+/-)** | **Avg FXII** | **Std Dev (+/-)** |
| **Group 1** (4.0 mg/kg AD04625) | 0.086 | 0.023 | 0.070 | 0.010 | 0.054 | 0.006 | 0.054 | 0.007 |
| **Group 2** (4.0 mg/kg AD04623) | 0.184 | 0.040 | 0.153 | 0.074 | 0.121 | 0.053 | 0.124 | 0.051 |
| **Group 3** (4.0 mg/kg AD04753) | 0.132 | 0.033 | 0.126 | 0.025 | 0.106 | 0.009 | 0.092 | 0.005 |
| **Group 4** (4.0 mg/kg AD04757) | 0.164 | 0.029 | 0.146 | 0.001 | 0.121 | 0.019 | 0.094 | 0.007 |

**Table 18. Mean Normalized FXII Activity from Example 9 at Nadir.**

| **Group ID** | **Mean FXII Activity Knockdown at Day 43 (nadir)** |
|---|---|
| **Group 1** (4.0 mg/kg AD04625) | 94.5% |
| **Group 2** (4.0 mg/kg AD04623) | 86% |
| **Group 3** (4.0 mg/kg AD04753) | 91% |
| **Group 4** (4.0 mg/kg AD04757) | 88% |

**Table 19. Normalized aPTT (seconds) (Normalized to Pre-Treatment) from Example 7.**

| **Group ID** | **Mean aPTT at apex (highest measured timepoint) after first dose** | **Mean aPTT at apex (highest measured timepoint) after second dose** |
|---|---|---|
| **Group 1** (4.0 mg/kg AD04625) | 2.02 | 2.435 |
| **Group 2** (4.0 mg/kg AD04623) | 1.435 | 2.125 |
| **Group 3** (4.0 mg/kg AD04753) | 1.65 | 2.035 |
| **Group 4** (4.0 mg/kg AD04757) | 1.5 | 1.675 |

In Example 9, each of the FXII RNAi agents showed substantial knockdown of the FXII gene. For example, AD04625 showed the greatest knockdown of the RNAi agents administered both after the first dose and after the second dose in FXII protein (91% knockdown after the first dose; 94.5% knockdown after the second dose). AD04625 also showed the greatest knockdown in FXII activity, and showed the most elongation of aPTT over baseline (2.02 over baseline after first dose; 2.435 over baseline at apex after second dose).

### Example 10. Factor 12 (FXII)-SEAP Mouse Model

Six to eight week old female C57BL/6 albino mice were transiently transfected *in vivo* with plasmid by hydrodynamic tail vein injection, administered at least 15 days prior to administration of an FXII RNAi agent or control. The plasmid contains the human FXII cDNA sequence (GenBank GenBank NM_000505.3 (SEQ ID NO:1)) inserted into the 3' UTR of the SEAP (secreted human placental alkaline phosphatase) reporter gene. 50 µg of the plasmid containing the FXII cDNA sequence in Ringer's Solution in a total volume of 10% of the animal's body weight was injected into mice via the tail vein to create FXII-SEAP model mice. The solution was injected through a 27-gauge needle in 5-7 seconds as previously described (Zhang G et al., "High levels of foreign gene expression in hepatocytes after tail vein injection of naked plasmid DNA." Human Gene Therapy 1999 Vol. 10, p1735-1737.). Inhibition of expression of FXII by an FXII RNAi agent results in concomitant inhibition of SEAP expression, which is measured by the Phospha-Light^{™} SEAP Reporter Gene Assay System (Invitrogen). Prior to treatment, SEAP expression levels in serum were measured and the mice were grouped according to average SEAP levels.

*Analyses:* SEAP levels may be measured at various times, both before and after administration of FXII RNAi agents.
*i) Serum collection:* Mice were anesthetized with 2-3% isoflurane and blood samples were collected from the submandibular area into serum separation tubes (Sarstedt AG & Co., Nümbrecht, Germany). Blood was allowed to coagulate at ambient temperature for 20 min. The tubes were centrifuged at 8,000 ×g for 3 min to separate the serum and stored at 4°C.
*ii) Serum SEAP levels:* Serum was collected and measured by the Phospha-Light^{™} SEAP Reporter Gene Assay System (Invitrogen) according to the manufacturer's instructions. Serum SEAP levels for each animal was normalized to the control group of mice injected with saline in order to account for the non-treatment related decline in FXII expression with this model. First, the SEAP level for each animal at a time point was divided by the pre-treatment level of expression in that animal ("pre-treatment") in order to determine the ratio of expression "normalized to pre-treatment". Expression at a specific time point was then normalized to the control group by dividing the "normalized to pre-treatment" ratio for an individual animal by the average "normalized to pre-treatment" ratio of all mice in the normal saline control group.

### Example 11. FXII RNAi Agents in the FXII-SEAP Mouse Model

The FXII-SEAP mouse model described in Example 10, above, was used. At day 1, each mouse was given a single subcutaneous injection of 200 µl of saline without an FXII RNAi agent to be used as a control, or 200 µl containing the amount FXII RNAi agent according to the following Table 20.

**Table 20. Dosing groups of FXII-SEAP mice of Example 11.**

| Group | RNAi Agent and Dose | Dosing Regimen |
|---|---|---|
| 1 | Saline (no RNAi agent) | Single injection on day 1 |
| 2 | 0.5 mg/kg AD04623 | Single injection on day 1 |
| 3 | 1.0 mg/kg AD04623 | Single injection on day 1 |
| 4 | 0.5 mg/kg AD04625 | Single injection on day 1 |
| 5 | 1.0 mg/kg AD04625 | Single injection on day 1 |
| 6 | 1.0 mg/kg AD04748 | Single injection on day 1 |
| 7 | 1.0 mg/kg AD04749 | Single injection on day 1 |
| 8 | 1.0 mg/kg AD04750 | Single injection on day 1 |
| 9 | 1.0 mg/kg AD04754 | Single injection on day 1 |
| 10 | 1.0 mg/kg AD04744 | Single injection on day 1 |
| 11 | 1.0 mg/kg AD04745 | Single injection on day 1 |

Each of the FXII RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3). Serum was collected on day -3, day 8, day 15, day 22, and day 29, and SEAP expression levels were determined pursuant to the procedure set forth in Example 10, above. Data from the experiment are shown in the following Table 21, with Average SEAP reflecting the normalized average value of SEAP:

**Table 21. Average SEAP normalized to pre-treatment and saline control in FXII-SEAP mice from Example 11.**

| | **Day 8** | | **Day 15** | | **Day 22** | | **Day 29** | |
|---|---|---|---|---|---|---|---|---|
| **Group ID** | **Avg SEAP** | **Std Dev (+/-)** | **Avg SEAP** | **Std Dev (+/-)** | **Avg SEAP** | **Std Dev (+/-)** | **Avg SEAP** | **Std Dev (+/-)** |
| **Group 1** (Saline) | 1.000 | 0.361 | 1.000 | 0.469 | 1.000 | 0.146 | 1.000 | 0.158 |
| **Group 2** (0.5 mg/kg AD04623) | 0.440 | 0.147 | 0.584 | 0.289 | 0.680 | 0.423 | 0.681 | 0.487 |
| **Group 3** (1.0 mg/kg AD04623) | 0.333 | 0.186 | 0.315 | 0.096 | 0.432 | 0.152 | 0.700 | 0.496 |
| **Group 4** (0.5 mg/kg AD04625) | 0.312 | 0.070 | 0.249 | 0.112 | 0.408 | 0.317 | 0.648 | 0.533 |
| **Group 5** (1.0 mg/kg AD04625) | 0.138 | 0.074 | 0.080 | 0.049 | 0.089 | 0.040 | 0.118 | 0.065 |
| **Group 6** (1.0 mg/kg AD04748) | 0.255 | 0.107 | 0.337 | 0.239 | 0.378 | 0.337 | 0.461 | 0.390 |
| **Group 7** (1.0 mg/kg AD04749) | 0.106 | 0.039 | 0.038 | 0.013 | 0.055 | 0.024 | 0.087 | 0.041 |
| **Group 8** (1.0 mg/kg AD04750) | 0.310 | 0.050 | 0.290 | 0.104 | 0.577 | 0.293 | 0.648 | 0.310 |
| **Group 9** (1.0 mg/kg AD04754) | 0.271 | 0.038 | 0.230 | 0.101 | 0.395 | 0.125 | 0.402 | 0.023 |
| **Group 10** (1.0 mg/kg AD04744) | 0.345 | 0.214 | 0.303 | 0.200 | 0.492 | 0.421 | 0.674 | 0.502 |
| **Group 11** (1.0 mg/kg AD04745) | 0.328 | 0.244 | 0.268 | 0.078 | 0.480 | 0.149 | 0.598 | 0.180 |

Each of the FXII RNAi agents tested in Example 11 showed substantial inhibition of expression of FXII in the SEAP mouse model compared to control.

### Example 12. FXII RNAi Agents in the FXII-SEAP Mouse Model

The FXII-SEAP mouse model described in Example 10, above, was used. At day 1, each mouse was given a single subcutaneous injection of 200 µl of saline without an FXII RNAi agent to be used as a control, or 200 µl containing 1.0 mg/kg of the FXII RNAi agent according to the following Table 22.

**Table 22. Dosing groups of FXII-SEAP mice of Example 12.**

| Group | RNAi Agent and Dose | Dosing Regimen |
|---|---|---|
| 1 | Saline | Single injection on day 1 |
| 2 | 1 mpk AD04625 | Single injection on day 1 |
| 3 | 1 mpk AD05330 | Single injection on day 1 |
| 4 | 1 mpk AD05331 | Single injection on day 1 |
| 5 | 1 mpk AD05332 | Single injection on day 1 |
| 6 | 1 mpk AD05333 | Single injection on day 1 |
| 7 | 1 mpk AD05334 | Single injection on day 1 |
| 8 | 1 mpk AD05335 | Single injection on day 1 |
| 9 | 1 mpk AD05336 | Single injection on day 1 |
| 10 | 1 mpk AD05337 | Single injection on day 1 |
| 11 | 1 mpk AD05338 | Single injection on day 1 |
| 12 | 1 mpk AD05339 | Single injection on day 1 |
| 13 | 1 mpk AD05340 | Single injection on day 1 |

Each of the FXII RNAi agents included N-acetyl-galactosamine targeting ligands conjugated to the 5'-terminal end of the sense strand, as shown in Tables 4 and 5. The injections were performed between the skin and muscle (i.e. subcutaneous injections) into the loose skin over the neck and shoulder area. Three (3) mice in each group were tested (n=3). Serum was collected on day -2, day 8, day 15, day 22, day 29, and day 36, and SEAP expression levels were determined pursuant to the procedure set forth in Example 10, above. Data from the experiment are shown in the following Table 23, with Average SEAP reflecting the normalized (to pre-treatment only) average value of SEAP:

**Table 23. Average SEAP normalized to pre-treatment in FXII-SEAP mice from Example 12.**

| | **Day 8** | | **Day 15** | | **Day 22** | | **Day 29** | | **Day 36** | |
|---|---|---|---|---|---|---|---|---|---|---|
| **Group ID** | **Avg SEAP** | **Std Dev (+/-)** | **Avg SEAP** | **Std Dev (+/-)** | **Avg SEAP** | **Std Dev (+/-)** | **Avg SEAP** | **Std Dev (+/-)** | **Avg SEAP** | **Std Dev (+/-)** |
| **Group 1** (Saline) | 0.637 | 0.035 | 0.315 | 0.062 | 0.258 | 0.111 | 0.195 | 0.027 | 0.241 | 0.040 |
| **Group 2** (1.0 mg/kg AD04625) | 0.204 | 0.108 | 0.062 | 0.039 | 0.094 | 0.062 | 0.099 | 0.066 | 0.144 | 0.082 |
| **Group 3** (1.0 mg/kg AD05330) | 0.141 | 0.007 | 0.052 | 0.009 | 0.099 | 0.059 | 0.117 | 0.048 | 0.170 | 0.054 |
| **Group 4** (1.0 mg/kg AD05331) | 0.146 | 0.034 | 0.045 | 0.011 | 0.060 | 0.007 | 0.058 | 0.005 | 0.125 | 0.015 |
| **Group 5** (1.0 mg/kg AD05332) | 0.265 | 0.033 | 0.128 | 0.041 | 0.171 | 0.056 | 0.256 | 0.100 | 0.249 | 0.063 |
| **Group 6** (1.0 mg/kg AD05333) | 0.081 | 0.014 | 0.013 | 0.005 | 0.018 | 0.004 | 0.030 | 0.008 | 0.043 | 0.012 |
| **Group 7** (1.0 mg/kg AD05334) | 0.244 | 0.030 | 0.095 | 0.044 | 0.128 | 0.077 | 0.094 | 0.086 | 0.124 | 0.052 |
| **Group 8** (1.0 mg/kg AD05335) | 0.210 | 0.092 | 0.150 | 0.056 | 0.268 | 0.078 | 0.233 | 0.086 | 0.163 | 0.079 |
| **Group 9** (1.0 mg/kg AD05336) | 0.192 | 0.122 | 0.052 | 0.039 | 0.071 | 0.047 | 0.100 | 0.075 | 0.192 | 0.171 |
| **Group 10** (1.0 mg/kg AD05337) | 0.514 | 0.142 | 0.304 | 0.088 | 0.319 | 0.100 | 0.257 | 0.211 | 0.26 | 0.067 |
| **Group 11** (1.0 mg/kg AD05338) | 0.680 | 0.123 | 0.405 | 0.177 | 0.301 | 0.132 | 0.468 | 0.188 | 0.429 | 0.145 |
| **Group 12** (1.0 mg/kg AD05339) | 0.238 | 0.028 | 0.075 | 0.035 | 0.050 | 0.029 | 0.068 | 0.028 | 0.165 | 0.055 |
| **Group 13** (1.0 mg/kg AD05340) | 0.396 | 0.088 | 0.248 | 0.091 | 0.300 | 0.165 | 0.218 | 0.088 | 0.344 | 0.164 |

As shown in Table 23, nearly all of the FXII RNAi agents tested showed a substantial inhibition of expression of FXII in the SEAP mouse model compared to control. For example, on Day 15, AD04625, AD05330, and AD05331 each showed knockdown of approximately 95% compared to pre-treatment expression levels.

### Example 13. FXII RNAi Agents in the FXII-SEAP Mouse Model)

The FXII-SEAP mouse model described in Example 10, above, was used. At day 1, each mouse was given a single subcutaneous injection of 200 µl of saline without an FXII RNAi agent to be used as a control, or 200 µl containing an amount of the FXII RNAi agent according to the following Table 24.

**Table 24. Dosing groups of FXII-SEAP mice of Example 13.**

| Group | RNAi Agent and Dose | Dosing Regimen |
|---|---|---|
| 1 | Saline (no RNAi agent) | Single injection on day 1 |
| 2 | 0.25 mg/kg AD04625 | Single injection on day 1 |
| 3 | 0.5 mg/kg AD04625 | Single injection on day 1 |
| 4 | 1.0 mg/kg AD04625 | Single injection on day 1 |
| 5 | 0.5 mg/kg AD05331 | Single injection on day 1 |
| 6 | 1.0 mg/kg AD05331 | Single injection on day 1 |
| 7 | 0.25 mg/kg AD05333 | Single injection on day 1 |
| 8 | 0.5 mg/kg AD05333 | Single injection on day 1 |
| 9 | 1.0 mg/kg AD05333 | Single injection on day 1 |
| 10 | 1.0 mg/kg AD05336 | Single injection on day 1 |
| 11 | 0.5 mg/kg AD05339 | Single injection on day 1 |
| 12 | 1.0 mg/kg AD05339 | Single injection on day 1 |

Each of the FXII RNAi agents included N-acetyl-galactosamine targeting ligands conjugated to the 5'-terminal end of the sense strand, as shown in Tables 3 and 4. The injections were performed between the skin and muscle (i.e. subcutaneous injections) into the loose skin over the neck and shoulder area. Three (3) mice in each group were tested (n=3). Serum was collected on day -3, day 8, day 15, day 22, and day 29, and SEAP expression levels were determined pursuant to the procedure set forth in Example 10, above. Data from the experiment are shown in the following Table 25, with Average SEAP reflecting the normalized (to pre-treatment only) average value of SEAP:

**Table 25. Average SEAP normalized to pre-treatment in FXII-SEAP mice from Example 13.**

| | **Day 8** | | **Day 15** | | **Day 22** | | **Day 29** | |
|---|---|---|---|---|---|---|---|---|
| **Group ID** | **Avg SEAP** | **Std Dev (+/-)** | **Avg SEAP** | **Std Dev (+/-)** | **Avg SEAP** | **Std Dev (+/-)** | **Avg SEAP** | **Std Dev (+/-)** |
| Group 1 (Saline) | 0.860 | 0.024 | 0.816 | 0.121 | 0.661 | 0.188 | 0.873 | 0.156 |
| Group 2 (0.25 mg/kg AD04625) | 0.373 | 0.031 | 0.369 | 0.093 | 0.226 | 0.035 | 0.425 | 0.048 |
| Group 3 (0.5 mg/kg AD04625) | 0.413 | 0.079 | 0.263 | 0.029 | 0.184 | 0.008 | 0.254 | 0.089 |
| Group 4 (1.0 mg/kg AD04625) | 0.149 | 0.07 | 0.077 | 0.058 | 0.013 | 0.019 | 0.062 | 0.046 |
| Group 5 (0.5 mg/kg AD05331) | 0.53 | 0.329 | 0.361 | 0.269 | 0.232 | 0.207 | 0.531 | 0.510 |
| Group 6 (1.0 mg/kg AD05331) | 0.153 | 0.090 | 0.078 | 0.073 | 0.047 | 0.038 | 0.095 | 0.069 |
| Group 7 (0.25 mg/kg AD05333) | 0.348 | 0.127 | 0.342 | 0.177 | 0.230 | 0.120 | 0.279 | 0.108 |
| Group 8 (0.5 mg/kg AD05333) | 0.186 | 0.033 | 0.128 | 0.078 | 0.117 | 0.065 | 0.158 | 0.035 |
| Group 9 (1.0 mg/kg AD05333) | 0.166 | 0.087 | 0.061 | 0.052 | 0.049 | 0.054 | 0.097 | 0.101 |
| Group 10 (1.0 mg/kg AD05336) | 0.198 | 0.042 | 0.134 | 0.032 | 0.082 | 0.044 | 0.194 | 0.171 |
| Group 11 (0.5 mg/kg AD05339) | 0.502 | 0.068 | 0.517 | 0.103 | 0.274 | 0.141 | 0.604 | 0.448 |
| Group 12 (1.0 mg/kg AD05339) | 0.221 | 0.042 | 0.164 | 0.101 | 0.139 | 0.069 | 0.182 | 0.082 |

### OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. An RNAi agent for inhibiting the expression of a Factor XII (FXII) gene, wherein the RNAi agent comprises
an antisense strand having the nucleotide sequence (5' → 3') of:
usUfsusGfuAfcUfuAfuGfcUfcCfuUfgGfsc (SEQ ID NO: 404) or
usUfsusGfuAfcUfuAfuGfcUfcCfuUfgGfsg (SEQ ID NO: 375); and
a sense strand having the nucleotide sequence (5' → 3') of:
(NAG37)gsccaaggaGfCfAfuaaguacaaas(invAb) (SEQ ID NO: 643) or
(NAG37)scsccaaggaGfCfAfuaaguacaaas(invAb) (SEQ ID NO: 611);
wherein a represents 2'-O-methyl adenosine, c represents 2'-O-methyl cytidine, g represents 2'-O-methyl guanosine, and u represents 2'-O-methyl uridine; Af represents 2'-fluoro adenosine, Cf represents 2'-fluoro cytidine, Gf represents 2'-fluoro guanosine, and Uf represents 2'-fluoro uridine; s represents a phosphorothioate linkage; (invAb) is inverted abasic deoxyribose;
(NAG37) has the structure: 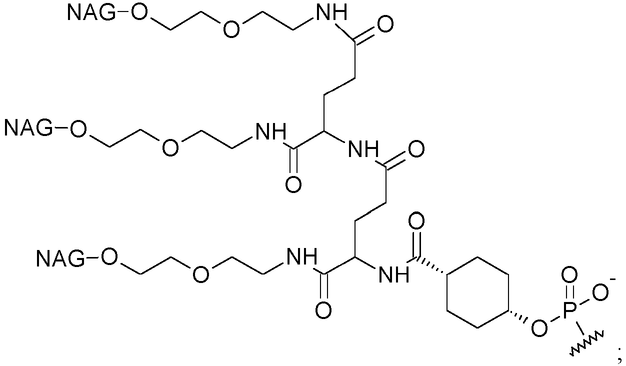
(NAG37)s has the structure:

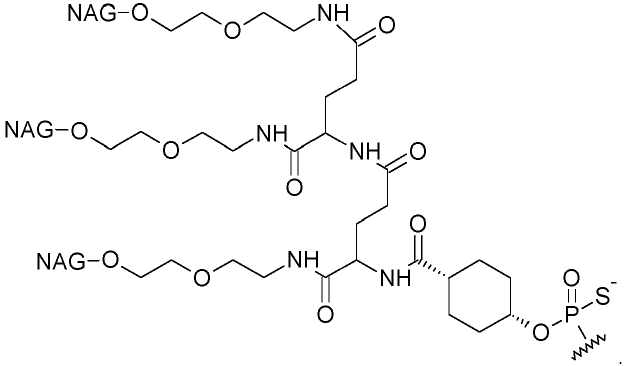

2. A composition comprising the RNAi agent of claim 1, and at least one pharmaceutically acceptable excipient.

3. The composition of claim 2, further comprising a second therapeutic or treatment.

4. The composition of claim 3, wherein said composition is packaged in a kit, container, pack, dispenser, pre-filled syringes, or vials.

5. A composition of any one of the claims 2 to 4 for use in the treatment of a pathological state that is mediated at least in part by FXII expression, wherein the pathological state is selected from the group consisting of: hereditary angioedema (HAE), acquired angioedema (AAE), ACE inhibitor associated angioedema, allergic angioedema, nonhistaminergic angioedema (INAE), idiopathic angioedema, thrombosis, venous thromboembolism (VTE), thrombotic occlusive disease, or peri-operative venous occlusive disease prophylaxis.

## Patentansprüche

1. RNAi-Mittel zum Hemmen der Expression eines Faktor XII (FXII)-Gens, wobei das RNAi-Mittel umfasst
einen Antisense-Strang, aufweisend die Nukleotidsequenz (5' → 3') von:
usUfsusGfuAfcUfuAfuGfcUfcCfuUfgGfsc (SEQ ID NO: 404) oder usUfsusGfuAfcUfuAfuGfcUfcCfuUfgGfsg (SEQ ID NO: 375); und
einen Sense-Strang, aufweisend die Nukleotidsequenz (5' → 3') von:
(NAG37)gsccaaggaGfCfAfuaaguacaaas(invAb) (SEQ ID NO: 643) oder (NAG37)scsccaaggaGfCfAfuaaguacaaas(invAb) (SEQ ID NO: 611);
wobei a 2'-O-Methyladenosin darstellt, c 2'-O-Methylcytidin darstellt, g 2'-O-Methylguanosin darstellt und u 2'-O-Methyluridin darstellt; Af 2'-Fluoradenosin darstellt, Cf 2'-Fluorcytidin darstellt, Gf 2'-Fluorguanosin darstellt, und Uf 2'-Fluoruridin darstellt; s eine Phosphorothioat-Verknüpfung darstellt; (invAb) invertierte abasische Desoxyribose ist;
(NAG37) die Struktur aufweist: 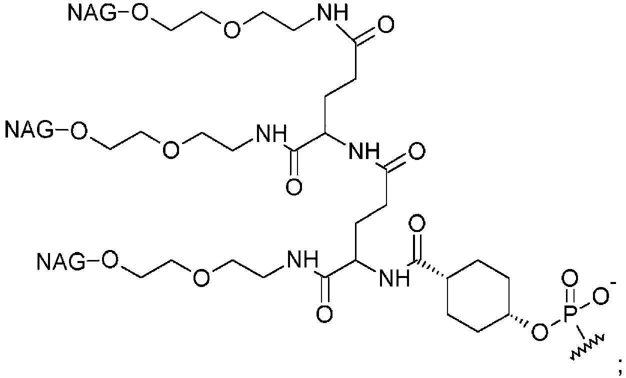
(NAG37)s die Struktur aufweist:

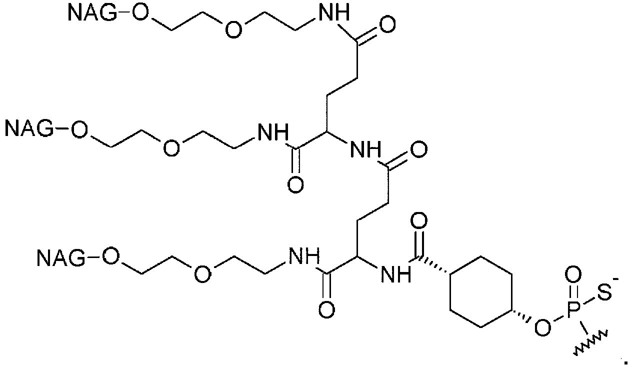

2. Zusammensetzung, umfassend das RNAi-Mittel nach Anspruch 1 und mindestens einen pharmazeutisch verträglichen Hilfsstoff.

3. Zusammensetzung nach Anspruch 2, ferner umfassend eine zweite Therapie oder Behandlung.

4. Zusammensetzung nach Anspruch 3, wobei die Zusammensetzung in einem Kit, Behälter, einer Packung, einem Spender, vorgefüllten Spritzen oder Fläschchen verpackt ist.

5. Zusammensetzung nach einem der Ansprüche 2 bis 4 zur Verwendung bei der Behandlung eines pathologischen Zustands, der mindestens teilweise durch FXII-Expression vermittelt wird, wobei der pathologische Zustand ausgewählt ist aus der Gruppe bestehend aus: hereditärem Angioödem (HAE), erworbenem Angioödem (AAE), ACE-Hemmer-assoziiertem Angioödem, allergischem Angioödem, nichthistaminergem Angioödem (INAE), idiopathischem Angioödem, Thrombose, venöser Thromboembolie (VTE), thrombotischer Verschlusskrankheit oder perioperativer venöser Verschlusskrankheitsprophylaxe.

## Revendications

1. Agent d'interférence ARN permettant d'inhiber l'expression d'un gène de Facteur XII (FXII), dans lequel l'agent d'interférence ARN comprend
un brin antisens ayant la séquence nucléotidique (5' → 3') de :
usUfsusGfuAfcUfuAfuGfcUfcCfuUfgGfsc (SEQ ID NO: 404) ou usUfsusGfuAfcUfuAfuGfcUfcCfuUfgGfsg (SEQ ID NO: 375) ; et
un brin sens ayant la séquence nucléotidique (5' → 3') de :
(NAG37)gsccaaggaGfCfAfuaaguacaaas(invAb) (SEQ ID NO: 643) ou
(NAG37)scsccaaggaGfCfAfuaaguacaaas(invAb) (SEQ ID NO: 611) ;
dans lequel a représente 2'-O-méthyladénosine, c représente 2'-O-méthylcytidine, g représente 2'-O-méthylguanosine, et u représente 2'-O-méthyluridine ; Af représente 2'-fluoroadénosine, Cf représente 2'-fluorocytidine, Gf représente 2'-fluoroguanosine, et Uf représente 2'-fluorouridine ; s représente une liaison phosphorothioate ; (invAb) est un désoxyribose abasique inversé ;
(NAG37) a la structure : 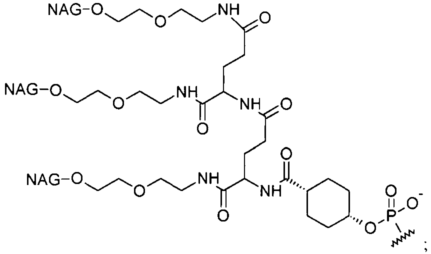
(NAG37)s a la structure :

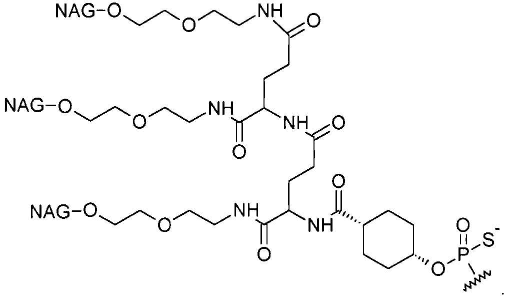

2. Composition comprenant l'agent d'interférence ARN selon la revendication 1, et au moins un excipient pharmaceutiquement acceptable.

3. Composition selon la revendication 2, comprenant en outre un second agent thérapeutique ou traitement.

4. Composition selon la revendication 3, dans laquelle ladite composition est conditionnée dans un kit, un récipient, un emballage, un distributeur, des seringues préremplies, ou des flacons.

5. Composition selon l'une quelconque des revendications 2 à 4 pour une utilisation dans le traitement d'un état pathologique qui est médié au moins en partie par l'expression de FXII, l'état pathologique étant choisi dans le groupe constitué de : œdème de Quincke héréditaire (HAE), œdème de Quincke acquis (AAE), œdème de Quincke associé à un inhibiteur de l'ECA, œdème de Quincke allergique, œdème de Quincke non histaminergique (INAE), œdème de Quincke idiopathique, thrombose, thromboembolie veineuse (VTE), maladie occlusive thrombotique, ou prophylaxie de la maladie occlusive veineuse périopératoire.
